# EUROPEAN PATENT SPECIFICATION

(11) **EP 1 636 213 B1**
(45) Date of publication and mention of the grant of the patent: **13.06.2007**
(21) Application number: 04736086.2
(22) Date of filing: 04.06.2004
(51) Int. Cl.: C07D 409/12, C07D 409/14, A61K 31/41, A61P 35/00

(54) **3-ARYLOXY AND 3-HETEROARYLOXY SUBSTITUTED BENZO(B) THIOPHENES AS THERAPEUTIC AGENTS WITH PI3K ACTIVITY**
3-ARYLOXY UND 3-HETEROARYLOXY SUBSTITUIERTE BENZO(B) THIOPHENE ALS THERAPEUTISCHE WIRKSTOFFE MIT PI3K AKTIVITÄT
BENZO B|THIOPHENES A SUBSTITUTION 3-ARYLOXY ET 3-HETEROARYLOXY EN TANT QU'AGENTS THERAPEUTIQUES A ACTIVITE PI3K

(30) Priority: 05.06.2003 US 475970 P
(43) Date of publication of application: 22.03.2006
(73) Proprietor: Warner-Lambert Company LLC, New York, NY 10017 (US)
(72) Inventor: BRUENDL, Michelle M., Ann Arbor, MI 48103 (US); CONNOLLY, Michael Kevin, Ann Arbor, MI 48103 (US); GOODMAN, Annise Paige, Ann Arbor, MI 48103 (US); GOGLIOTTI, Rocco, Dean, Ann Arbor, MI 48103 (US); LEE, Helen Tsenwhei, Ann Arbor, MI 48103 (US); PLUMMER, Mark Stephen, Ann Arbor, MI 48103 (US); SEXTON, Karen Elaine, Ann Arbor, MI 48103 (US); REICHARD, Gregory Adam, Ann Arbor, MI 48103 (US); VISNICK, Melean, Ann Arbor, MI 48103 (US); WILSON, Michael William, Ann Arbor, MI 48103 (US); SHAHRIPOUR, Aurash, Bavarsad, Ann Arbor, MI 48103 (US)
(74) Representative: Dekker, Henrike Cornelie Christine
(86) International application number: PCT/IB2004/001823
(87) International publication number: WO 2004/108715

(56) References cited:
- EP-A- 0 299 457
- WO-A-01/53266
- US-A- 5 426 113
- US-A1- 2002 151 549

## Description

### BACKGROUND OF THE INVENTION

Phosphoinositide-3-kinases (PI3Ks) are a family of lipid kinases that phosphorylate phosphoinositols on the 3'-OH to generate PI-3-P (phosphatidylinositol 3-phosphate), PI-3,4-P2 and PI-3,4,5-P3. One class of PI3Ks that are stimulated by growth factors. A separate class of PI3Ks are activated by G-protein coupled receptors and include PI3Kγ. The growth-factor stimulated PI3Ks (e.g., PI3Kα), have been implicated in cellular proliferation and cancer. PI3Kγ has been demonstrated to be involved in signaling cascades. For example, PI3Kγ is activated in response to ligands such as C5a, fMLP, ADP, and IL-8. In addition, PI3Kγ has been implicated in immune diseases (Hirsch et al. Science 2000;287:1049-1053)..PI3Kγ null macrophages show a reduced chemotactic response and a reduced ability to fight inflammation (Hirsch et al., 2000, supra). Furthermore, PI3Kγ has also been implicated in thrombolytic diseases (e.g., thromboembolism, ischemic diseases, heart attacks, and stroke) (Hirsch et al. FASEB J. 2000;15(11):2019-2021; and Hirsch et al. FASEB J., July 9 2001;10.1096/fj.00-0810fje (cited herein as Hirsch et al., 2001).

Inhibitors of members of the PI3Ks are being developed for the treatment of human disease (see e.g., WO 01/81346; WO 01/53266; and WO 01/83456). There is a need for additional compounds that can inhibit PI3Ks for use as pharmaceutical agents.

### SUMMARY OF THE INVENTION

In one aspect, the present invention provides for benzo[b]thiophenes of formula I: 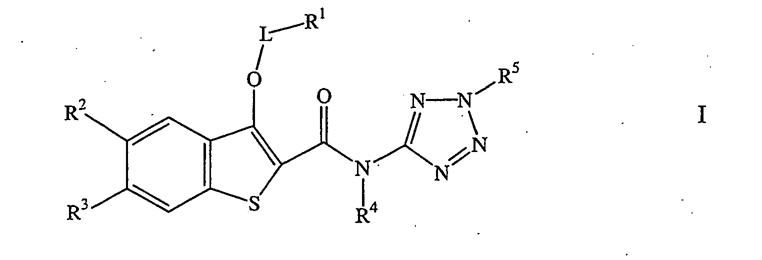 or a pharmaceutically acceptable salt thereof;
wherein R² and R³ are selected from the group consisting of:
(i) R² is methoxy and R³ is selected from the group consisting of H, methyl, and methoxy;
(ii) R² is methyl and R³ is methoxy;
(iii) R² is -O-CHF₂ and R³ is methyl;
(iv) R² is -OH and R³ is methyl;
(v) R² is cyclopropyloxy and R³ is selected from the group consisting of H, methyl, and methoxy;
(vi) R² is -O-CHF₂, and R³ is cyclopropyloxy; and
(vii) R² is ethoxy and R³ is methyl;
wherein R⁴ is H or CH₃;
wherein R⁵ is H or CH₃;
wherein L is absent, a C₁-C₄ alkylene, or a C₁-C₄ alkylene-C(O)-;
wherein R¹ is a substituted phenyl that is substituted as in group (a), (b), (c), or (d); or
R¹ is an optionally substituted group selected from the group consisting of: naphthalenyl, a 5-membered heteroaryl, 6-membered ' heteroaryl, pyrimidinyl, pyridinyl, quinolinyl, and indanyl, wherein said optionally substituted group can be substituted as in (a), (b), (c), or (d);
wherein said group (a), (b), (c), or (d) are:
(a) 1 to 3 substituents independently selected from the group consisting of:
   Br, F, Cl, -CN, -NO₂, -CF₃, -OH, -OCF₃, -SO₂-CH₃, C₁-C₄ alkyl, -CH₂CH₂-Br, -CH₂CH₂-S-(t-butyl), O-C₁-C₆alkyl, -C(NH)(NH₂), -NH-C(O)-CH₃, NH₂, N(CH₃)₂, -CH₂-C(O)-O-CH₂CH₃, C(O)-C₁-C₄ alkyl, and C(O)H;
(b) 1 substituent of J-R⁸,
   wherein J is absent, -O-, C₁-C₄-alkylene, O-C₁-C₄-alkylene, C₁-C₄-alkylene-C(O)-, or C₁-C₄-alkyleneS-,
   wherein R⁸ is an optionally substituted group selected from the group consisting of:
   phenyl, piperidinyl, morpholinyl, tetrahydropyranyl, tetrahydrothiopyranyl, 1,1 -dioxo-hexahydro-1λ⁶-thiopyranyl, a 5-membered heterocyaoalkyl, or a 6-membered heterocycloalkyl, that are optionally substituted with 1 to 3 groups independently selected from the group consisting of:
      Br, F, Cl, -CN, -NO₂, -CF₃, -OH, -OCF₃, -SO₂-CH₃, C₁-C₄ alkyl, -O-C ₁-C₆alkyl, - C(NH)(NH₂), NH-C(O)-CH₃, NH₂, N(CH₃)₂, - C(O)-NH₂, C(O)-CH₃, -C(O)-C₁-C₄ alkyl, C(O)H, and C(O)-C(CH₃)₂-NH-C(O)-O-t-butyl;
(c) 1 substituent of Z-R⁹,
   wherein Z is absent, -O-, -C₁-C₆alkylene, -O-C ₁-C₆alkylene, -C(O)-, or -CH(OH)-, -C ₁-C₄alkylene-S-, -C₁-C₆alkylene-O-, or C₁-C₄-alkylene-C(O)-;
   wherein R⁹ is a C₄-C₇ cycloalkyl optionally substituted with 4 methyl groups, or 1 to 3 groups independently selected from the group consisting of:
   =O, Br, F, Cl, -CF₃, -OH, -OCF₃, -SO₂-CH₃, C₁-C₄ alkyl, -C(O)-NH₂, CH₂-O-CH₃, piperidinyl, and 1,3-dioxolan-2-yl; and
(d) 1 or 2 substituents independently selected from (a) and 1 substituent from (b) or (c).

In certain embodiments of Formula I, R² is methoxy, and R³ is methyl -a compound of Formula II: 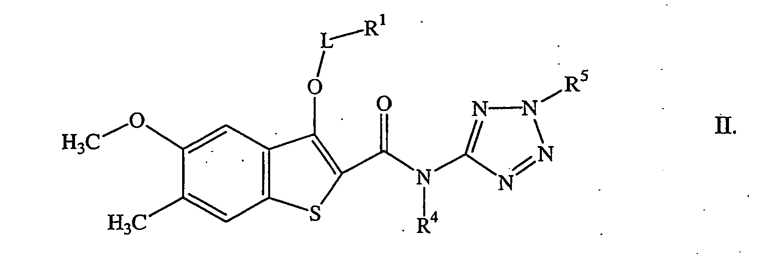 In certain embodiments of Formula II, R¹ is a substituted phenyl. In certain embodiments of Formula II, R¹ is a substituted phenyl that is substituted as in group (a), (b), (c), or (d):
(a) 1 to 3 substituents independently selected from the group consisting of:
   Br, F, Cl, -CN, -NO₂, -CF₃, -OH, -OCF₃, -SO₂-CH₃, C₁-C₄ alkyl, -CH₂CH₂-Br, -CH₂CH₂-S-(t-butyl), O-C₁-C₆alkyl, -CH₂-C(O)-O-CH₂CH₃, and C(O)-C₁-C₄ alkyl;
(b) 1 substituent of J-R⁸,
   wherein J is absent, -O-, C₁-C₄-alkylene, O-C₁-C₄-alkylene, C₁-C₄-alkylene-C(O)-, or C₁-C₄-alkylene-S-,
   wherein R⁸ is an optionally substituted group selected from the group consisting of:
   piperidinyl, morpholinyl, tetrahydropyranyl, tetrahydrothiopyranyl, 1,1-dioxo-hexahydro-1λ⁶-thiopyranyl, that are optionally substituted with 1 to 3 groups independently selected from the group consisting of:
      Br, F, Cl, -CN, -NO₂ -CF₃, -OH, -OCF₃, -SO₂ CH₃, C₁-C₄ alkyl, -O-C ₁-C₆alkyl, and -C(O)-NH₂;
(c) 1 substituent of Z- R⁹,
   wherein Z is absent, -O-, -C₁ -C₆alkylene, -O-C ₁-C₆alkylene, -C ₁-C₆alkylene-O-, or C₁-C₄-alkylene-C(O)-;
   wherein R⁹ is a C₄-C₇ cycloalkyl optionally substituted with 1 to 3 groups independently selected from the group consisting of: =O, Br, F, Cl, -CF₃, -OH, -OCF₃, -SO₂-CH₃, C₁-C₄ alkyl, and -C(O)-NH₂; and
(d) 1 or 2 substituents independently selected from (a) and 1 substituent from (b) or (c).

Examples of compounds of Formula II include, but are not limited to:
{4-[5-Methoxy-6-methyl-2-(2H-tetrazol-5-ylcarbamoyl)-benzo[*b*]thiophene-3-yloxy]-phenyl}-acetic acid ethyl ester;
3-(4-Isopropyl-phenoxy)-5-methoxy-6-methyl-benzo[*b*]thiophene-2-carboxylic acid (2H-tetrazol-5-yl)-amide;
3-(4-Cyclopentyloxy-phenoxy)-5-methoxy-6-methyl-benzo[*b*]thiophene-2-carboxylic acid (2H-tetrazol-5-yl)-amide;
3-(4-tert-Butyl-phenoxy)-5-methoxy-6-methyl-benzo[*b*]thiophene-2-carboxylic acid (2H-tetrazol-5-yl)-amide;
3-(4-Bromo-phenoxy)-5-methoxy-6-methyl-benzo[*b*]thiophene-2-carboxylic acid (2H-tetrazol-5-yl)-amide;
3-(4-Fluoro-phenoxy)-5-methoxy-6-methyl-benzo[*b*]thiophene-2-carboxylic acid (2H-tetrazol-5-yl)-amide;
3-(4-Chloro-2-fluoro-phenoxy)-5-methoxy-6-methyl-benzo[*b*]thiophene-2-carboxylic acid (2H-tetrazol-5-yl)-amide;
5-Methoxy-6-methyl-3-(4-trifluoromethoxy-phenoxy)-benzo[*b*]thiophene-2-carboxylic acid (2H-tetrazol-5-yl)-amide;
3-[4-(1-carbamoyl-cyclopentyl)-phenoxy]-5-methoxy-6-methyl-benzo[b]thiophene-2-carboxylic acid (2H-tetrazol-5-yl)-amide;
5-methoxy-6-methyl-3-[4-(tetrahydro-pyran-4-yl)-phenoxy]-benzo[b]thiophene-2-carboxylic acid(2H-tetrazol-5-yl)-amide;
3-[4-(1,1-Dioxo-hexahydro-1λ⁶-thiopyran-4-yl)-phenoxy]-5-methoxy-6-methyl-benzo[b]thiophene-2-carboxylic acid (2H-tetrazol-5-yl)-amide;
5-Methoxy-6-methyl-3-(2-nitro-4-cyclohexyl-phenoxy)-benzo[b]thiophene-2-carboxylic acid (2H-tetrazol-5-yl)-amide;
3-(2-Chloro-4-cyclohexyl-phenoxy)-5-methoxy-6-methyl-benzo[b]thiophene-2-carboxylic acid (2H-tetrazol-5-yl)-amide; and
3-(2-Cyano-4-cyclohexyl-phenoxy)-5-methoxy-6-methyl-benzo[b]thiophene-2-carboxylic acid (2H-tetrazol-5-yl)-amide.

In certain embodiments of Formula I, R² is methoxy and R³ is methoxy - a compound of Formula III: 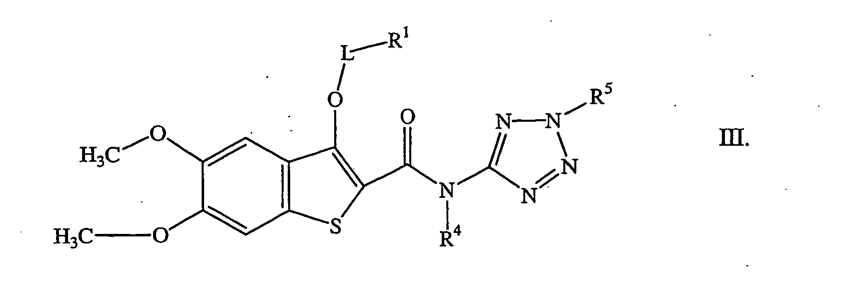 In certain embodiments of Formula III, R¹ is a substituted phenyl. An example of a compound of Formula III is 3-(2-Cyclohexylmethoxy-benzyloxy)-5,6-dimethoxy-benzo[b]thiophene-2-carboxylic acid (1H-tetrazol-5-yl)-amide.

In certain embodiments of Formula I, R² is methyl and R³ is-methoxy -a compound of Formula IV: 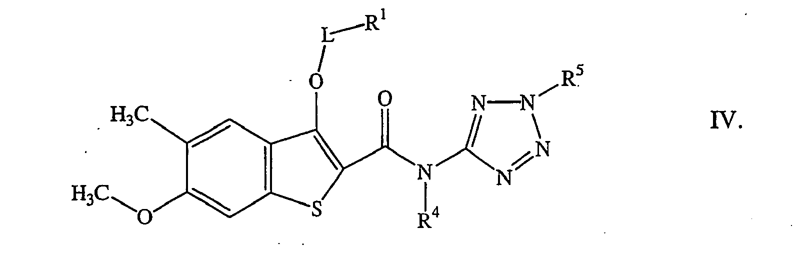 In certain embodiments of Formula IV, R¹ is a substituted phenyl. An example of a compound of Formula IV is 3-(3-Cyano-phenoxy)-6-methoxy-5-methyl-benzo[*b*]thiophene-2-carboxylic acid (2H-tetrazol-5-yl)-amide.

In certain embodiments of Formula I, R² is -O-CHF₂ and R³ is methyl -a compound of Formula V: 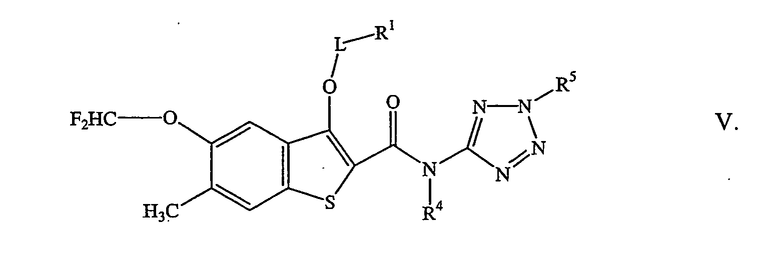 In certain embodiments of Formula V, R¹ is a substituted phenyl. An example of a compound of Formula V is 3-(4-Cyclohexyl-phenoxy)-5-difluoromethoxy-6-methyl-benzo[b]thiophene-2-carboxylic acid (2H-tetrazol-5-yl)-amide.

In certain embodiments of Formula I, R² is -OH and R³ is methoxy -a compound of Formula VI: 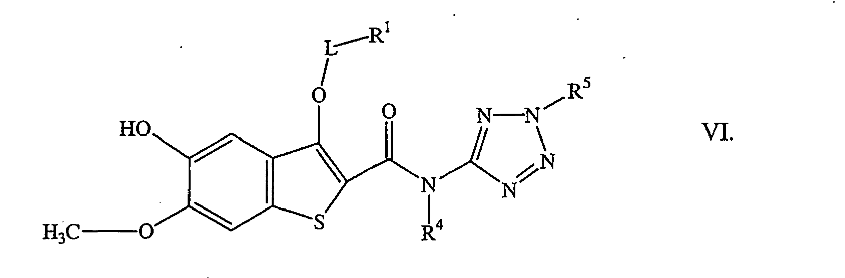 In certain embodiments of Formula VI, R¹ is a substituted phenyl. An example of a compound of Formula VI is 3-(4-Cylohexyl-phenoxy)-5-hydroxy-6-methyl-benzo[b]thiophene-2-carboxylic acid (2H-tetrazol-5-yl)-amide.

In certain embodiments of Formula I, R² is methoxy, and R³ is H-a compound of Formula VII: 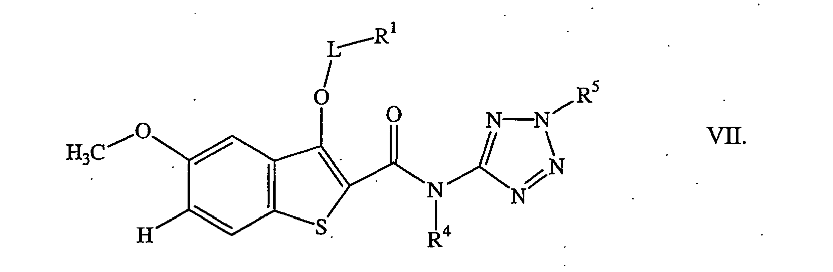 In certain embodiments of Formula VII, R¹ is a substituted phenyl. An example of a compound of Formula VII is 3-(4-cyclohexyl-phenoxy)-5-Methoxy-benzo[b]thiophene-2-carboxylic acid (2H-tetrazol-5-yl)-amide.
In certain embodiments of Formula I, R² is cyclopropyloxy and R³ is selected from the group consisting of H, methyl, methoxy;-a compound of Formula VIII: 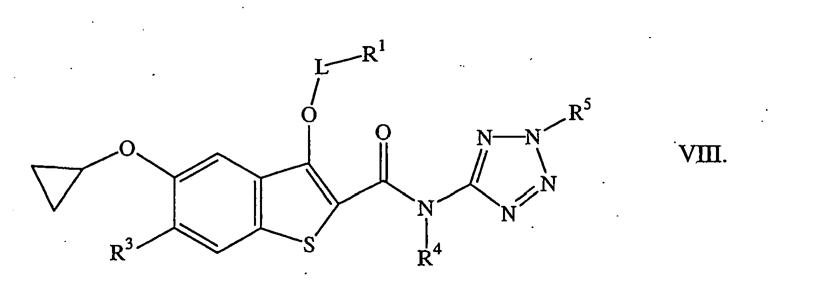 In certain embodiments of Formula V, R¹ is a substituted phenyl. An example of a compound of Formula V is 3-(4-Cyclohexyl-phenyl)-5-cyclopropyl-6-methoxy-benzo[b]thiophene-2-carboxylic acid (2H-tetrazol-5-yl)-amide.

In certain embodiments of Formula I, R² is -O-CHF₂, and R³ is cyclopropyloxy -a compound of Formula IX:

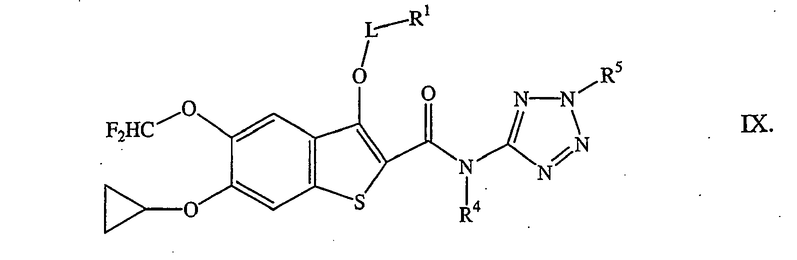

In certain embodiments of Formula IX, R¹ is a substituted phenyl. An example of a compound of Formula IX is 3-(4-Cyclohexyl-phenyl)-6-cyclopropyl-5-difluoromethyl-benzo[b]thiophene-2-carboxylic acid (2H-tetrazol-5-yl)-amide.

In certain embodiments of Formula I, R² is ethoxy, and R³ is methyl - a compound of Formula X: 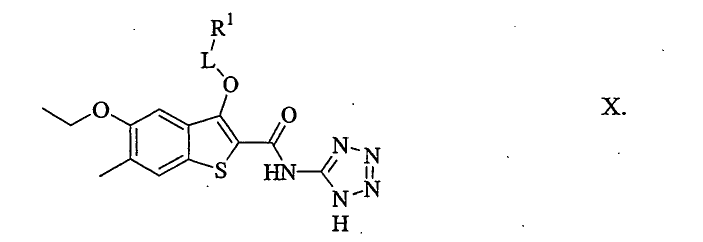 In certain embodiments of Formula X, R¹ is a substituted phenyl. An example of a compound of Formula X is 3-(4-cyclohexyl-phenoxy)-5-ethoxy-6-methyl-benzo[b]thiophene-2-carboxylic acid (1H-tetrazol-5-yl)-amide.

In certain embodiments of Formula I, R¹ is a substituted phenyl, -a compound of Formula XI: 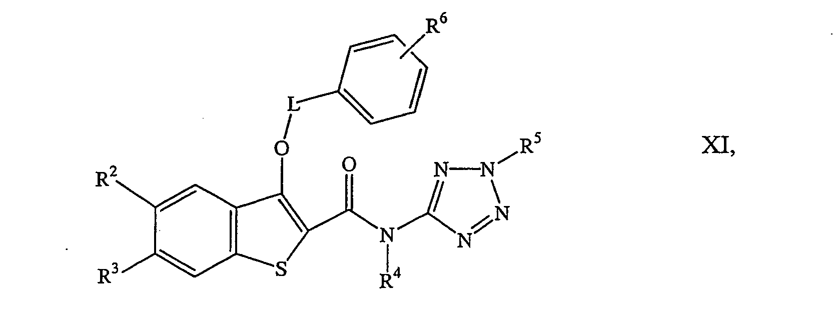 wherein R⁶ is a group selected from groups (a), (b), (c), and (d) as set out above. In certain embodiments of Formula XI, R² or R³ are methoxy. In other embodiments of Formula XI, L is a C₁-C₄ alkylene, or a C₁-C₄ alkyl-C(O)-. An example of a compound of Formula XI is 3-(2-Cyano-4-cyclohexyl-phenoxy)-5-methoxy-6-methyl-benzo[b]thiophene-2-carboxylic acid (2H-tetrazol-5-yl)-amide.

In certain embodiments, R¹ is an unsubstituted phenyl, -a compound of Formula XII: 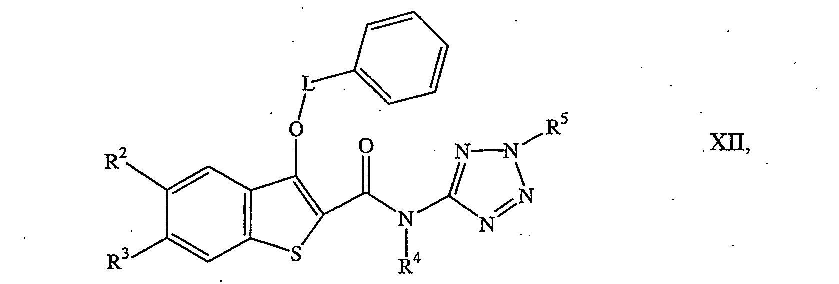 wherein L is a C₂-C₄ alkylene, or a C₂-C₄ alkyl-C(O)-. In certain embodiments of Formula XII, R² is methoxy and R³ is methoxy. Examples of compounds of Formula II include, but are not limited to:
5,6-Dimethoxy-3-((S)-1-methyl-2-phenyl-ethoxy)-benzo[b]thiophene-2-carboxylic acid (1H-tetrazol-5-yl)-amide;
5,6-Dimethoxy-3-(3-phenyl-propoxy)-benzo[b]thiophene-2-carboxylic acid (1H-tetrazol-5-yl)-amide; and
5,6-Dimethoxy-3-(2-methyl-2-phenyl-propoxy)-benzo[b]thiophene-2-carboxylic acid (1H-tetrazol-5-yl)-amide.

In another aspect, the invention provides for pharmaceutical compositions that comprise a therapeutically effective amount of a compound of Formulas I-XII and a pharmaceutically acceptable carrier. In certain embodiments, these compositions are useful in the treatment of a PI3K-mediated disorder or condition. The compounds of the invention can also be combined in a pharmaceutical composition that also comprise compounds that are useful for the treatment of cancer, a thrombolytic disease, heart disease, stroke, an inflammatory disease such as rheumatoid arthritis, or another PI3K-mediated disorder.

Also disclosed are methods of treating a subject suffering from a PI3K-mediated disorder or condition comprising: administering, to a subject suffering from a PI3K-mediated condition or disorder, a pharmaceutical composition comprising a therapeutically effective amount of a compound of Formulas I-XII and a pharmaceutically acceptable carrier. In certain embodiments, the PI3K-mediated condition or disorder is selected from the group consisting of: rheumatoid arthritis, osteoarthritis, psoriatic arthritis, psoriasis, inflammatory diseases, and autoimmune diseases. In other embodiments, the PI3K-mediated condition or disorder is selected from the group consisting of: cardiovascular diseases, atherosclerosis, hypertension, deep venous thrombosis, stroke, myocardial infarction, unstable angina, thromboembolism, pulmonary embolism, thrombolytic diseases, acute arterial ischemia, peripheral thrombotic occlusions, and coronary artery disease. In still other embodiments, the PI3K-mediated condition or disorder is selected from the group consisting of: cancer, colon cancer, glioblastoma, endometrial carcinoma, hepatocellular cancer, lung cancer, melanoma, renal cell carcinoma, thyroid carcinoma, cell lymphoma, lymphoproliferative disorders, small cell lung cancer, squamous cell lung carcinoma, glioma, breast cancer, prostate cancer, ovarian cancer, cervical cancer, and leukemia. In yet another embodiment, the PI3K-mediated condition or disorder is selected from the group consisting of: type II diabetes. In still other embodiments, the PI3K-mediated condition or disorder is selected from the group consisting of: respiratory diseases, bronchitis, asthma, and chronic obstructive pulmonary disease. In certain embodiments, the subject is a human.

### DEFINITIONS

As used herein, the following terms have the meanings ascribed to them unless specified otherwise.

A "PI3K-mediated disorder or condition" is characterized by the participation of one or more PI3Ks or a PI3P phosphatase, (e.g., PTEN, etc.) in the inception, manifestation of one or more symptoms or disease markers, severity, or progression of a disorder or condition. PI3K-mediated disorders and conditions include, but are not limited to: rheumatoid arthritis, osteoarthritis, psoriatic arthritis, psoriasis, inflammatory diseases, pulmonary fibrosis, autoimmune diseases, cardiovascular diseases, atherosclerosis, hypertension, deep venous thrombosis, stroke, myocardial infarction, unstable angina, thromboembolism, pulmonary embolism, thrombolytic diseases, acute arterial ischemia, peripheral thrombotic occlusions, coronary artery disease, cancer, breast cancer, gliobastoma, endometrial carcinoma, hepatocellular carcinoma, colon cancer, lung cancer, melanoma, renal cell carcinoma, thyroid carcinoma, small cell lung cancer, squamous cell lung carcinoma, glioma, prostate cancer, ovarian cancer, cervical cancer, leukemia, cell lymphoma, lymphoproliferative disorders, type II diabetes, respiratory diseases, bronchitis, asthma, and chronic obstructive pulmonary disease.

A PI3K is an enzyme that is able to phosphorylate the 3'-OH of a phosphoinositol to generate PI3P. PI3Ks include, but are not limited to, PI3Kα, PI3Kβ, PI3Kγ, and PI3Kδ. A PI3K typically comprises at least one catalytic subunit (e.g., p110γ), and may further comprise a regulatory subunit (e.g., p101, etc.).

The term "alkyl group" or "alkyl" includes straight and branched carbon chain radicals. The term "alkylene" refers to a diradical of an unsubstituted or substituted alkane. For example, a "C₁₋₆ alkyl" is an alkyl group having from 1 to 6 carbon atoms. Examples of straight-chain alkyl groups include, but are not limited to, methyl, ethyl, n-propyl, n-butyl, n-pentyl, n-hexyl, n-heptyl, n-octyl, n-nonyl, n-decyl, etc. Examples of branched-chain alkyl groups include, but are not limited to, isopropyl, *tert*-butyl, isobutyl, etc. Examples of alkylene groups include, but are not limited to, -CH₂-, -CH₂-CH₂-, -CH₂-CH(CH₃)-CH₂-, and - (CH₂)₁₋₆. Alkylene groups can be substituted with groups as set forth below for alkyl.

Moreover, the term alkyl includes both "unsubstituted alkyls" and "substituted alkyls," the latter of which refers to alkyl moieties having substituents replacing a hydrogen on one or more carbons (e.g., replacing a hydrogen on 1, 2, 3, 4, 5, or 6 carbons) of the hydrocarbon backbone. Such substituents can include, but are not limited to, C₂-C₆-alkenyl, C₂-C₆-alkynyl, halo, I, Br, Cl, F, -OH,-COOH, sulfhydryl, (C₁-C₆-alkyl)S-, C₁-C₆-alkylsulfinyl, nitro, cyano, trifluoromethyl, -NH₂, =O, =S, =N-CN, =N-OH, -OCH₂F, -OCHF₂, -OCF₃,-SCF₃, -SO₂-NH₂,C₁-C₆-alkoxy, -C(O)O-(C₁-C₆ alkyl), -O-C(O)-(C₁-C₆ alkyl), -C(O)-NH₂, -C(O)-N(H)-C₁-C₆ alkyl, -C(O)-N(C₁-C₆ alkyl)₂, -OC(O)-NH₂, - C(O)-H, -C(O)-(C₁-C₆ alkyl), -C(S)-(C₁-C₆ alkyl), -NR⁷⁰R⁷², where R⁷⁰ and R⁷² are each independently selected from H, C₁-C₆-alkyl, C₂-C₆-alkenyl, C₂-C₆-alkynyl, and C(O)-C₁-C₆-alkyl.

Typical substituted alkyl groups thus are aminomethyl, 2-nitroethyl, 4-cyanobutyl, 2,3-dichloropentyl, and 3-hydroxy-5-carboxyhexyl, 2-aminoethyl, pentachloroethyl, trifluoromethyl, 2-diethylaminoethyl, 2-dimethylaminopropyl, ethoxycarbonylmethyl, methanylsulfanylmethyl, methoxymethyl, 3-hydroxypentyl, 2-carboxybutyl, 4-chlorobutyl, and pentafluoroethyl.

"Alkoxy" refers to the alkyl groups mentioned above bound through oxygen, examples of which include methoxy, ethoxy, isopropoxy, *tert*-butoxy, and the like. In addition, alkoxy refers to polyethers such as O-(CH₂)₂-O-CH₃, and the like. The term "alkoxy" is intended to include both substituted and unsubstituted alkoxy groups. Alkoxy groups can be substituted on carbon atoms with groups such as those set out above for alkyl. Typical substituted alkoxy groups include aminomethoxy, trifluoromethoxy, 2-diethylaminoethoxy, 2-ethoxycarbonylethoxy, 3-hydroxypropoxy, and the like.

"Halo" includes fluoro, chloro, bromo, and iodo.

"Alkenyl" means straight and branched hydrocarbon radicals having 2 or more carbon atoms and comprising at least one carbon-carbon double bond and includes ethenyl, 3-buten-1-yl, 2-ethenylbutyl, 3-hexen-1-yl, and the like. The term "alkenyl" is intended to include both substituted and unsubstituted alkenyl groups. A "C₂-C₆-alkenyl" is an alkenyl group having from from 2 to 6 carbon atoms. Alkenyl groups can be substituted with groups such as those set out above for alkyl. The term "alkenylene" refers to a diradical of a substituted or unsubstituted alkene. Examples of alkenylene groups include, but are not limited to, -CH=CH-, -CH=CH-CH₂-, and -(CH₂)₁₋₆-CH=CH-CH₂-.

"Alkynyl" means straight and branched hydrocarbon radicals having 2 or more carbon atoms and comprising at least one carbon-carbon triple bond and includes ethynyl, 3-butyn-1-yl, propynyl, 2-butyn-1-yl, 3-pentyn-1-yl, and the like. The term "alkynyl" is intended to include both substituted and unsubstituted alkynyl groups. Alkynyl groups can be substituted with groups such as those set out above for alkyl. In certain embodiments, a straight chain or branched chain alkynyl group has 6 or fewer carbon atoms in its backbone (e.g., C₂-C₆ for straight chain, C₃-C₆ for branched chain). The term C₂-C₆ includes alkynyl groups containing 2 to 6 carbon atoms. The term "alkynylene" refers to a diradical of a substituted or unsubstituted alkyne. Examples of alkynylene groups include, but are not limited to, -CH≡CH-, -C≡C-CH₂-, and -(CH₂)₁₋₆-C≡C-CH₂-.

"Carbocycle" or "Cycloalkyl" means a mono or bicyclic carbocyclic ring functional group including, but not limited to, cyclopropyl, cyclobutyl, cyclopentyl, cyclohexyl, cycloheptyl, cyclooctyl, cyclononyl, bicyclo[2.2.1]heptanyl, bicyclo[3.2.1]octanyl, and bicyclo[5.2.0]nonanyl; wherein the cycloalkyl group may optionally contain 1 or 2 double bonds (i.e., a cycloalkylenyl) including, but not limited to, cyclopentenyl, cyclohexenyl, and cycloheptenyl. The term "cycloalkyl" is intended to include both substituted and unsubstituted cycloalkyl groups. Cycloalkyl groups and cyclohexyl groups can be substituted with groups such as those set out above for alkyl. Unless otherwise indicated, the term "(C₃-C₈)cycloalkyl" refers to a cycloalkyl group containing from 3 to 8 carbons. Thus, the term "(C₃-C₈)cycloalkyl" encompasses a monocyclic cycloalkyl group containing from 3 to 8 carbons and a bicyclic cycloalkyl group containing from 6 to 8 carbons. Examples of substituted cycloalkyl groups include, but are not limited to, 2-methyl-cyclohexyl, 3-methyl-cyclohexyl, and 4-methyl-cyclohexyl.

The phrase "3- to 8-membered heterocycloalkyl" means a stable cyclic group having carbon atoms and 1 to 3 heteroatoms independently selected from S, N or O, wherein when two O atoms or one O atom and one S atom are present, the two O atoms or one O atom and one S atom are not bonded to each other, respectively. Optionally, a 3- to 8-membered heterocycloalkyl may contain 1 or 2 carbon-carbon or carbon-nitrogen double bonds. Illustrative examples of 3- to 8-membered heterocycloalkyl include aziridin-1-yl, 1-oxa-cyclobutan-2-yl, tetrahydrofuran-3-yl, morpholin-4-yl, 2-thiacyclohex-1-yl, 2-oxo-2-thiacyclohex-1-yl, 2,2-dioxo-2-thiacyclohex-1-yl, and 4-methyl-piperazin-2-yl.

The term "heterocycloalkyl" is intended to include both substituted and unsubstituted heterocycloalkyl groups. Heterocycloalkyl groups can be substituted with 1 to 4 groups such as those set out above for alkyl. Illustrative examples of substituted 3- to 8-membered heterocycloalkyl include 2-hydroxy-aziridin-1-yl, 3-oxo-1-oxacyclobutan-2-yl, 2,2-dimethyl-tetrahydrofuran-3-yl, 3-carboxy-morpholin-4-yl, and 1-cyclopropyl-4-methyl-piperazin-2-yl,

Unless otherwise indicated, the foregoing heterocycloalkyls can be C-attached or N-attached where such is possible and which results in the creation of a stable structure. For example, piperidinyl can be piperidin-1-yl (N-attached) or piperidin-4-yl (C-attached).

Embraced within the term "heterocycloalkyl" are 5 membered rings having one carbon-carbon or one carbon-nitrogen double bond in the ring (e.g., 2-pyrrolinyl, 3-pyrrolinyl, etc.) and 6 membered rings having one carbon-carbon or one carbon-nitrogen double bond in the ring (e.g., dihydro-2H-pyranyl, 1,2,3,4-tetrahydropyridine, 3,4-dihydro-2H-[1,4]oxazine, etc.).

A "3-membered heterocycloalkyl" is a stable 3-membered, monocyclic cycloalkyl ring having 2 carbon atoms and 1 heteroatom selected from the group consisting of: 1 O; 1 S; and 1 N. Illustrative examples of stable 3-membered heterocycloalkyls include oxiranyl, aziridinyl, and thiiranyl.

A "4-membered heterocycloalkyl" is a stable 4-membered, monocyclic cycloalkyl ring having 3 carbon atoms and 1 heteroatom selected from the group consisting of: 1 O; 1 S; and 1 N. Illustrative examples of stable 4-membered heterocycloalkyls include oxetanyl, azetidinyl, and thietanyl.

A "5-membered heterocycloalkyl" is a stable 5-membered, monocyclic cycloalkyl ring having from 2 to 4 carbon atoms and from 1 to 3 heteroatoms selected from the group consisting of: 1 O; 1 S; 1 N; 2 N; 3 N; 1 S and 1 N; 1 S, and 2 N; 1 O and 1 N; and 1 0 and 2 N. Illustrative examples of stable 5-membered heterocycloalkyls include tetrahydrofuranyl, dihydrofuranyl, tetrahydrothienyl, dihydrothienyl, imidazolidinyl, oxazolidinyl, imidazolinyl, isoxazolidinyl, pyrrolidinyl, 2-pyrrolinyl, and 3-pyrrolinyl.

A "6-membered heterocycloalkyl" is a stable 6-membered, monocyclic cycloalkyl ring having from 3 to 5 carbon atoms and from 1 to 3 heteroatoms selected from the group consisting of: 1 O; 2 O;1 S; 2 S; 1 N; 2 N; 3 N; 1 S, 1 O, and 1 N; 1 S and 1 N; 1 S and 2 N; 1 S and 1 O; 1 S and 2 O; 1 O and 1 N; and 1 O and 2 N. Illustrative examples of stable 6-membered heterocycloalkyls include tetrahydropyranyl, dihydropyranyl, dioxanyl, 1,3-dioxolanyl, 1,4-dithianyl, hexahydropyrimidine, morpholinyl, piperazinyl, piperidinyl, 2H-pyranyl, 4H-pyranyl, pyrazolidinyl, pyrazolinyl, 1.,2,3,6-tetrahydropyridinyl, tetrahydrothiopyranyl, 1,1-dioxo-hexahydro-1λ⁶-thiopyranyl, 1,1-dioxo-1λ⁶-thiomorpholinyl, thiomorpholinyl, thioxanyl, and trithianyl.

A "7-membered heterocycloalkyl" is a stable 7-membered, monocyclic cycloalkyl ring having from 4 to 6 carbon atoms and from 1 to 3 heteroatoms selected from the group consisting of: 1 O; 2 O; 1 S; 2 S; 1 N; 2 N; 1 S, 1 O, and 1N; 1 S and 1 N; 1 S and 2 N ;1 S and 1 O; 1 S and 2 O ; 1 O and 1 N ;and1 O and 2 N. Illustrative examples of stable 7-membered heterocycloalkyls include azepanyl, 2,3,4,5-tetrahydro-1H-azepinyl, oxepanyl, 2,3,4,5-tetrahydro-1H-oxepinyl, thiepanyl, and 2,3,4,5-tetrahydro-1H-thiepinyl.

An "8-membered heterocycloalkyl" is a stable 8-membered, monocyclic cycloalkyl ring having from 5 to 7 carbon atoms and from 1 to 3 heteroatoms selected from the group consisting of: 1 O; 2 O; 3 O;1 S; 2 S; 3 S;1 N; 2 N; 3 N; 1 S, 1 O, and 1 N; 1 S and 1 N; 1 S and 2 N; 1 S and 1 O; 1 S and 2 O; 1 O and 1 N; and 1 O and 2 N. Illustrative examples of stable 8-membered heterocycloalkyls include azocanyl, thiocanyl, oxocanyl, 3,4,5,6-tetrahydro-2H-oxocinyl, etc.

The term "3- to 8-membered heterocycloalkyl" includes saturated and unsaturated "3- to 8-membered heterocycloalkyls." "3- to 8-membered heterocycloalkyls" may be substituted as set out above for alkyl.

The term "6- to 12-membered bicyclic heterocycloalkyl" refers to a stable ring structure which is either saturated or unsaturated, and which is the result of the fusion of a 5-, 6-, or 7-membered heterocycloalkyl to a 3-, 4-, 5-, 6-, or 7-membered heterocycloalkyl; or a 5-, 6-, or 7-membered heterocycloalkyl to a C₃₋₇-cycloalkyl, wherein the fusion junctions are at adjacent ring atoms. The term "6- to 12-membered bicyclic heterocycloalkyl" includes saturated and unsaturated "6- to 12-membered bicyclic heterocycloalkyls." "6- to 12-membered bicyclic heterocycloalkyls" may be substituted as set out above for alkyl. Examples of "6- to 12-membered bicyclic heterocycloalkyls" include 3-azabicyclo[3.1.0]hexanyl, and 3-azabicyclo[4.1.0]-heptanyl.

The term "6- to 11-membered bridged bicyclic heterocycloalkyl" refers to a stable ring structure which is either saturated or unsaturated, and which is the result of the fusion of 5-, 6-, or 7-membered heterocycloalkyl to a 4-, or 5-membered heterocycloalkyl; or a 5-, 6-, or 7-membered heterocycloalkyl to a C₅₋₇-cycloalkyl, wherein the fusion junctions have 1 to 3 intervening ring atoms. The term "6- to 11-membered bridged bicyclic heterocycloalkyl" includes saturated and unsaturated "6- to 11-membered bridged bicyclic heterocycloalkyls." "6- to 11-membered bridged bicyclic heterocycloalkyls" may be substituted as set out above for alkyl. Examples of "6- to 11-membered bridged bicyclic heterocycloalkyls" include 3-azabicyclo[4.2.1]nonanyl and 7-azabicyclo[2.2.1]heptanyl.

An aryl group is an aromatic hydrocarbon radical. Furthermore, the term "aryl" includes multicyclic aryl groups, for example bicyclic aryl groups such as naphthyl. Typical aryl groups include phenyl, and naphthyl. Phenyl may be unsubstituted or substituted at one or more positions with a substituent such as, but not limited to, those substituents described above for alkyl. Typical substituted phenyl groups include, but are not limited to, 3-chlorophenyl, 2,6-dibromophenyl, 2,4,6-tribromophenyl, 2,6-dichlorophenyl, 4-trifluoromethylphenyl, 3-amino-4-nitrophenyl, 3,5-dihydroxyphenyl, 3-methyl-phenyl, 4-methyl-phenyl, 3,5-dimethyl-phenyl, 3,4,5-trimethoxy-phenyl, 3,5-dimethoxy-phenyl, 3,4-dimethoxy-phenyl, 3-methoxy-phenyl, 4-methoxy-phenyl, 4-tert-butyl-phenyl, 4-hexyl-phenyl, 4-cyano-phenyl, 3,5-di-triflouromethyl-phenyl, 3,5-difluoro-phenyl, 4-chloro-phenyl, 3-trifluoromethyl-phenyl, 4-methoxycarbonyl-phenyl, 2-trifluoromethoxy-phenyl, 3,5-dichloro-phenyl, 2-methoxy-5-methyl-phenyl, 2-fluoro-5-methyl-phenyl, 4-phenoxy-phenyl, 4-chloro-2-trifluoromethyl-phenyl, and the like. Polycyclic aryl groups such as naphthalenyl may be unsubstituted or substituted at one or more positions with a substituent such as, but not limited to, those substituents described above for alkyl. The term "aryl" is intended to include both substituted and unsubstituted phenyl groups.

A "9- to 12-membered bicyclic aryl" is a stable ring structure formed by the fusion of a benzene ring to:
(1) a C₅₋₈ monocyclic cycloalkyl to give, e.g., indanyl; 1,2,3,4-tetrahydro-naphthalenyl; 6,7,8,9-tetrahydro-5H-benzocycloheptenyl, etc.;
(2) a 5- to 7-membered heterocycloalkyl to give, e.g., benzoxazine, benzthiazine, chromanyl, 1,2,3,4-tetrahydro-quinolinyl, etc.; or
(3) another benzene ring to give, e.g., naphthalenyl, etc.;
wherein the fusion junctions are at adjacent carbons on the benzene ring.

A "5-membered heteroaryl" is a stable 5-membered, monocyclic, aromatic ring radial having from 1 to 4 carbon atoms and from 1 to 4 heteroatoms selected from the group consisting of: 1 O; 1 S; 1 N; 2 N; 3 N; 4 N; 1 S and 1 N; 1 S and 2 N; 1 O and 1 N; and 1 O and 2 N. Illustrative examples of stable 5-membered heteroaryls include, but are not limited to, furanyl, 2-furanyl, 3-furanyl, imidazolyl, isoxazolyl, isothiazolyl, oxadiazolyl, oxazolyl, pyridinyl, 2-, 3-, or 4-pyridinyl, pyrimidinyl, 2-, 4-, or-5-pyrimidinyl, pyrazolyl, pyrrolyl, 2- or 3-pyrrolyl, pyrazinyl, pyridazinyl, 3- or 4-pyridazinyl, 2-pyrazinyl, thienyl, 2-thienyl, 3-thienyl, tetrazolyl, thiazolyl, thiadiazolyl, triazinyl and triazolyl.

A "6-membered heteroaryl" is a stable 6-membered, monocyclic, aromatic ring radical having from 3 to 5 carbon atoms and from 1 to 3 heteroatoms selected from the group consisting of: 1 N; 2 N; and 3 N. Illustrative examples of stable 6-membered heteroaryl include pyridin-2-yl, pyridin-4-yl, pyrimidin-2-yl, pyridazin-4-yl, and pyrazin-2-yl.

An "8- to 12-membered bicyclic heteroaryl" is a stable ring structure formed by the fusion of 5- or 6-membered heteroaryl to:
(1) an independently selected 5-membered heteroaryl;
(2) an independently selected 6-membered heteroaryl to give, e.g., naphthyridinyl, pteridinyl, phthalazinyl, purinyl, etc.;
(3) a C₅₋₈ monocyclic cycloalkyl;
(4) a 5- to 7-membered heterocycloalkyl; or
(5) a benzene ring to give, e.g., benzimidazolyl, benzofuranyl, benzofurazanyl, 2H-1-benzopyranyl, benzothiadiazine, benzothiazinyl, benzothiazolyl, benzothiophenyl, benzoxazolyl, cinnolinyl, furopyridinyl, indolinyl, indolizinyl, indolyl, or 2-, 3-, 4-, 5-, 6-, or 7-indolyl, 3H-indolyl, quinolinyl, quinazolinyl, quinoxalinyl, isoindolyl, and isoquinolinyl, wherein the fusion junctions are at adjacent ring atoms. The fusion junctions may be at a nitrogen (e.g., indolizine) or at carbon atoms in a 5- or 6-membered heteroaryl.

A heteroaryl can also include ring systems substituted on ring carbons with one or more -OH functional groups (which may tautomerize to give a ring C=O group) and/or substituted on a ring sulfur atom by 1 or 2 oxygen atoms to give S=O, or SO₂ groups, respectively.

Some of the compounds in the present invention may exist as stereoisomers, including enantiomers, diastereomers, and geometric isomers. Geometric isomers include compounds of the present invention that have alkenyl groups, which may exist as entgegen or zusammen conformations, in which case all geometric forms thereof, both entgegen and zusammen, *cis* and *trans,* and mixtures thereof, are within the scope of the present invention. Some compounds of the present invention have cycloalkyl groups, which may be substituted at more than one carbon atom, in which case all geometric forms thereof, both *cis* and *trans,* and mixtures thereof, are within the scope of the present invention. All of these forms, including (R), (S), epimers, diastereomers, cis, trans, syn, anti, (E), (Z), tautomers, and mixtures thereof, are contemplated in the compounds of the present invention.

### DETAILED DESCRIPTION OF THE INVENTION

### I. INTRODUCTION

The present invention relates to benzo[b]thiophenes of Formulas I-XII, wherein R¹, R², R³, R⁴, R⁵, and L have any of the values defined therefor in the specification, and pharmaceutically acceptable salts thereof, that are useful as agents in the treatment of diseases and conditions, including inflammatory diseases, cardiovascular diseases, and cancers. Also provided are pharmaceutical compositions comprising one or more compounds of Formulas I-XII.

### II. PREPARATION OF COMPOUNDS

Compounds of the present invention (e.g., compounds of Formulas 1-XII) can be prepared by applying synthetic methodology known in the art and synthetic methodology outlined in the schemes set forth below.

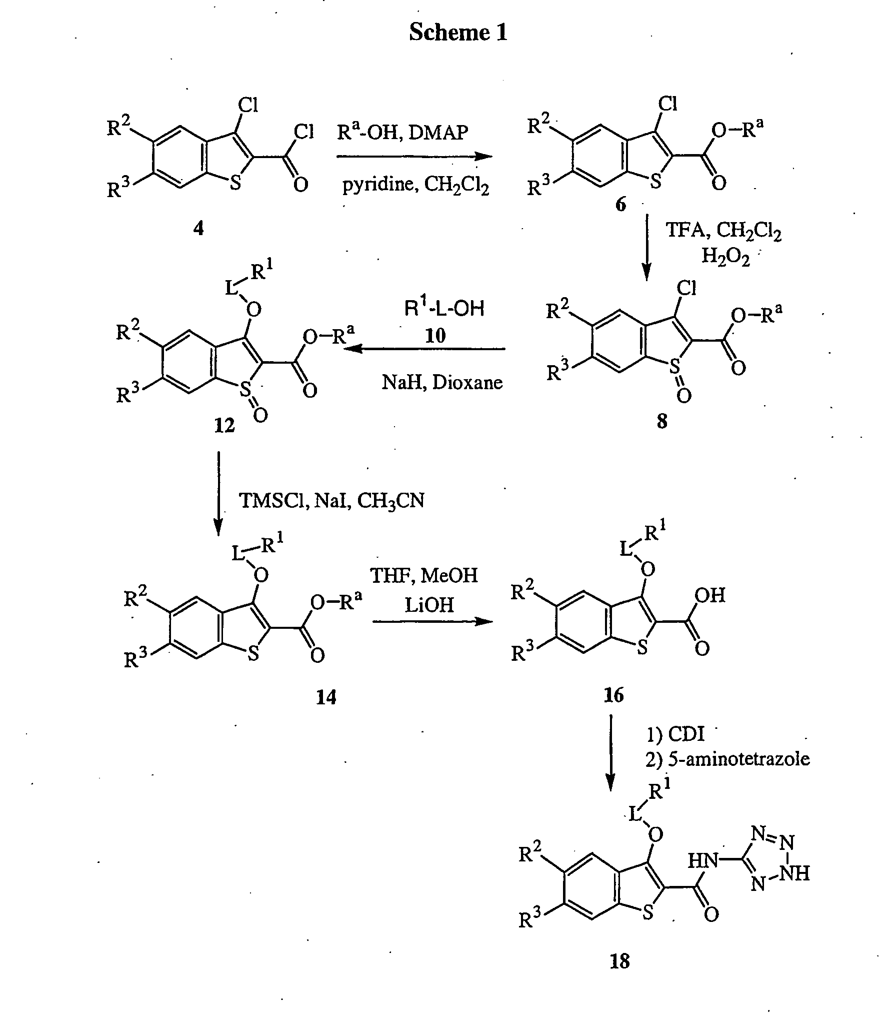

In Scheme 1, an acid chloride **4** (e.g., 3-Chloro-5-methoxy-benzo[b]thiophene-2-carbonyl chloride) is reacted with R^{a}-OH (e.g., phenol, isopropyl alcohol, methanol, etc.), pyridine or triethylamine (TEA), and 4-dimethylaminopyridine (DMAP) in CH₂Cl₂ to yield the ester **6** (e.g., 3-chloro-5-methoxy-benzo[b]thiophene-2-carboxylic acid phenyl ester). R^{a}-OH can be any suitable alcohol, where R^{a} is a C₁-C₄ alkyl, phenyl, benzyl, isopropyl, methyl, etc., that protects the carboxylic acid group and can be removed subsequently by base hydrolysis. Acid chlorides of formula **4** can be synthesized using methods that are well-known in the art (see e.g., Pakray and Castle (1986) J. Heterocyclic Chem. 23: 1571-1577; Boschelli et al. (1995) J. Med. Chem. 38: 4597-4614; Connor et al. (1992) J. Med. Chem. 35: 958-965).

The ester **6** is then oxidized to the 1-oxo compound **8** (e.g., 3-chloro-5-methoxy-1-oxo-benzo[*b*]thiophene-2-carboxylic acid phenyl ester) using trifluroacetic acid (TFA), CH₂Cl₂, and hydrogen peroxide (H₂O₂). Sodium hydride treated **10** (R¹-L-OH) is then added to a heterogeneous mixture of **8** in dioxane to yield **12** (e.g., 5-methoxy-1-oxo-3-phenoxy-benzo[*b*]thiophene-2-carboxylic acid phenyl ester). Alternatively, **8** can be reacted with butyl-lithium treated **10** in THF or diethylether to provide **12.** R¹ and L are as defined herein. A variety of R¹-L-OH compounds can be used including but not limited to, phenol, biphenyl-3-ol, 3-methyl-phenol, 3-nitro-phenol, 3-acetylamino-phenol, naphthalen-2-ol, 3-ethyl-phenol, 3-morpholin-4-yl-phenol, 3-isopropyl-phenol, 3-isopropyl-5-methyl-phenol, 2-ethyl-phenol, 4-cyaohlxyl-phenol, and phenyl-methanol.

**12** in acetonitrile is then treated with sodium iodide (NaI) followed by chlorotrimethylsilane (TMSCI) to provide **14** (e.g., 5-methoxy-3-phenoxy-benzo[b]thiophene-2-carboxylic acid phenyl ester). **14** is then saponified with an inorganic base such as LiOH or NaOH in a solution of MeOH and THF; dioxane and water; or methanol and water, to provide **16** (e.g., 5-methoxy-3-phenoxy-benzo[*b*]thiophene-2-carboxylic acid). The carboxylic acid **16** is then treated with carbonyl diimidazole (CDI) in an aprotic solvent such as THF (tetrahydrofuran), followed by the addition of a 5-aminotetrazole to provide the carboxamide **18** (e.g., 5-methoxy-3-phenoxy-benzo[b]thiophene-2-carboxylic acid (2H-tetrazol-5-yl)-amide).

Alternatively, **16** (e.g., 5-methoxy-6-methyl-3-phenoxy-benzo[b]thiophene-2-carboxylic acid) in anhydrous CH₂Cl₂ can be treated with a catalytic amount of DMF followed by oxalyl chloride. Acetonitrile is then added to this mixture, followed by the addition of 5-aminotetrazole and triethylamine to give **18** (e.g., 5-methoxy-6-methyl-3-phenoxy-benzo[b]thiophene-2-carboxylic acid (2H-tetrazol-5-yl)-amide).

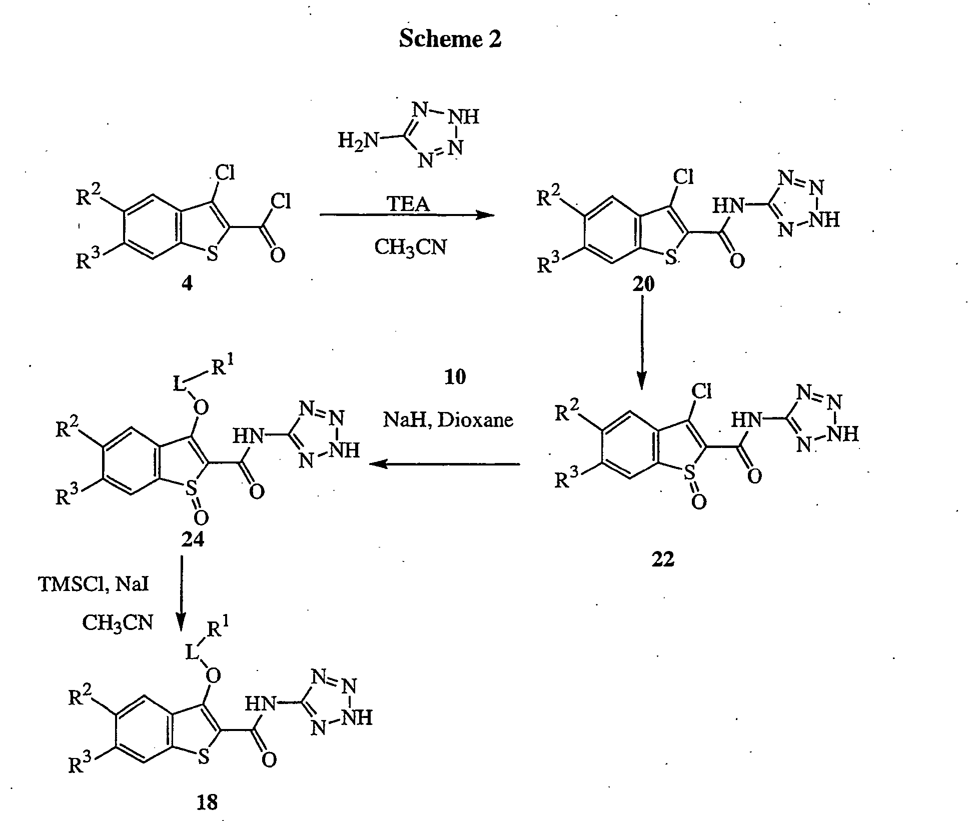

In Scheme 2, a compound **4** (e.g., 3-chloro-5-methoxy-benzo[b]thiophene-2-carbonyl chloride) is refluxed with 5-aminotetrazole and triethylamine (TEA) in acetonitrile (CH₃CN) to provide **20** (e.g., 3-chloro-5-methoxy-benzo[b]thiophene-2-carboxylic acid (2H-tetrazol-5-yl)-amide). **20** is then oxidized using aqueous H₂O₂ in CH₂Cl₂ and trifluoroacetic acid (TFA) to give **22** (e.g., 3-chloro-5-methoxy-1-oxo-benzo[*b*]thiophene-2-carboxylic acid (2H-tetrazol-5-yl)-amide.

**22** is mixed with **10** in dioxane and then reacted with 2 equivalents of NaH to give **24** (e.g., 3-(3,5-dimethyl-phenoxy)-5-methoxy-1-oxo-benzo[*b*]thiophene-2-carboxylic acid (2H-tetrazol-5-yl)-amide). **24** is then mixed with sodium iodide in acetonitrile followed by the addition of chlorotrimethylsilane (TMSCl) to give **26** (e.g., 3-(3,5-dimethyl-phenoxy)-5-methoxy -benzo[*b*]thiophene-2-carboxylic acid (2H-tetrazol-5-yl)-amide).

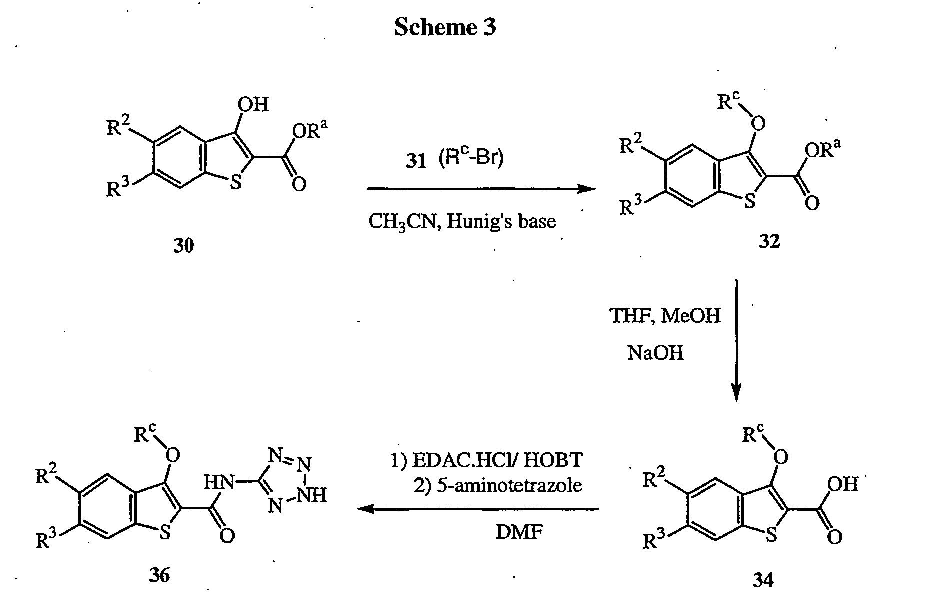

In Scheme 3, **30** (e.g., 3-Hydroxy-5,6-dimethoxy-benzo[b]thiophene-2-carboxylic acid methyl ester) in acetonitrile is treated with di-isopropyl ethyl amine (Hünig's base) followed by **31** (e.g., 3-bromo benzyl bromide) to provide 32 (e.g., 3-(3-bromo-benzyloxy)-5,6-dimethoxy-benzo[b]thiophene-2-carboxylic acid methyl ester). Compounds of formula **30,** e.g., 3-Hydroxy-5,6-dimethoxy-benzo[b]thiophene-2-carboxylic acid methyl ester, can be prepared according to procedures such as those described in U.S. Patent No. 3,954,748. Examples of **31** (R^{c}-Br) include, but are not limited to, 3,4-difluoro-benzylbromide, 4-bromomethyl-biphenyl, 1-bromomethyl-3-trifluoromethyl-benzene, 1-bromomethyl-3,5-dimethoxy-benzene, 1-bromomethyl-4-tert-butyl-benzene, 2-bromomethyl-1,3,4-trifluoro-benzene, and 2-bromomethyl-naphthalene. R^{c}-Br is a compound of formula R¹-L-Br, where L is a C₁-C₄ alkylene or a C₁-C₄-alkylene-C(O)- and R¹ has any one of values defined herein.

**32** is then saponified with an inorganic base as described in Scheme 1 to provide the carboxylic acid **34** (e.g., 3-(3-Bromo-benzyloxy)-5,6-dimethoxy-benzo[b]thiophene-2-carboxylic acid). **34** in DMF is then treated with EDAC.HCl (N-(3-dimethylaminopropyl)-N'-ethylcarbodiimide hydrochloride), HOBT (1-hydroxy-6-(trifluoromethyl)benzotriazole) and 5-aminotetrazole to provide the carboxamide **36** (e.g., 3-(3-bromo-benzyloxy)-5,6-dimethoxy-benzo[b]thiophene-2-carboxylic acid (1H-tetrazol-5-yl)-amide).

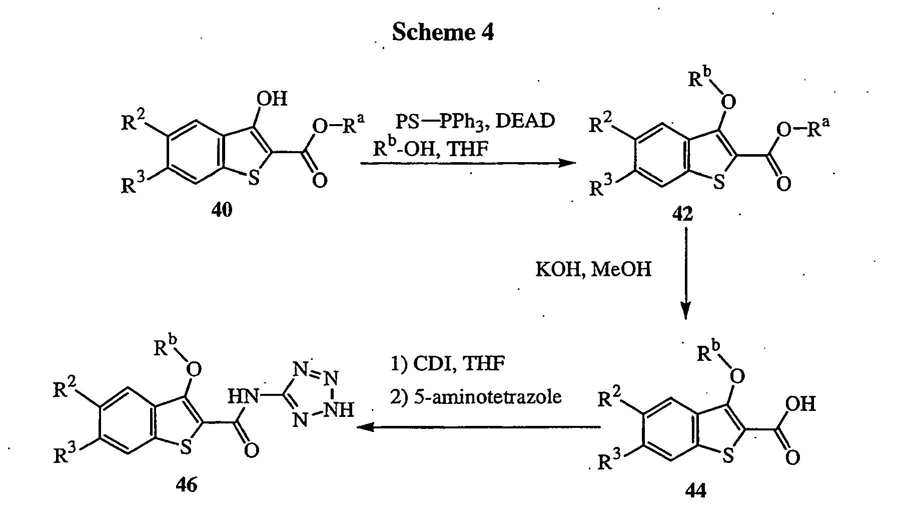

In Scheme 4, PS-triphenylphosphine (polystyrene-triphenylphosphine) is added to a solution of **40** (e.g., 3-hydroxy-5,6-dimethoxy-benzo[b]thiophene-2-carboxylic acid methyl ester) in THF under nitrogen gas. Diethyl azodicarboxylate (DEAD) is added, followed by the addition of R^{b}-OH to yield **42** (e.g., 5,6-dimethoxy-3-phenethyloxy-benzo[b]thiophene-2-carboxylic acid methyl ester). Compounds such as 3-hydroxy-5,6-dimethoxy-benzo[b]thiophene-2-carboxylic acid methyl ester can be prepared by the methods describe in U.S. Patent No. 3,954,748. R^{b}-OH is a compound of formula R¹-L-OH, where L is a C₁-C₄ alkylene or a C₁-C₄-alkylene-C(O)- and R¹ has any one of values defined herein.

The ester **42** in methanol is hydrolyzed using an inorganic base, such as potassium hydroxide, to yield the corresponding carboxylic acid **44** (e.g., 5,6-dimethoxy-3-phenethyloxy-benzo[b]thiophene-2-carboxylic acid). **44** is converted to the carboxamide **46** (e.g., 5,6-dimethoxy-3-phenethyloxy-benzo[b]thiophene-2-carboxylic acid (1H-tetrazol-5-yl)-amide) in an analogous manner to the transformation of **16** to **18** in Scheme 1.

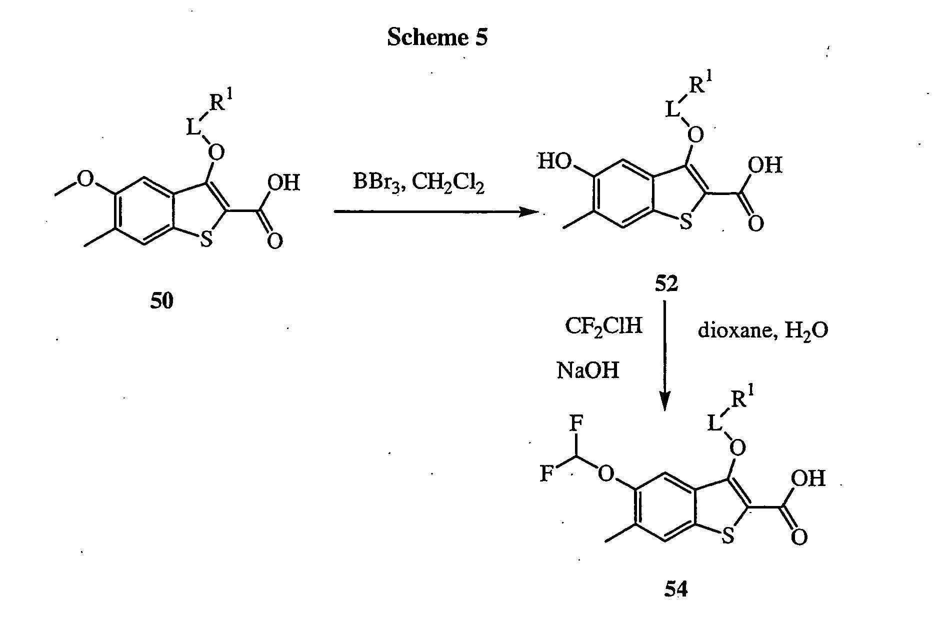

In Scheme 5, the 5-methoxy-6-methyl-benzo[b]thiophene **50** (e.g., 3-(4-cyclohexyl-phenoxy)-5-methoxy-6-methyl-benzo[b]thiophene-2-carboxylic acid) is coverted to the corresponding 5-hydroxy-6-methyl-benzo[b]thiophene **52** (e.g., 3-(4-cyclohexyl-phenoxy)-5-hydroxy-6-methyl-benzo[b]thiophene-2-carboxylic acid) using boron tribromide in anhydrous CH₂Cl₂.

The 5-difluoromethyl-6-methyl-benzo[b]thiophene **54** can be provided from **52.** The treatment of **52** with an inorganic base (e.g., NaOH) and chlorodifluoromethane (CF₂ClH) in dioxane and water yields **54** (e.g., 3-(4-cyclohexyl-phenoxy)-5-difluoromethoxy-6-methyl-benzo[*b*]thiophene-2-carboxylic acid). **52** or **54** are then coupled with a 5-aminotetrazole as described in Scheme 1 to give the corresponding aminotetrazole derivative of **52** (e.g., 3-(4-cyclohexyl-phenoxy)-5-hydroxy-6-methyl-benzo[*b*]thiophene-2-carboxylic acid (2H-tetrazol-5-yl)-amide) or **54** (e.g., 3-(4-cyclohexyl-phenoxy)-5-difluoromethoxy-6-methyl-benzo[*b*]thiophene-2-carboxylic acid (2H-tetrazol-5-yl)-amide), respectively.

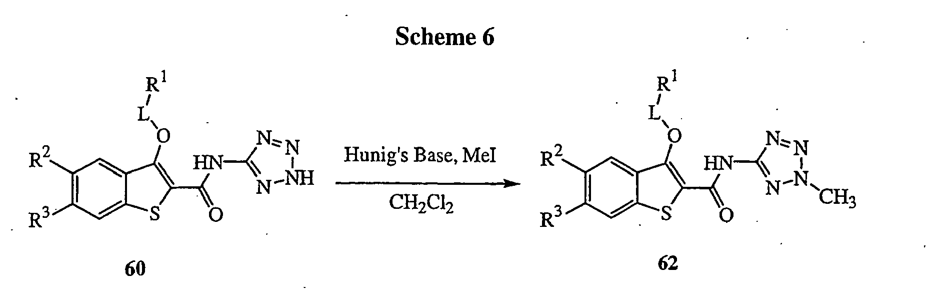

In Scheme 6, 2-methyl-2H-tetrazol-5-yl-amides of **62** can be generated from the 2H-tetrazol-5-yl-amides of formula **60.** The benzo[*b*]thiophene-2-carboxylic acid (2H-tetrazol-5-yl)-amide **60** (e.g., 3-(4-Cyclohexyl-phenoxy)-5-methoxy-6-methyl-benzo[*b*]thiophene-2-carboxylic acid (2H-tetrazol-5-yl)-amide) is treated with Hunig's base (diisopropyl ethyl amine), in dichloromethane followed by the addition of a methyl halide (e.g., methyl iodide) to provide **62** (e.g., 3-(4-Cyclohexyl-phenoxy)-5-methoxy-6-methyl-benzo[*b*]thiophene-2-carboxylic acid (2-methyl-2H-tetrazol-5-yl)-amide).

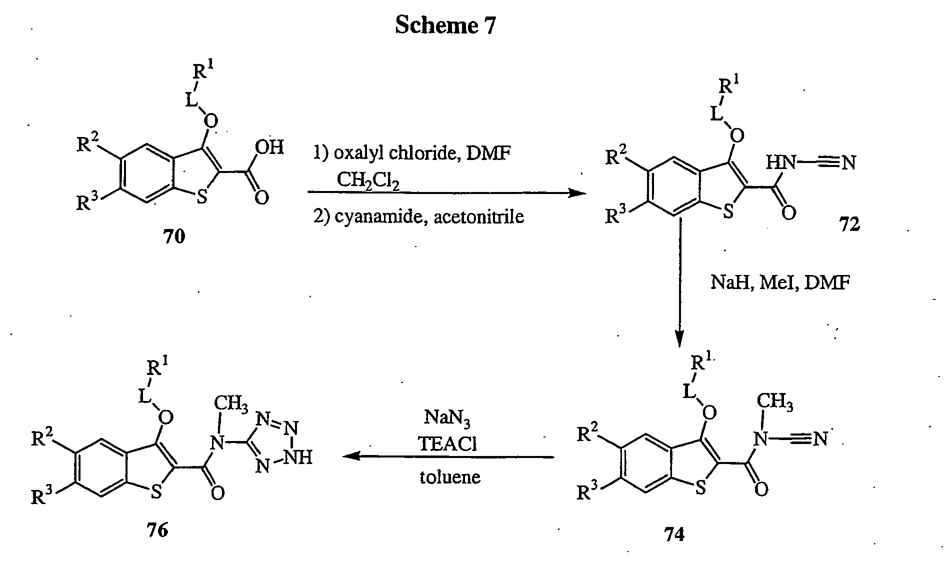

In Scheme 7, the benzo[b]thiophene-2-carboxylic acid **70** is treated in an analogous manner to the conversion of **16** to **18** in Scheme 1 using oxalyl chloride to form the acid chloride, followed by the addition of cyanamide instead of 5-amino-tetrazole to form a compound of formula **72** (e.g., 3-(4-cyclohexyl-phenoxy)-5-methoxy-6-methyl-N-cyano-benzo[b]thiophene-2-carboxamide).

**72** is then treated with a hydride base such as sodium hydride (NaH) and a methylhalide (e.g., methyl iodide (MeI)) in DMF to give **74** (e.g., 3-(4-cyclohexyl-phenoxy)-5-methoxy-6-miethyl- N-cyano-N-methyl-benzo[b]thiophene-2-carboxamide). **74** in toluene is then reacted with an azide (e.g., sodium azide, tri-*n*-butyltin azide, or trimethylsilyl azide, etc.) and triethyl ammonium chloride (TEACl) or ammonium chloride to provide the benzo[*b*]thiophene-2-carboxylic acid methyl-(2H-tetrazol-5-yl)-amide **76.**

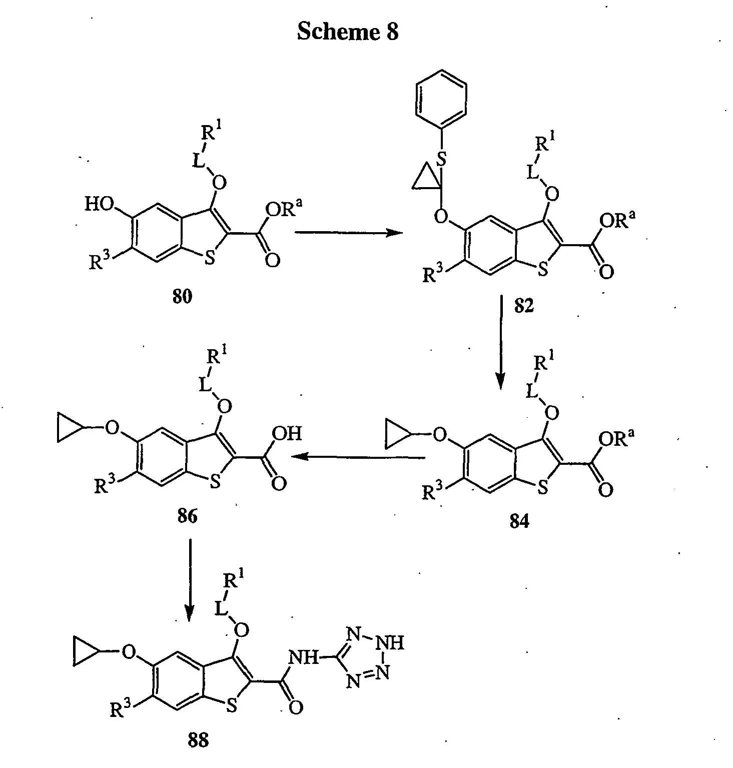

In Scheme 8, the 5-cyclopropyloxy substituted benzo[b]thiophene **88** can be generated as depicted. 6-cyclopropyloxy benzo[b]thiophenes can also be generated in an analogous manner. The 5-hydroxy-benzo[b]thiophene **80** can be subjected to a cyclopropanation procedure known in the art such as those described in United States Patent Application 20010029297. The benzothiophene **80** can be reacted with (1-iodo-cycloprop-1-yl)phenylsulfide and a base such as silver carbonate to yield **82. 82** can then be reacted with a metal naphthalenide (e.g., lithium naphthalenide) in an aprotic solvent such as THF or ether at a temperature of around -80°C to yield a compound of formula **84.** Alternatively, the phenylthio group of **82** can be oxidized with a reagent such as ozone in the presence of aluminium oxide, in a chlorinated hydrocarbon solvent (e.g., chloroform) at room temperature. The resulting phenylsulfonyl group can be removed with sodium amalgam in the presence of disodium hydrogen orthophosphate, in an alcohol solvent such as methanol to yield **84.** The ester **84** is then saponified as described in Scheme 1 to the acid **86. 86** can be coupled to an aminotetrazole as described in Scheme 1 to yield **88.**

### III. EVALUATION OF COMPOUNDS

Compounds of the present invention (e.g., compounds of Formulas I-XII and pharmaceutically acceptable salts thereof) can be assayed for their ability to inhibit a PI3K. Examples of these assays are set out below and include in vitro and in vivo assays of PI3K activity.

In certain embodiments of the present invention are compounds that selectively inhibit one or more PI3Ks as compared to one or more enzymes including, but not limited to, a cyclic nucleotide dependent protein kinase, PDGF, a tyrosine kinase, a MAP kinase, a MAP kinase kinase, a MEKK, a cyclin-dependent protein kinase. In other embodiments of the invention are compounds that selectively inhibit one PI3K as compared to another PI3K. For example, in certain embodiments, compounds of the present invention display the ability to selectively inhibit PI3Kγ as compared to PI3Kα or PI3Kβ. A compound selectively inhibits a first enzyme as compared to a second enzyme, when the IC₅₀ of the compound towards the first enzyme is less than the IC₅₀ of the compound towards the second compound. The IC₅₀ can be measured, for example, in an in vitro PI3K assay.

In presently preferred embodiments, compounds of the present invention can be assessed for their ability to inhibit PI3Kactivity in an in vitro or an in vivo assay (see below).

PI3K assays are carried out in the presence or absence of a PI3K inhibitory compound, and the amount of enzyme activity is compared for a determination of inhibitory activity of the PI3K inhibitory compound.

Samples that do not contain a PI3K inhibitory compound are assigned a relative PI3K activity value of 100. Inhibition of PI3K activity is achieved when the PI3K activity in the presence of a PI3K inhibitory compound is less than the control sample (i.e., no inhibitory compound). The IC₅₀ of a compound is the concentration of compound that exhibits 50% of the control sample activity. In certain embodiments, compounds of the present invention have an IC₅₀ of less than about 100 µM. In other embodiments, compounds of the present invention have an IC₅₀ of about 1 µM or less. In still other embodiments, compounds of the present invention have an IC₅₀ of about 200 nM or less.

PI3Kγ assays have been described in the art (see e.g., Leopoldt et al. J. Biol. Chem., 1998;273:7024-7029). Typically, a sample containing a complex of p101 and p110γ protein are combined with Gβ and Gγ proteins (e.g., G protein β₁/γ₂ subunits). Radiolabeled ATP (e.g., γ-³²P-ATP) is then added to this mixture. The lipid substrates are formed by creating PIP₂ containing lipid micelles. The reactions are then started by adding the lipid and enzyme mixtures and are stopped with the addition of H₃PO₄. The lipid products are then transferred to a glass fiber filter plate, and washed with H₃PO₄ several times. The presence of radioactive lipid product (PIP₃) can be measured using radiometric methods that are well-known in the art.

The activity of growth factor regulated PI3Ks can also be measured using a lipid kinase assay. For example, PI3Kα can be assayed using samples that contain a regulatory and a catalytic subunit. An activating peptide (e.g., pY peptide, SynPep Corp.) is added to the sample with radiolabeled ATP. PIP₂ containing lipid micelles are then added to the sample to start the reaction. The reactions are worked up and analyzed as described for the PI3Kγ assay just described. Assays can also be carried out using cellular extracts (Susa et al. J. Biol. Chem., 1992;267:22951-22956).

### IV. PHARMACEUTICALLY ACCEPTABLE SALTS AND SOLVATES

The compounds to be used in the present invention can exist in unsolvated forms as well as solvated forms, including hydrated forms. In general, the solvated forms, including hydrated forms, are equivalent to unsolvated forms and are intended to be encompassed within the scope of the present invention.

The compounds of the present invention (e.g., compounds of Formulas I-XII) are capable of further forming both pharmaceutically acceptable salts, including but not limited to acid addition and/or base salts. Pharmaceutically acceptable salts of the compounds of formula (I) include the acid addition and base salts (including disalts) thereof. Examples of suitable salts can be found for example in Stahl and Wermuth, Handbook of Pharmaceutical Salts: Properties, Selection, and Use, Wiley-VCH, Weinheim, Germany (2002); and Berge et al., "Pharmaceutical Salts," J. of Pharmaceutical Science, 1977;66:1-19.

Pharmaceutically acceptable acid addition salts of the compounds of Formulas I-XII include non-toxic salts derived from inorganic acids such as hydrochloric, nitric, phosphoric, sulfuric, hydrobromic, hydriodic, phosphorus, and the like, as well as the salts derived from organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include the acetate, aspartate, benzoate, besylate (benzenesulfonate), bicarbonate/carbonate, bisulfate, caprylate, camsylate (camphor sulfonate), chlorobenzoate, citrate, edisylate (1,2-ethane disulfonate), dihydrogenphosphate, dinitrobenzoate, esylate (ethane sulfonate), fumarate, gluceptate, gluconate, glucuronate, hibenzate, hydrochloride/chloride, hydrobromide/bromide, hydroiodide/iodide, isobutyrate, monohydrogen phosphate, isethionate, D-lactate, L-lactate, malate, maleate, malonate, mandelate, inesylate (methanesulfonate), metaphosphate, methylbenzoate, methylsulfate, 2-napsylate (2-naphthalene sulfonate), nicotinate, nitrate; orotate, oxalate, palmoate, phenylacetate, phosphate, phthalate, propionate, pyrophosphate, pyrosulfate, saccharate, sebacate, stearate, suberate, succinate sulfate, sulfite, D-tartrate, L-tartrate, tosylate (toluene sulfonate), and xinafoate salts, and the like of compounds of Formulas I-XII. Also contemplated are the salts of amino acids such as arginate, gluconate, galacturonate, and the like.

The acid addition salts of the basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metal hydroxides, or of organic amines. Examples of metals used as cations are aluminium, calcium, magnesium, potassium, sodium, and the like. Examples of suitable amines include arginine, choline, chloroprocaine, N,N'-dibenzylethylenediamine, diethylamine, diethanolamine, diolamine, ethylenediamine (ethane-1,2-diamine), glycine, lysine, meglumine, N-methylglucamine, olamine, procaine (benzathine), and tromethamine.

The base addition salts of acidic compounds are prepared by contacting the free acid form with a sufficient amount of the desired base to produce the salt in the conventional manner. The free acid form may be regenerated by contacting the salt form with an acid and isolating the free acid in a conventional manner. The free acid forms differ from their respective salt forms somewhat in certain physical properties such as solubility in polar solvents, but otherwise the salts are equivalent to their respective free acid for purposes of the present invention.

### V. PHARMACEUTICAL COMPOSITIONS AND METHODS OF ADMINISTRATION

This invention also provides for pharmaceutical compositions comprising a therapeutically effective amount of a compound of Formulas I-XII, or a pharmaceutically acceptable salt thereof together with a pharmaceutically acceptable carrier, diluent, or excipient therefor. The phrase "pharmaceutical composition" refers to a composition suitable for administration in medical or veterinary use. The phrase "therapeutically effective amount" means an amount of a compound, or a pharmaceutically acceptable salt thereof, sufficient to inhibit, halt, or allow an improvement in the disorder or condition being treated when administered alone or in conjunction with another pharmaceutical agent or treatment in a particular subject or subject population. For example in a human or other mammal, a therapeutically effective amount can be determined experimentally in a laboratory or clinical setting, or may be the amount required by the guidelines of the United States Food and Drug Administration, or equivalent foreign agency, for the particular disease and subject being treated.

It should be appreciated that determination of proper dosage forms, dosage amounts, and routes of administration is within the level of ordinary skill in the pharmaceutical and medical arts, and is described below.

A compound of the present invention can be formulated as a pharmaceutical composition in the form of a syrup, an elixir, a suspension, a powder, a granule, a tablet, a capsule, a lozenge, a troche, an aqueous solution, a cream, an ointment, a lotion, a gel, an emulsion, etc. Preferably, a compound of the present invention will cause a decrease in symptoms or a disease indicia associated with a PI3K-mediated disorder as measured quantitatively or qualitatively.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component. In tablets, the active component is mixed with the carrier having the necessary binding properties in suitable proportions and compacted in the shape and size desired.

The powders and tablets contain from 1% to 95% (w/w) of the active compound. In certain embodiments, the active compound ranges from 5% to 70% (w/w). Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized molds, allowed to cool, and thereby to solidify.

Liquid form preparations include solutions, suspensions, and emulsions, for example, water or water/propylene glycol solutions. For parenteral injection, liquid preparations can be formulated in solution in aqueous polyethylene glycol solution.

Aqueous solutions suitable for oral use can be prepared by dissolving the active component in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampules. Also, the unit dosage form can be a capsule, tablet, cachet, or lozenge itself, or it can be the appropriate number of any of these in packaged form.

The quantity of active component in a unit dose preparation may be varied or adjusted from 0.1 mg to 1000 mg, preferably 1.0 mg to 100 mg, or from 1% to 95% (w/w) of a unit dose, according to the particular application and the potency of the active component. The composition can, if desired, also contain other compatible therapeutic agents.

Pharmaceutically acceptable carriers are determined in part by the particular composition being administered, as well as by the particular method used to administer the composition. Accordingly, there is a wide variety of suitable formulations of pharmaceutical compositions of the present invention (see, e.g., Remington: The Science and Practice of Pharmacy, 20th ed., Gennaro et al. Eds., Lippincott Williams and Wilkins, 2000).

A compound of the present invention, alone or in combination with other suitable components, can be made into aerosol formulations (i.e., they can be "nebulized") to be administered via inhalation. Aerosol formulations can be placed into pressurized acceptable propellants, such as dichlorodifluoromethane, propane nitrogen, and the like.

Formulations suitable for parenteral administration, such as, for example, by intravenous, intramuscular, intradermal, and subcutaneous routes, include aqueous and non-aqueous, isotonic sterile injection solutions, which can contain antioxidants, buffers, bacteriostats; and solutes that render the formulation isotonic with the blood of the intended recipient, and aqueous and nonaqueous sterile suspensions that can include suspending agents, solubilizers, thickening agents, stabilizers, and preservatives. In the practice of this invention, compositions can be administered, for example, by intravenous infusion, orally, topically, intraperitoneally, intravesically or intrathecally. The formulations of compounds can be presented in unit-dose or multi-dose sealed containers, such as ampules and vials. Injection solutions and suspensions can be prepared from sterile powders, granules, and tablets of the kind previously described.

The dose administered to a subject, in the context of the present invention should be sufficient to affect a beneficial therapeutic response in the subject over time. The term "subject" refers to a member of the class Mammalia. Examples of mammals include, without limitation, humans, primates, chimpanzees, rodents, mice, rats, rabbits, horses, livestock, dogs, cats, sheep, and cows.

The dose will be determined by the efficacy of the particular compound employed and the condition of the subject, as well as the body weight or surface area of the subject to be treated. The size of the dose also will be determined by the existence, nature, and extent of any adverse side-effects that accompany the administration of a particular compound in a particular subject. In determining the effective amount of the compound to be administered in the treatment or prophylaxis of the disorder being treated, the physician can evaluate factors such as the circulating plasma levels of the compound, compound toxicities, and/or the progression of the disease, etc. In general, the dose equivalent of a compound is from about 1 µg/kg to 100 mg/kg for a typical subject. Many different administration methods are known to those of skill in the art.

For administration, compounds of the present invention can be administered at a rate determined by factors that can include, but are not limited to, the LD₅₀ of the compound, the pharmacokinetic profile of the compound, contraindicated drugs, and the side-effects of the compound at various concentrations, as applied to the mass and overall health of the subject. Administration can be accomplished via single or divided doses.

Examples of a typical tablet, parenteral, and patch formulation include the following:

### TABLET FORMULATION EXAMPLE 1

| Tablet Formulation | |
|---|---|
| Ingredient | Amount |
| Compound of Formulas I-XII | 50 mg |
| Lactose | 80 mg |
| Cornstarch (for mix) | 10 mg |
| Cornstarch (for paste) | 8 mg |
| Magnesium Stearate (1%) | 2 mg |
| | 150 mg |

The compounds of the present invention (e.g., a compound of Formulas I-XII, or a pharmaceutically acceptable salt thereof) can be mixed with the lactose and cornstarch (for mix) and blended to uniformity to a powder. The cornstarch (for paste) is suspended in 6 mL of water and heated with stirring to form a paste. The paste is added to the mixed powder, and the mixture is granulated. The wet granules are passed through a No. 8 hard screen and dried at 50°C. The mixture is lubricated with 1% magnesium stearate and compressed into a tablet. The tablets are administered to a patient at the rate of 1 to 4 each day for treatment of a PI3K-mediated disorder or condition.

### PARENTERAL SOLUTION FORMULATION EXAMPLE 1

In a solution of 700 mL of propylene glycol and 200 mL of water for injection can be added 20.0 g of a compound of the present invention. The mixture is stirred, and the pH is adjusted to 5.5 with hydrochloric acid. The volume is adjusted to 1000 mL with water for injection. The solution is sterilized, filled into 5.0 mL ampules, each containing 2.0 mL (40 mg of invention compound), and sealed under nitrogen. The solution is administered by injection to a subject suffering from a PI3K-mediated disorder or condition and in need of treatment..

### PATCH FORMULATION EXAMPLE 1

Ten milligrams of a compound of the present invention can be mixed with 1 mL of propylene glycol and 2 mg of acrylic-based polymer adhesive containing a resinous cross-linking agent. The mixture is applied to an impermeable backing (30 cm²) and applied to the upper back of a patient for sustained release treatment of a PI3K-mediated disorder or condition.

### V. METHODS FOR TREATING PI3K-MEDIATED DISORDERS AND CONDITIONS

The compounds of the present invention and pharmaceutical compositions comprising a compound of the present invention can be administered to a subject suffering from a PI3K-mediated disorder or condition. PI3K-mediated disorders and conditions can be treated prophylactically, acutely, and chronically using compounds of the present invention, depending on the nature of the disorder or condition. Typically, the host or subject in each of these methods is human, although other mammals can also benefit from the administration of a compound of the present invention.

In therapeutic applications, the compounds of the present invention can be prepared and administered in a wide variety of oral and parenteral dosage forms. The term "administering" refers to the method of contacting a compound with a subject. Thus, the compounds of the present invention can be administered by injection, that is, intravenously, intramuscularly, intracutaneously, subcutaneously, intraduodenally, parentally, or intraperitoneally. Also, the compounds described herein can be administered by inhalation, for example, intranasally. Additionally, the compounds of the present invention can be administered transdermally, topically, via implantation, transdermally, topically, and via implantation. In certain embodiments, the compounds of the present invention are delivered orally. The compounds can also be delivered rectally, bucally, intravaginally, ocularly, andially, or by insufflation.

The compounds utilized in the pharmaceutical method of the invention can be administered at the initial dosage of about 0.001 mg/kg to about 100 mg/kg daily. In certain embodiments, the daily dose range is from about 0.1 mg/kg to about 10 mg/kg. The dosages, however, may be varied depending upon the requirements of the subject, the severity of the condition being treated, and the compound being employed. Determination of the proper dosage for a particular situation is within the skill of the practitioner. Generally, treatment is initiated with smaller dosages, which are less than the optimum dose of the compound. Thereafter, the dosage is increased by small increments until the optimum effect under circumstances is reached. For convenience, the total daily dosage may be divided and administered in portions during the day, if desired. The term "treatment" includes the acute, chronic, or prophylactic diminishment or alleviation of at least one symptom or characteristic associated with or caused by the disorder being treated. For example, treatment can include diminishment of several symptoms of a disorder, inhibition of the pathological progression of a disorder, or complete eradication of a disorder. The compounds of the present invention can be co-administered to a subject. The term "co-administered" means the adminstration of two or more different pharmaceutical agents or treatments (e.g., radiation treatment) that are administered to a subject by combination in the same pharmacetical composition or separate pharamaceutical compositions. Thus co-adminstration involves adminstration at the same time of a single pharmaceutical composition comprising two or more pharmaceutical agents or administration of two or more different compositions to the same subject at the same or different times. For example, a subject that is administered a first dosage that comprises a compound of the present invention at 8 a.m. and then is adminstred CELEBREX® at 1-12 hours later, e.g., 6 p.m., of that same day has been co-administered with a compound of the present invention and CELEBREX®. Alternatively, for example, a subject could be administred with a single dosage comprising a compound of the present invention and CELEBREX ® at 8 a.m. has been co-administered with a compound of the present invention and CELEBREX®.

Thus, compounds of the invention can also be co-administered with compounds that are useful for the treatment of cancer (e.g., cytotoxic drugs such as TAXOL®, taxotere, GLEEVEC® (Imatinib Mesylate), adriamycin, daunomycin, cisplatin, etoposide, a vinca alkaloid, vinblastine, vincristine, methotrexate, or adriamycin, daunomycin, cis-platinum, etoposide, and alkaloids, such as vincristine, farnesyl transferase inhibitors, endostatin and angiostatin, VEGF inhibitors, and antimetabolites such as methotrexate. The compounds of the present invention may also be used in combination with a taxane derivative, a platinum coordination complex, a nucleoside analog, an anthracycline, a topoisomerase inhibitor, or an aromatase inhibitor). Radiation treatments can also be co-administered with a compound of the present invention for the treatment of cancers.

The compounds of the invention can also be co-administered with compounds that are useful for the treatment of a thrombolytic disease, heart disease, stroke, etc., (e.g., aspirin, streptokinase, tissue plasminogen activator, urokinase, anticoagulants, antiplatelet drugs (e.g., PLAVIX®; clopidogrel bisulfate), a statin (e.g., LIPITOR® (Atorvastatin calcium), ZOCOR® (Simvastatin), CRESTOR® (Rosuvastatin), etc.), a Beta blocker (e.g, Atenolol), NORVASC® (amlodipine besylate), and an ACE inhibitor (e.g., Accupril® (Quinapril Hydrochloride), Lisinopril, etc.).

The compounds of the invention can also be co-administered for the treatment of hypertension with compounds such as ACE inhibitors, lipid lowering agents such as statins, LIPITOR® (Atorvastatin calcium), calcium channel blockers such as NORVASC® (amlodipine besylate). The compounds of the present invention may also be used in combination with fibrates, beta-blockers, NEPI inhibitors, Angiotensin-2 receptor antagonists and platelet aggregation inhibitors.

For the treatment of inflammatory diseases, including rheumatoid arthritis, the compounds of the invention may be co-administered with agents such as TNF-α inhibitors such as anti-TNFα monoclonal antibodies (such as REMICADE®, CDP-870 and HUMIRA^{™} (adalimumab) and TNF receptor-immunoglobulin fusion molecules (such as ENBREL®), IL-1 inhibitors, receptor antagonists or soluble IL-1Rα (e.g. KINERET^{™} or ICE inhibitors), nonsteroidal anti-inflammatory agents (NSAIDS), piroxicam, diclofenac, naproxen, flurbiprofen, fenoprofen, ketoprofen ibuprofen, fenamates, mefenamic acid, indomethacin, sulindac, apazone, pyrazolones, phenylbutazone, aspirin,COX-2 inhibitors (such as CELEBREX® (celecoxib), VIOXX® (rofecoxib), BEXTRA® (valdecoxib) and etoricoxib, metalloprotease inhibitors (preferably MMP-13 selective inhibitors), NEUROTIN®, pregabalin, low dose methotrexate, leflunomide, hydroxychloroquine, d-penicillamine, auranofin or parenteral or oral gold.

The compounds of the invention may be co-administered with existing therapeutic agents for the treatment of osteoarthritis. Suitable agents to be used in combination include standard non-steroidal anti-inflammatory agents (hereinafter NSAID's) such as piroxicam, diclofenac, propionic acids such as naproxen, flurbiprofen, fenoprofen, ketoprofen and ibuprofen, fenamates such as mefenamic acid, indomethacin, sulindac, apazone, pyrazolones such as phenylbutazone, salicylates such as aspirin, COX-2 inhibitors such as celecoxib, valdecoxib, rofecoxib and etoricoxib, analgesics and intraarticular therapies such as corticosteroids and hyaluronic acids such as hyalgan and synvisc.

The compounds of the invention may also be co-administered with antiviral agents such as Viracept, AZT, aciclovir and famciclovir, and antisepsis compounds such as Valant.

The compounds of the present invention may further be co-administered with CNS agents such as antidepressants (such as sertraline), anti-Parkinsonian drugs (such as deprenyl, L-Dopa, Requip, Mirapex, MAOB inhibitors such as selegine and rasagiline, comP inhibitors such as Tasmar, A-2 inhibitors, dopamine reuptake inhibitors, NMDA antagonists, Nicotine agonists, Dopamine agonists and inhibitors of neuronal nitric oxide synthase), and anti-Alzheimer's drugs such as donepezil, tacrine, NEUROTIN®, pregabalin, COX-2 inhibitors, propentofylline or metryfonate.

The compounds of the present invention may additionally be co-administered with osteoporosis agents such as EVISTA® (raloxifene hydrochloride) droloxifene, lasofoxifene or fosomax and immunosuppressant agents such as FK-506 and rapamycin.

### EXAMPLES

### EXAMPLES A.1-A.16

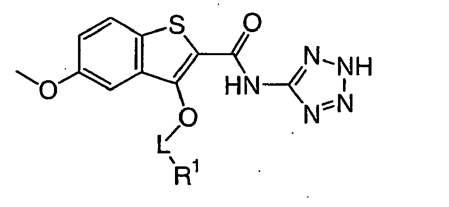

| Ex. | -L-R¹ | MS (M+1) | NMR*^{a}* |
|---|---|---|---|
| A.1 | 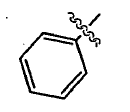 | 368.2 | 16.10 (s,1H), 11.70 (s, 1H), 8.01 (d, *J* = 9.0 Hz, 1H), 7.34 (m, 2H), 7.22 (m, 1H), 7.07 (m, 3H), 6.81 (d, *J* = 2.2 Hz, 1H), 3.64 (s, 3H) |

| | | | |
|---|---|---|---|
| ^{a 1}H-NMR (400 MHz, CDCl₃) | | | |

| Ex. | -L-R¹ | MS (M+1) | NMR^{a} |
|---|---|---|---|
| A.2 | 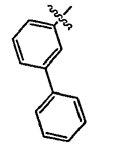 | 444.3 | 16.10 (s,1H), 11.81 (s,1H), 8.02 (d, *J* = 9.2 Hz, 1H), 7.57 (m, 2H), 7.40 (m, 6H), 7.23 (dd, *J* = 2.4, 8.8 Hz, 1H), 6.97 (m, 1H), 6.91 (d, *J* =2.4 Hz, 1H), 3.65 (s, 3H) |
| A.3 | 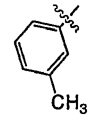 | 382.2 | 16.09 (s, 1H), 11.74 (s, 1H), 8.01 (d, *J* = 11.6, 1H), 7:21 (m, 2H), 6.91 (m, 2H), 6.81 (m,2H), 3.65 (s, 3H), 2.23 (s, 3H) |
| A.4 | 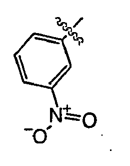 | 413.2 | 16.09 (s, 1H), 12.16 (s, 1H), 8.07 (d, *J* = 8.8 Hz, 1H), 7.95 (m, 1H), 7.79 (t, *J* = 2.4 Hz, 1H), 7.61 (t, *J* = 8.4 Hz, 1H), 7.44 (m, 1H), 7.26 (dd, *J* = 2.4, 8.8 Hz, 1H), 6.96 (d, *J* = 2.4, 1H), 3.89 (s, 3H) |
| A.5 | 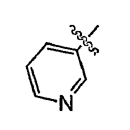 | 369.2 | 16.12 (s, 1H), 12.09 (s, 1H), 8.45 (t, *J*= 1.8 Hz, 1H), 8.30 (t, *J* = 3 Hz, 1H), 8.05 (d, *J* = 9.2 Hz, 1H), 7.34 (t, *J* = 2.4 Hz, 2H), 7.25 ( dd, *J*= 2.4, 9.2,1H), 6.91 (d, J = 2.4 Hz, 1H), 3.68 (s, 1H) |
| A.6 | 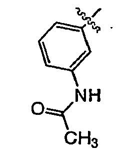 | 425.2 | 16.09 (s, 1H), 11.75 (s, 1H 6.96 (s, 1H), 8.02 (d, *J* = 8.8 Hz, 1H), 7.28 (m, 4H), 6.85 (s, 1H), 6.71 (dd, *J* = 8 Hz, 1H), 3.66 (s, 3H), 1.96 (s, 3H) |
| A.7 | 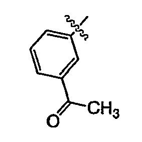 | 408.2 (M-1) | 16.00 (2,1H), 11.97(s, 1H), 8.04 (d, *J* = 8.8 Hz, 1H), 7.70 (d, *J* = 7.2 Hz, 1H), 7.49 (m, 2H), 7.26 (t, *J* = 8.8 Hz, 2H), 6.88 (s, 1H), 3.66 (s, 3H), 2.52 (s, 3H) |
| A.8 | 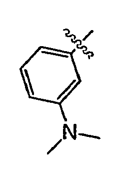 | 411.2 | 11.61 (s, 1H), 8.99 (d, *J* = 9.2 Hz, 1H), 7.22 (dd, *J* =2.4, 8.8 Hz, 1H), 7.06 (t, *J* = 8.4 Hz, 1H), 6.89 (s, 1H), 6.53 (s, 1H), 6.45 (d, *J* = 8.4 Hz, 1H), 6.14 (d, *J* = 8.0 Hz, 1H), 3.66 (s, 3H), 2.84 (s, 6H) |
| A.9 | 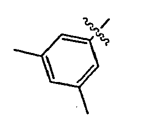 | 396.1 | 16.08 (s, 1H), 11.71 (s, 1H), 8:01 (d, *J* = 8.8 Hz, 1H), 7.22 (dd, *J* = 2.0, 9.2 Hz, 1H), 6.85 (s, 1H), 6.73 (s, 1H), 6.66 (s, 2H), 3.66 (s, 3H), 2.18 (s, 6H) |
| A.10 | 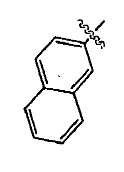 | 418.1 | 16.08 (s, 1H), 11.88 (s, 1H), 8.05 (d, *J* = 8.8 Hz, 1H), 7.96 (d, *J* = 8.8 Hz, 1H), 7.88 (d, *J* = 7.6 Hz, 1H), 7.74 (d, 8.0 Hz, 1H), 7.42 (m, 3H), 7.30 (s, 1H), 7.24 (m, 1H), 6.87 (s, 1H), 3.59 (s, 3H) |
| A.11 | 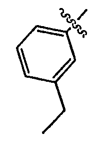 | 396.1 | 16.10 (s, 1H), 11.73 (s, 1H), 8.01 (d, *J* = 8.8 Hz, 1H), 7.22 (m, 2H), 6.95 (m, 2H), 6.80 (m, 2H), 3.64 (s, 3H), 2.53 (q, *J* = 7.6 Hz, 2H), 1.08 (t, *J* = 7.6 Hz, 3H) |
| A.12 | 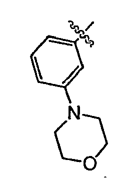 | 453.1 | 16.10 (s, 1H), 11.65 (s, 1H), 7.97 (d, *J =* 9.2 Hz, 1H), 7.20 (dd, *J* = 2.6, 9.0 Hz, 1H), 7.10 (t, *J* = 8 Hz, 1H), 6.84 (d, *J* = 2.4 Hz, 1H) 6.73 (t, *J* = 2.2 Hz, 1H), 6.66 (dd, *J* = 1.8, 8.4 Hz, 1H), 6.30 (dd, J = 2.0, 8.0 Hz, 1H), 3.65 (m, 7H), 3.02 (t, *J* = 4.8 Hz, 4H) |
| A.13 | 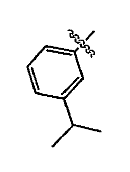 | 410.1 | 16.08 (s, 1H), 11.71 (s, 1H), 7.99 (d, J = 9.2 Hz, 1H), 7.22 (m, 2H), 7.05 (s; 1H), 6.98 (d, *J* = 7.6 Hz, 1H), 6.81 (s, 1H), 6.76 (d, *J* = 8.0 Hz, 1H), 3.63 (s, 3H), 2.83 (m, 1H), 1.11 (d, *J* = 6.8 Hz, 6H) |
| A.14 | 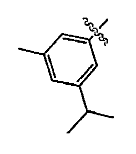 | 424.1 | 16.06 (s, 1H), 11.64 (s, 1H), 7.99 (d, *J* = 8.4 Hz, 1H), 7.21 (d, *J* = 9.2 Hz, 1H), 6.82 (m, 3H), 6.61 (s, 1H), 3.64 (s, 3H), 2.76 (m, 1H), 2.18 (s, 3H), 1.09 (d, *J* = 6.8 Hz, 6H) |
| A.15 | 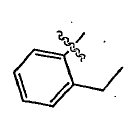 | 396.1 | 16.12 (s, 1H), 11.60 (s, 1H), 8.01 (d, *J* = 9.2 Hz, 1H), 7.34 (t, *J* = 4.4 Hz, 1H), 7.21 (d, *J* = 8.8 Hz, 1H), 7.05 (m, 2H), 6.60 (m, 2H), 3.58 (s, 3H), 2.91 (q, *J* = 7.6 Hz, 2H), 1.30 (t, *J*= 7.6 Hz, 3H) |
| A.16 | 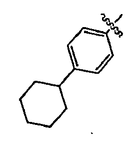 | 450.1 | 16.10 (s, 1H), 11.64 (s, 1H), 7.99 (d, *J* = 8.4 Hz, 1H), 7.19 (m, 3H), 6.96 (d, *J*= 8.0 Hz, 2H), 6.78 (s, 1H), 3.61 (s, 3H), 2.43 (m, 1H), 1.70 (m, 5H), 1.18-1.36 (m, 5H) |

| | | | |
|---|---|---|---|
| ^{a 1}H-NMR (400 MHz, CDCl₃) | | | |

Intermediate 1 **-3-Chloro-5-methoxy-benzo[b]thiophene-2-carbonyl chloride.** Methoxycinnamic acid (15.0 g, 84.3 mmol) was dissolved in a mixture of pyridine (0.643 mL, 7.96 mmol), DMF (6.16 mL, 80.0 mmol), and chlorobenzene (105 mL) in an argon purged, round bottom flask fitted with a reflux condenser. Thionyl chloride (31.4 mL, 430 mmol) was added to the mixture via syringe. The reaction was stirred and heated to vigorous reflux for 48 hours. The reaction was allowed to cool to room temperature and then was concentrated *en vacuo.* The residue was dissolved in CH₂Cl₂ (~40 mL) and then was diluted with an excess of hexanes. The dilution was concentrated to about one half volume to give a precipitate. The solid precipitate was filtered, collected, and dried *en vacuo* to give 3-Chloro-5-methoxy-benzo[b]thiophene-2-carbonyl chloride (Intermediate 1, 9.8 g, 45% yield) as an orange solid. ¹H-NMR (400 MHz, CDCl₃) δ 7.71 (d, *J* = 8.9 Hz, 1H), 7.33 (d, *J* = 2.2, 11H), 7.26 (dd, *J* = 8.2, 2.4 Hz, 1H), 3.93 (s, 3H).

Intermediate 2 - **3-chloro-5-methoxy-benzo[b]thiophene-2-carboxylic acid phenyl ester**. Intermediate 1 (9.8 g, 38 mmol) was stirred with phenol (17.7 g, 189 mmol), pyridine (~6mL), and 4-dimethylaminopyridine (400 mg, 3.3 mmol) in CH₂Cl₂ (47 mL) at room temperature under ambient atmosphere for six hours. The reaction mix was concentrated. The residue was dissolved in EtOAc (ethyl acetate), washed with 5% aqueous citric acid, followed by a brine wash, then dried over Na₂SO₄ and concentrated. The product was purified by silica gel flash chromatography [CH₂Cl₂/hexanes (1:4)] to give 3-chloro-5-methoxy-benzo[*b*]thiophene-2-carboxylic acid phenyl ester (Intermediate 2, 7.879 g, 66% yield) as a solid. MS: M+1=319.1 (APCI). ¹H-NMR (400 MHz, CDCl₃) δ 7.73 (d, *J* = 9.0 Hz, 1H), 7.45 (m, 2H), 7.38 (d, *J* = 2.4 Hz, 1H), 7.26 (m, 4H), 3.94 (s, 3H).

Intermediate 3 - **3-chloro-5-methoxy-1-oxo-benzo[*b*]thiophene-2-carboxylic acid phenyl ester.** To a 0 °C, stirring solution of Intermediate 2 (6.00 g, 18.9 mmol) in CH₂Cl₂ (20 mL) and Trifluoroacetic acid (20 mL) was added 30% aq. H₂O₂ (6.4 mL, 56.6 mmol) dropwise via syringe. The reaction was stirred at 0 °C for 15 minutes and then at room temperature for 90 minutes. The mix was chilled again to 0 °C and then added dropwise to a 0 °C saturated aq. sodium bisulfite solution. The quenched mix was extracted several times with EtOAc. The extracts were washed with brine, dried over Na₂SO₄, and concentrated. The product was purified by silica gel flash chromatography [EtOAc/hexanes (1:4 then 2:3)] to give 3-chloro-5-methoxy-1-oxo-benzo[b]thiophene-2-carboxylic acid phenyl ester (Intermediate 3, 3.01 g, 48 % yield) as a solid. MS: M+1=335.1 (APCI). ¹H-NMR (400 MHz, CDCl₃) δ 7.89 (d, *J* = 8.5 Hz, 1H), 7.44 (m, 2H), 7.28 (m, 4H), 7.22 (dd, 2.44, 8.5 Hz, 1H), 3.96 (s, 3H). Microanalysis (C₁₆H₁₁O₄SCl): calculated: C, 57.40; H, 3.31; N, 0.00. Found: C, 57.39; H, 3.16, N, <0.05.

Intermediate 4- **5-methoxy-1-oxo-3-phenoxy-benzo[*b*]thiophene-2-carboxylic acid phenyl ester.** To a stirring solution of phenol (0.102 g, 1.09 mmol) in dioxane (2 mL), was added sodium hydride (0.0237 g, 0.988 mmol). The reaction was stirred at room temperature for fifteen minutes and then was added dropwise to a heterogeneous mixture of Intermediate 3 (0.300 g, 0.898 mmol) in dioxane (2 mL). The mix became homogeneous. The reaction was stirred at room temperature for 30 minutes and then was quenched with water. The mix was extracted several times with EtOAc. The organic extracts were washed with brine, dried over Na₂SO₄, filtered through celite, and concentrated. The product was purified by silica gel flash chromatography [EtOAc/CH₂Cl₂ (1:49 then 1:20)] to give 5-methoxy-1-oxo-3-phenoxy-benzo[*b*]thiophene-2-carboxylic acid phenyl ester (Intermediate 4, 0.167 g, 47 % yield) as a clear glass. MS: M+1=393.2 (APCI). ¹H-NMR (400 MHz, CDCl₃) δ 7.90 (d, *J*= 8.3 Hz, 1H), 7.28 (m, 9H), 6.95 (m, 3H), 3.80 (s, 3H). Anal. Calcd for C₂₂H₁₆O₅S : C, 67.33; H, 4.11; N, 0.00. Found: C, 67.02; H, 4.14, N, <0.05.

**Intermediate 5- 5-methoxy-3-phenoxy-benzo[b]thiophene-2-carboxylic acid phenyl ester.** To a stirring solution of Intermediate 4 (0.157 g, 0.400 mmol) in acetonitrile (2 mL) was added sodium iodide (0.180 g, 1.20 mmol). The mix was stirred at room temperature for five minutes and then chilled to 0 °C in an ice-water bath. Chlorotrimethylsilane (0.101 mL, 0.800 mmol) was added via syringe. The ice-water bath was removed and the reaction was stirred at room temperature ten minutes. The reaction mix was diluted with 10% sodium thiosulfate aq. (~40 mL) and was extracted several times with EtOAc. The organic extracts were washed with brine, dried over Na₂SO₄, filtered through celite, and concentrated. The product was purified by silica gel flash chromatography [5%EtOAc-hexanes] to give 5-methoxy-3-phenoxy-benzo[b]thiophene-2-carboxylic acid phenyl ester (Intermediate 5, 0.126g, 84 % yield). MS: M+1=377.2 (APCI). ¹H-NMR (400 MHz, CDCl₃) δ 7.73 (d, *J* = 9.0, 1H), 7.31 (m, 5H), 7.20 (m, 2H), 7.06 (m, 2H), 6.99 (m, 3H), 3.70 (s, 3H).

Intermediate 6- **5-methoxy-3-phenoxy-benzo[*b*]thiophene-2-carboxylic acid**. Intermediate 5 (0.120 g, 0.319 mmol) was stirred with 1.0 M LiOH aq. (0.638 mL, 0.638 mmol), MeOH (0.25 mL), and THF (2.25 mL) at room temperature for 16 hours. The mix was diluted with saturated aqueous sodium bicarbonate and washed twice with diethyl ether. The aqueous portion was acidified with 1N HCl. The acidified aqueous portion was then extracted several times with EtOAc. The organic extracts were washed with brine, dried over Na₂SO₄, filtered through celite, and concentrated to give 5-methoxy-3-phenoxy-benzo[*b*]thiophene-2-carboxylic acid (Intermediate 6, 0.091 g, 95 % yield). MS: M+1=301.1 (APCI). ¹H-NMR (400 MHz, CDCl₃) δ 7.67 (d, *J* = 8.8 Hz, 1H), 7.29 (m, 2H), 7.09 (m, 2H), 6.97 (m, 2H), 6.84 (d, *J* = 2.4 Hz, 1H), 3.66 (s, 3H).

Example A.1. **5-methoxy-3-phenoxy-benzo[*b*]thiophene-2-carboxylic acid (2H-tetrazol-5-yl)-amide.** Intermediate 6 (0.086 g, 0.29 mmol) was stirred with carbonyl diimidazole (0.097 g, 0.60 mmol) in freshly distilled THF (6 mL) in an argon purged flask at room temperature for 16 hours. 5-Aminotetrazole (0.056 g, 0.66 mmol) was added and the reaction was stirred at vigorous reflux for five hours. The reaction was allowed to cool to room temperature and then was diluted with H₂O (~15 mL). 1N HCl was added dropwise until a solid precipitated. The solid was filtered and washed with H₂O. The cake material was collected and purified by preparative scale HPLC chromatography to give 5-methoxy-3-phenoxy-benzo[*b*]thiophene-2-carboxylic acid (2H-tetrazol-5-yl)-amide (Example A.1, Intermediate 7,0.0062 g, 6 % yield). MS: M+1=368.2 (APCI). ¹H-NMR (400 MHz, DMSO) δ 16.10 (s,1H), 11.70 (s, 1H), 8.01 (d, *J* = 9.0 Hz, 1H), 7.34 (m, 2H), 7.22 (m, 1H), 7.07 (m, 3H), 6.81 (d, *J*=2.2 Hz, 1H), 3.64 (s, 3H).

The title compounds of Examples A.2 through A.8 were synthesized in a manner analogous to Example A.1 by replacing phenol in the synthesis of Intermediate 4 with an appropriately substituted phenol or other alcohol (e.g., pyridin-ol).
Example A.2. **3-(Biphenyl-3-yloxy)-5-methoxy -benzo[*b*]thiophene-2-carboxylic acid (2H-tetrazol-5-yl)-amide.**
Example A.3. **5-Methoxy-3-m-tolyloxy-benzo[b]thiophene-2-carboxylic acid (2H-tetrazol-5-yl)-amide.**
Example A.4. **5-Methoxy-3-(3-nitro-phenoxy)-benzo[*b*]thiophene-2-carboxylic acid (2H-tetrazol-5-yl)-amide.**
Example A.5. **5-Methoxy-3-(pyridin-3-yloxy)benzo[*b*]thiophene-2-carboxylic acid (2H-tetrazol-5-yl)-amide.**
Example A.6. **3-(3-Acetylamino-phenoxy)-5-methoxy-benzo[*b*]thiophene-2-**carboxylic acid **(2H-tetrazol-5-yl)-amide.**
Example A.7. **3-(3-Acetyl-phenoxy)-5-methoxy-benzo[*b*]thiophene-2-carboxylic acid (2H-tetrazol-5-yl)-amide.**
Example A.8. **3-(3-dimethylamino-phenoxy)-5-methoxy-benzo[*b*]thiophene-2-carboxylic acid (2H-tetrazol-5-yl)-amide.**

Intermediate 8- **3-Chloro-5-methoxy-benzo[b]thiophene-2-carboxylic acid (2H-tetrazol-5-yl)-amide**. Intermediate 1 (2.00 g, 7.69 mmol) was refluxed with 5-aminotetrazole (0.719 g, 8.46 mmol) and triethylamine (1.22 mL, 8.80 mmol) in acetonitrile (38 mL) for 90 minutes. The reaction was allowed to cool to room temperature and a solid precipitated. The mixture was filtered and the cake was rinsed with acetonitrile and diethyl ether. The cake was dried *en vacuo* to give 3-Chloro-5-methoxy-benzo[b]thiophene-2-carboxylic acid (2H-tetrazol-5-yl)-amide (Intermediate 8, 1.72 g, 72% yield). MS: M+1=310.1 (APCI). ¹H-NMR (400 MHz, DMSO) δ 16.29 (s, 1H), 12.59 (s, 1H), 8.06 (d, *J* = 8.8 Hz, 1H), 7.34 (d, *J* =2.4 Hz, 1H), 7.28 (dd, *J* = 2.4, 8.8 Hz, 1H), 3.89 (s, 3H).

Intermediate 9- **3-chloro-5-methoxy-1-oxo-benzo[*b*]thiophene-2-carboxylic acid (2H-tetrazol-5-yl)-amide.** To a 0 °C, stirring solution of Intermediate 8 (5.12 16.6 mmol) in CH₂Cl₂ (26 mL) and Trifluoroacetic acid (26 mL) was added 30% aq. H₂O₂ (3.75 mL, 33.1 mmol) dropwise via syringe. The reaction was stirred at 0 °C for ten minutes and then at room temperature for 60 minutes. The mix was chilled again to 0 °C and then added dropwise to a 0 °C saturated aq. sodium bisulfite solution. A solid precipitated and was filtered. The cake was rinsed with H₂O and then was dried *en vacuo* to give 3-chloro-5-methoxy-1-oxo-benzo[b]thiophene-2-carboxylic acid (2H-tetrazol-5-yl)-amide (Intermediate 9, 4.89 g, 91 % yield). MS: M+1=326.1 (APCI). ¹H-NMR (400 MHz, DMSO) δ 16.31 (s, 1H), 12.95 (s, 1H), 8.10 (d, *J* = 8.4 Hz, 1H), 7.34 (m, 2H), 3.93 (s, 3H).

Intermediate 10 - **3-(3,5-dimethyl-phenoxy)-5-methoxy-1-oxo-benzo[*b*]thiophene-2-carboxylic acid (2H-tetrazol-5-yl)-amide. Intermediate 9** (0.200 g, 0.615 mmol) was mixed with 3,5-dimethylphenol (0.090 g, 0.738 mmol) in dioxane (6 mL). Sodium hydride (0.0389 g, 1.62 mmol) was added. The reaction was stirred at room temperature for one hour and then was concentrated *en vacuo.* The concentrate was slurried in diethyl ether (~15 mL) and acetic acid (~0.5 mL) and then was filtered. The cake was rinsed with diethyl ether. The cake was collected, dissolved into a minimum of acetonitrile, and titurated with H₂O. The resulting solid was filtered, rinsed with H₂O, and dried *en vacuo* to give 3-(3,5-dimethyl-phenoxy)-5-methoxy-1-oxo-benzo[*b*]thiophene-2-carboxylic acid (2H-tetrazol-5-yl)-amide (Intermediate 10, 0.093 g, 37 % yield). MS: M+1=412.1 (APCI). ¹H-NMR (400 MHz, DMSO) δ 15.71 (s, 1H), 12.26 (s, 1H), 8.03 (d, *J* = 8.4 Hz, 1H), 7.29 (d, *J* = 8.4 Hz, 1H), 7.08 (s, 1H), 6.89 (s, 2H), 6.61 (s, 1H), 3.85 (s, 3H), 2.11 (s, 6H).

Example A.9 - **3-(3,5-dimethyl-phenoxy)-5-methoxy-benzo[*b*]thiophene-2-carboxylic acid (2H-tetrazol-5-yl)-amide.** 3-(3,5-Dimethyl-phenoxy)-5-methoxy-1-oxo-benzo[b]thiophene-2-carboxylic acid (2H-tetrazol-5-yl)-amide (0.089 g, 0.22 mmol) was mixed stirred with sodium iodide (0.098 g, 0.65 mmol) in acetonitrile (1.0 mL) at room temperature for two minutes. Chlorotrimethylsilane (0.051 mL, 0.43 mmol) was added via syringe. The reaction was stirred at room temperature for two minutes and then was quenched with 10% aqueous sodium thiosulfate until the red color dissipated. The quenched reaction was diluted with H₂O until a solid precipitated. The solid was filtered and dried *en vacuo* to give 3-(3,5-Dimethyl-phenoxy)-5-methoxy -benzo[*b*]thiophene-2-carboxylic acid (2H-tetrazol-5-yl)-amide (Example A.9, Intermediate 11) in quantitative yield. MS: M+1=396.1 (APCI). ¹H-NMR (400 MHz, DMSO) δ 16.08 (s, 1H), 11.71 (s, 1H), 8.01 (d, *J* =8.8 Hz, 1H), 7.22 (dd, *J* = 2.0, 9.2 Hz, 1H), 6.85 (s, 1H), 6.73 (s, 1H), 6.66 (s, 2H), 3.66 (s, 3H), 2.18 (s, 6H).

The title compounds of Examples A.10 through A.16 were synthesized in a manner analogous to Example A.9 by replacing 3,5-dimethylphenol with an appropriately substituted phenol or other alcohol (e.g., naphthalen-2-ol).
Example A. 10. **5-Methoxy-3-(naphthalene-2-yloxy)-benzo[*b*]thiophene-2-carboxylic acid (2H-tetrazol-5-yl)-amide.**
Example A.11. **3-(3-Ethyl-phenoxy)-5-Methoxy-benzo[*b*]thiophene-2-carboxylic acid (2H-tetrazol-5-yl)-amide.**
Example A.12. **5-Methoxy-3-(3-morpholin-4-yl-phenoxy)-benzo[*b*]thiophene-2-carboxylic acid (2H-tetrazol-5-yl)-amide.**
Example A.13. **3-(3-Isopropyl-phenoxy)-5-Methoxy-benzo[*b*]thiophene-2-carboxylic acid (2H-tetrazol-5-yl)-amide.**
Example A. 14. **3-(3-Isopropyl-5-methyl-phenoxy)-5-Methoxy-benzo[*b*]thiophene-2-carboxylic acid (2H-tetrazol-5-yl)-amide.**
Example A.15. **3-(2-Ethyl-phenoxy)-5-Methoxy-benzo[*b*]thiophene-2-carboxylic acid (2H-tetrazol-5-yl)-amide.**
Example A.16. **3-(4-cyclohexyl-phenoxy)-5-Methoxy-benzo[*b*]thiophene-2-carboxylic acid (2H-tetrazol-5-yl)-amide.**

### EXAMPLES B.1-B.75

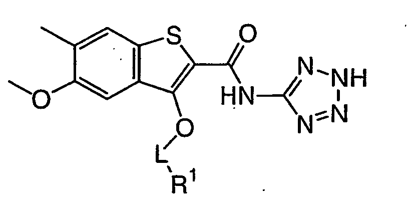

| Ex. | -L-R¹ | MS (M+1) | NMR*^{a}* |
|---|---|---|---|
| B.1 | 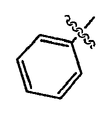 | 382.1 | δ16.12 (s,1H), 11.66 (s, 1H), 7.86 (s, 1H), 7.33 (t, *J* = 8.4 Hz, 2H), 7.08 (t, *J* = 7.4 Hz, 1H), 7.03 (d, *J* = 8.8 Hz, 2H), 6.74 (s, 1H), 3.59 (s, 3H), 2.24 (s, 3H) |
| B.2 | 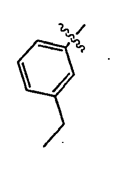 | 410.1 | 16.08 (s, 1H), 11.60 (s, 1H), 7.86 (s, 1H), 7.22 (t, *J* = 7.4 Hz, 1H), 6.96 (m, 2H), 6.79 (d, *J* = 7.6 Hz, 1H), 6.75 (s, 1H), 3.60 (s, 3H), 2.53 (q, *J* = 7.2 Hz, 2H), 2.25 (s, 3H), 1.08 (t, *J* = 7.2 Hz, 311) |
| B.3 | 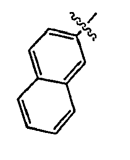 | 432.0 | 16.03 (s, 1H), 11.79 (s, 1H), 7.95 (d, *J* = 9.2 Hz, 1H), 7.87 (m, 2H), 7.73 (d, *J* = 8.4 Hz, 1H), 7.42 (m, 3H), 7.29 (s, 1H), 6.82 (s, 1H), 3.54 (s, 3H), 2.56 (s, 3H) |
| B.4 | 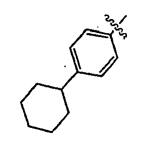 | 464.1 | 16.05 (s, 1H), 11.51 (s, 1H), 7.83 (s, 1H), 7.16 (m, 2H), 6.96 (m, 2H), 6.66 (s, 1H), 3.55 (s, 3H), 2.42 (m, 1H), 2.22 (s, 3H), 1.68 (m, 5H), 1.27 (m, 5H) |
| B.5 | 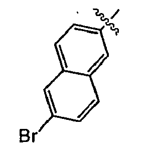 | 509.9 | 16.01 (s, 1H), 11.85 (s, 1H), 8.17 (s, 1H), 7.97 (m, 2H), 7.73 (d, *J* = 8.8 Hz, 1H), 7.53 (m, 2H), 7.31 (s, 1H), 6.82 (s, 1H), 3.56 (s. 3H), 2.26 (s, 3H) |
| B.6 | 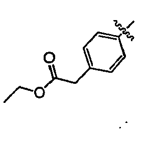 | 468.0 | 16.08 (s, 1H), 11.65 (s, 1H), 7.86 (s, 1H), 7.22 (d, *J* = 8.6 Hz, 2H), 7.01 (d, *J* = 8.6 Hz, 2H), 6.72 (s, 1H), 4.02 (q, *J* = 7.2 Hz, 2H), 3.59 (s, 5H), 2.25 (s, 3H), 1.13 (t, *J* = 7.2 Hz, 3H) |
| B.7 | 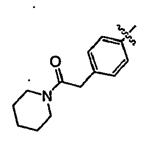 | 507.1 | 16.05 (s, 1H), 11.65 (s, 1H), 7.86 (s, 1H), 7.17 (d, *J* = 8.8 Hz, 2H), 6.98 (d, *J* = 8.8 Hz, 2H), 6.75 (s, 1H), 3.61 (s, 5H), 3.37 (m, 4H), 2.25 (s, 3H), 1.24-1.47 (m, 6H) |
| B.8 | 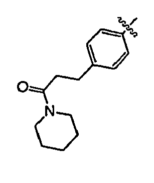 | 521.1 | 16.05 (s, 1H), 11.60 (s, 1H), 7.86 (s, 1H), 7.18 (d, *J* = 8.8 Hz, 2H), 6.95 (d, *J* = 8.8 Hz, 2H), 6.75 (s, 1H), 3.61 (s, 3H), 3.55 (m, 4H), 2.72 (t, *J* = 7.2 Hz, 2H), 2.50 (t, *J* = 7.2 Hz, 2H), 2.25 (s, 3H), 1.33-1.49 (m, 6H) |
| B.9 | 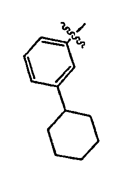 | 464.1 | 16.05 (s, 1H), 11.55 (s, 1H), 7.85 (s, 1H), 7.22 (t, *J* = 8.4 Hz, 1H), 7.02 (s, 1H), 6.97 (d, *J* = 7.6 Hz, 1H), 6.80 dd, *J* = 2.0, 7.6 Hz, 1H), 6.71 (s, 1H), 3.58 (s, 3H), 2.25 (s, 3H), 1.66 (m, 5H), 1.27 (m, 5H) |
| B.10 | 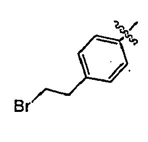 | 488.0 | 16.05 (s, 1H), 11.60 (s, 1H), 7.86 (s, 1H), 7.24 (d, *J* = 8.8 Hz, 2H), 7.00 (d, J = 8.8 Hz, 2H), 6.71 (s, 1H), 3.68 (t, *J* = 7.2 Hz, 2H), 3.56 (s, 3H), 3.06 (t, *J* = 7.2 Hz, 2H), 2.25 (s, 3H) |
| B.11 | 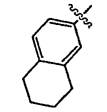 | 436.1 | 16.10 (s, 1H), 11.55 (s, 1H), 7.85 (s, 1H), 6.99 (m, 1H), 6.76 (m, 3H), 3.64 (s, 3H), 2.61 (m, 4H), 2.26 (s, 3H), 1.65 (m, 4H) |
| B.12 | 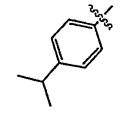 | 424.1 | 16.10 (s, 1H), 11.55 (s, 1H), 7.85 (s, 1H), 7.20 (d, *J* = 8.4 Hz, 2H), 6.99 (d, *J* = 8.4 Hz, 2H), 6.69 (s, 1H), 3.57 (s, 3H), 2.83 (m, 1H), 2.24 (s, 3H), 1.12 (d, *J* = 6.8 Hz, 6H) |
| B.13 | 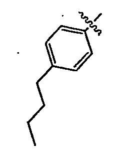 | 438.1 | 16.05 (s, 1H), 11.55 (s, 1H), 7.85 (s, 1H), 7.15 (d, *J* = 8.0 Hz, 2H), 6.97 (d, *J* = 8.0 Hz, 2H), 6.70 (s, 1H), 3.58 (s, 3H), 2.45 (s, 3H), 1.47 (m, 2H), 1.24 (m, 2H), 0.84 (t, *J* = 7.2 Hz, 3H) |
| B.14 | 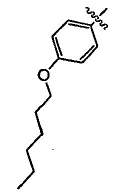 | 482.1 | 16.10 (s, 1H), 11.45 (s, 1H), 7.84 (s, 1H), 7.01 (d, *J* = 9.4 Hz, 2H), 6.88 (d, *J* = 9.4 Hz, 2H), 6.73 (s, 1H), 3.87 (t, *J* = 6.8 Hz, 2H), 3.60 (s, 3H), 2.25 (s, 3H), 1.65 (m, 2H), 1.27 (m, 6H), 0.84 (t, *J* = 6.8 Hz, 3H) |
| B.15 | 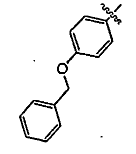 | 488.1 | 16.15 (s, 1H), 11.40 (s, 1H),1.84 (s, 1H), 7.35 (m, 5H), 7.02 (m,4H), 6.70 (s, 1H), 5.04 (s, 2H), 3.57 (s, 3H), 2.24 (s, 3H) |
| B.16 | 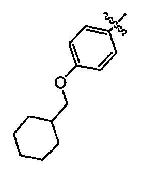 | 494.1 | 16.10 (s, 1H), 11.42 (s, 1H), 7.84 (s, 1H), 7.00 (d, *J* = 8.8 Hz, 2H), 6.88 (d, *J* = 8.8 Hz, 2H), 5.73 (s, 1H), 3.69 (d, *J* = 6.0 Hz, 2H), 3.60 (s, 3H), 2.45 (s, 3H), 1.69 (m, 6H), 1.62 (m, 3H), 0.99 (m, 2H) |
| B.17 | 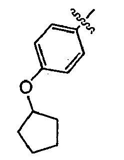 | 466.1 | 16.10 (s, 1H), 11.43 (s, 1H), 7.84 (s, 1H), 7.01 (d, *J* = 9.2 Hz, 2H), 6.86 (d, *J* = 9.2 Hz, 2H), 6.708 (s, 1H), 4.71 (m, 1H), 3.59 (s, 3H), 2.24 (s, 3H), 1.83 (m, 2H), 1.60 (m, 6H) |
| B.18 | 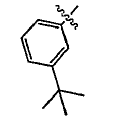 | 438.1 | 16.05 (s, 1H), 11.60 (s, 1H), 7.85 (s, 1H), 7.25 (m, 2H), 7.15 (m, 1H), 6.74 (s, 2H), 3.59 (s, 3H), 2.25 (s, 3H), 1.21 (s, 9H) |
| B.19 | 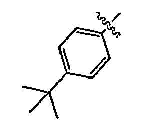 | 438.1 | 16.10 (s, 1H), 11.57 (s, 1H), 7.85 (s, 1H), 7.36 (d, *J* = 8.8 Hz, 2H), 6.99 (d, *J* = 8.8 Hz, 2H), 6.68 (s, 1H), 3.57 (s, 3H), 2.24 (s, 3H), 1.21 (s, 9H) |
| B.20 | 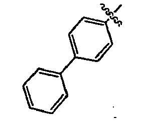 | 458.0 | 16.07 (s, 1H), 11.80 (s, 1H), 7.88 (s, 1H), 7.60 (m, 4H), 7.40 (m, 2H), 7.30 (m, 1H), 7.09 (m, 2H), 6.82 (s, 1H), 3.62 (s, 3H), 2.25 (s, 3H) |
| B.21 | 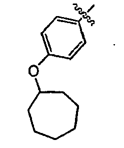 | 494.1 | 16.10 (s, 1H), 11.40 (s, 1H), 7.83 (s, 1H), 7.00 (m, 2H), 6.85 (m, 2H), 6.69 (s, 1H), 4.37 (m, 1H), 3.58 (s, 3H), 2.23 (s, 3H), 1.85 (m, 2H), 1.29-1.71 (m, 10H) |
| B.22 | 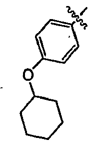 | 480.1 | 16.08 (s, 1H), 11.42 (s, 1H), 7.83 (s, 1H), 7.01 (m, 2H), 6.90 (m, 2H), 6.69 (s, 1H), 4.20 (m, 1H), 3.58 (s, 3H), 2.05 (s, 3H), 1.81 (m, 2H), 1.65 (m, 2H), 1.47 (m, 1H), 1.31 (m, 5H) |
| B.23 | 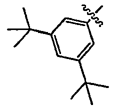 | 494.2 | 16.03 (s, 1H), 11.56 (s, 1H), 7.82 (s, 1H), 7.15 (s, 1H), 6.96 (s, 2H), 6.24 (s, 1H), 3.55 (s, 3H), 2.24 (s, 3H), 1.18 (s, 18H) |
| B.24 | 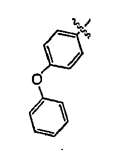 | 474.2 | 16.08 (s, 1H), 11.64 (s, 1H), 7.85 (s, 1H), 7.32 (t, *J* = 7.6 Hz, 2H), 7.06 (m, 5H), 6.85 (d, *J* = 8.8 Hz, 2H), 6.81 (s, 1H), 3.66 (s, 3H), 2.25 (s, 3H) |
| B.25 | 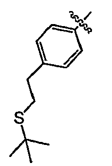 | 498.2 | 16.07 (s, 1H), 11.58 (s, 1H), 7.84 (s, 1H), 7.19 (d,*J* = 8.4 Hz, 2H), 6.98 (d, *J* = 8.4 Hz, 2H); 6.71 (s, 1H), 3.59 (s, 3H), 2.65 (m, 4H), 2.47 (s, 3H), 1.20 (s, 9H) |
| B.26 | 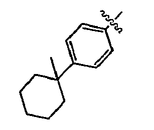 | 478.2 | 16.07 (s, 1H), 11.57 (s,- 1H), 7.83 (s, 1H), 7.32 (d, *J* = 8.8 Hz, 2H), 7.02 (d, *J* = 8.8 Hz, 2H), 6.64 (s, 1H), 3.54 (s, 3H), 2.23 (s, 3H), 1.90 (m, 2H), 1.22-1.48 (m, 8H), 1.05 (s, 3H) |
| B.27 | 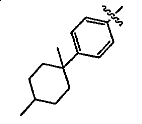 | 492.2 | 16.10 (s, 1H), 11.50 (s, 1H), 7.84 (s, 1H), 7.33 (m, 2H), 7.02 (m, 2H), 6.66 (s, 1H), 3.55 (s, 3H), 2.23 (s, 3H), 0.67-1.70 (m, 15H) |
| B.28 | 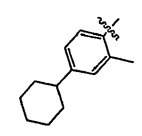 | 478.2 | 16.10 (s, 1H), 11.38 (s, 1H), 7.81 (s, 1H), 7.15 (s, 1H), 6.90 (d, *J* = 7.4 Hz, 1H), 6.62 (d, *J* = 8.8 Hz, 1H), 6.49 (s, 1H), 3.50 (s, 3H), 2.41 (s, 3H), 2.21 (s, 3H), 1.67 (m, 5H), 1.30 (m, 5H) |
| B.29 | 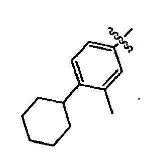 | 478.2 | 16.07 (s, 1H), 11.44 (s, 1H), 7.82 (s, 1H), 7.12 (d, *J* = 8.4 Hz, 1H), 6.90 (s, 1H), 6.80 (d, *J* = 8.4 Hz, 1H), 6.67 (s, 1H), 3.55 (s, 3H), 2.60 (m, 1H), 2.22 (s, 3H), 2.19 (s, 3H), 1.68 (m, 5H), 1.25 (m,5H) |
| B.30 | 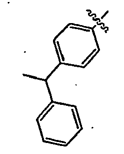 | 486.2 | 16.10 (s, 1H), 11.61 (s, 1H), 7.82 (s, 1H), 7.16 (m, 7H), 6.99 (d, *J* = 8.4 Hz, 1H), 6.60 (s, 1H), 4.10 (q, J = 7.6 Hz, 1H), 3.47 (s; 3H), 2.22 (s, 3H), 1.50 (d, *J* = 7.2 Hz, 3H) |
| B.31 | 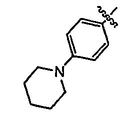 | 465.2 | 7.80 (s, 1H), 7.63 (m, 2H), 7.17 (m, 2H), 6.78 (s, 1H), 3.60 (m, 3H), 3.45 (m, 4H), 2.22 (s, 3H), 1.82 (m, 4H), 1.58 (m, 2H) |
| B.32 | 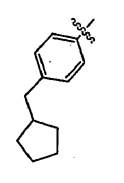 | 464.2 | 16.10 (s, 1H), 11.58 (s, 1H), 7.84 (s, 1H), 7.13 (d, *J* = 8.4 Hz, 2H), 6.96 (d, *J* = 8.4 Hz, 2H), 6.69 (s, 1H), 3.88 (d, *J* = 27.6 Hz, 1H), 3.56 (s, 3H), 2.28 (d, *J* = 16.4 Hz, 1H), 2.23 (s, 3H), 1.96 (m, 1H), 1.53 (m, 6H), 1.09 (m, 2H) |
| B.33 | 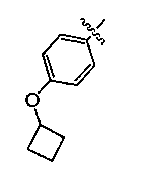 | 452.2 | 16.10 (s, 1H), 11.55 (s, 1H), 7.84 (s, 1H), 7.01 (d, *J* = 9.2 Hz, 2H), 6.80 (d, *J* = 8.8 Hz, 2H), 6.70 (s, 1H), 4.58 (m, 1H),3.59 s, 3H), 2.35 (m, 2H), 2.24 (s, 3H), 1.95 (m, 2H), 1.74 (m, 1H), 1.58 (m, 1H) |
| B.34 | 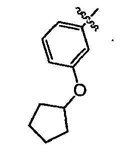 | 466.3 | 16.10 (s, 1H), 11.71 (s, 1H), 7.87 (s, 1H), 7.19 (t, 8.4 Hz, 1H), 6.81 (s, 1H), 6.58 (m, 3H), 4.73 (m, 1H), 3.64 (s, 3H), 2.26 (s, 3H), 1.82 (m, 2H), 1.61 (m, 6H) |
| B.35 | 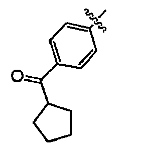 | 478.2 | |
| B.36 | 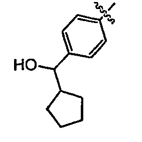 | 478.2 (M-1) | |
| B.37 | 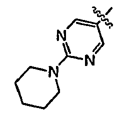 | 467.1 | |
| B.38 | 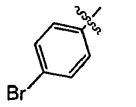 | 460.0 | |
| B.39 | 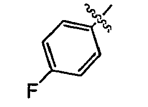 | 400.1 | |
| B.40 | 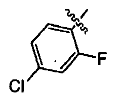 | 434.0 | |
| B.41 | 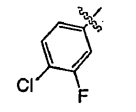 | 434.0 | |
| B.42 | 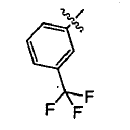 | 450.1 | |
| B.43 | 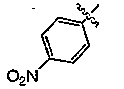 | 427.0 | |
| B.44 | 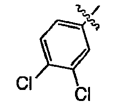 | 450.0 | |
| B.45 | 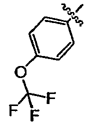 | 466.0 | |
| B.46 | 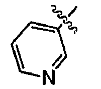 | 383.0 | |
| B.47 | 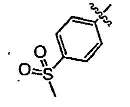 | 460.0 | |
| B.48 | 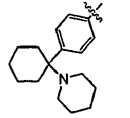 | 547.2 | |
| B.49 | 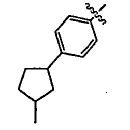 | 464.2 | |
| B.50 | 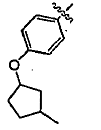 | 480.2 | |
| B.51 | 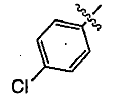 | 416.1 | |
| B.52 | 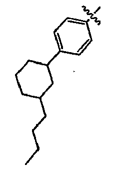 | 518 (M-1) | 11.55(s,1H 7.85(s,1H), 7.18(d,2H), 6.97(d,2H), 6.67(s,1H), 3.55(s,3H), 2.24(s,3H), 1.70 (m,4H), 1.45-1.05(m, 11H), 1.00-0.75(m,4H) |
| B.53 | 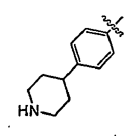 | 463 (M-1) | 8.50(brs,1H), 8.20(brs,1H), 7.88(s,1H), 7.18(d,2H), 7.03(d,2H), 6.75(s,1H), 3.63(s,3H), 3.30(m,3H), 2.92(m,2H), 2.13(s,3H), 1.95(m,2H), 1.68(m,2H) |
| B.54 | 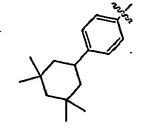 | 518 (M-1) | (CDCl₃) 7.50(s,1H 7.18(d,2H), 7.00(d,2H), 6.38(s,1H), 3.46(s,3H), 2.80(s,1H), 2.22(s,3H), 1.43(m,6H), 1.03(s,6H), 0.83(s,6H) |
| B.55 | 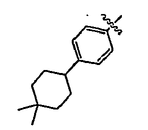 | 490 (M-1) | 16.06,11.57(brs,1H), 7.86(s,1H), 7.20(d,2H), 6.98(d,2H), 6.70(s,1H), 3.57(s,3H), 2.35(m,1H), 2.25(s,3H), 1.60-1.20(m,8H), 0.92(s,3H), 0.90(s,3H) |
| B.56 | 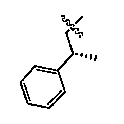 | 429 (M-1) | (CDCl3) 7.75(s,1H), 7.39(d,12H), 7.25(t,2H),m 7.16(t,1H), 6.85(s,1H), 4.47(m,2H), 3.73(s,3H), 2.23(s,3H), 1.38(d,3H) |
| B.57 | 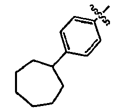 | 476 (M-1) | 7.83(s,1H), 7.18(d,2H), 6.98(d,2H), 6.67(s,1H), 3.57(s,3H), 2.60(m,1H), 2.23(s,3H), 1.80-1.40(m,12H). |
| B.58 | 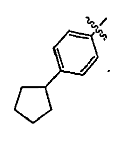 | 448 (M-1) | 7.84(s,1H), 7.20(d,2H), 6.98(d,2H), 6.72(s,1H), 3.59(s,3H), 2.90(m,1H), 2.25(s,3H), 2.95(M,2H), 1.70(m,2H), 1.60(m,2H), 1.42(m,2H) |
| B.59 | 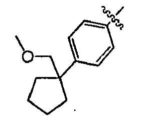 | 492 (M-1) | (CDCl3) 7.55(s,1H), 7.35(d,2H), 7.07(d,2H), 6.45(s,1H), 3.50(s,3H), 3.34(s,2H), 3.22(s,3H), 2.30(s,3H), 2.00-1.83(m,8H) |
| B.60 | 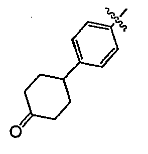 | 476 (M-1) | 7.84(s,1H),7.28(d,2H), 7.02(d,2H), 6.74(s,1H), 3.92(s,1H), 3.57(s,3H), 3.00(m,1H), 2.45(M,2H), 2.25(m,2H), 2,23(s,3H), 1.98(M,2H), 1.80(m,2H). |
| B.61 | 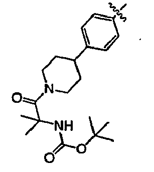 | 648 (M-1) | |
| B.62 | 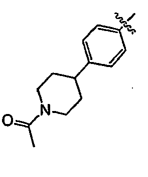 | 505 (M-1) | 7.85(s,1H), 7.23(d,2H), 6.99(d,2H), 6.72(s,1H), 4.47(m,1H), 3.86(m,1H), 3.58(s,3H), 3.07(t,1H), 2.70(m,1H), 2.52(m,1H), 2.25(s,3H), 1.96(s,3H), 1.69(m,2H), 1.5o(m,1H), 1.35(m,1H). |
| B.63 | 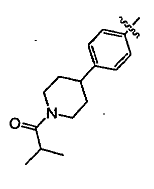 | 533 (M-1) | 7.86(lh), 7.22(d,2H), 6.98(d,2H), 6.71(s,1H), 4.53(d,1H), 4.02(d,1H), 3.58(s,3H), 3.06(t,1H), 2.86(7 line m, 1H), 2.73(tt, -1H), 2.50(m,1H), 2.23(s,3H), 1.74(m,2H), 1.50-1.30(m,2H),0.97(t,6H) |
| B.64 | 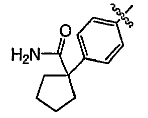 | 491 (M-1) | 7.83(s,1H), 7.33(d,2H), 7.02(d,2H), 6.97(s,1H), 6,75(s,1H), 6.62(s,1H), 3.53(s,3H), 2.22(s,3H), 1.60-1.43(m,8H) |
| B.65 | 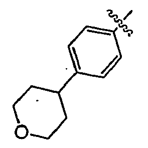 | 464 (M-1) | (CDCl3) 13.23(s,1H), 10.24(s,1H), 7.78(s,1h), 7.25(d,2H), 7.18(d,1H), 6.47(s,1H), 4.16(m,2H), 3.51(s,3H), 3.50(m,2H), 2.77(m,1H), 2.32(s,3H), 1.75(m,4H). |
| B.66 | 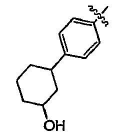 | 478 (M-1) | |
| B.67 | 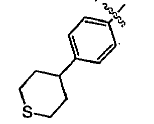 | 480 (M-1) | 7.86(s,1H), 7.20(d,2H), 7.00(d,2H), 6.73(s,1H), 3.58(s,3H), 2.80-2.25(m,3H), 2.24(s,3H), 1.95(m,2H), 1.63(m,2H) |
| B.68 | 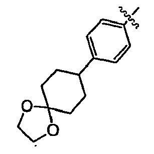 | 520 (M-1) | |
| B.69 | 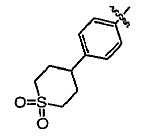 | 512 (M-1) | |
| B.70 | 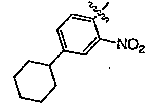 | 509.3 | |
| B.71 | 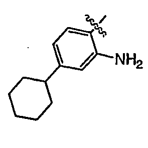 | | NMR (DMSO, 400MHz): 7.77 (s, 1H), 6.71 (s, 1H), 6.54 (m, 2H), 6.31 (d, 1H), 3.45 (s, 3H), 2.30 (m, 1H), 2.20 (s, 3H), 1.69 (m, 5H), 1.28 (m, 5H). |
| B.72 | 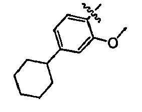 | 494.2 | |
| B.73 | 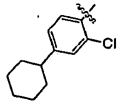 | 498.1 | |
| B.74 | 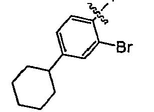 | 542.1 | |
| B.75 | 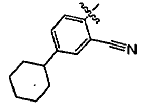 | 489.1 | |

| | | | |
|---|---|---|---|
| ^{a 1}H-NMR (400 MHz, CDCl₃) | | | |

Example B.1. **5-Methoxy-6-methyl-3-phenoxy-benzo[*b*]thiophene-2-carboxylic acid (2H-tetrazol-5-yl)-amide** was synthesized in an analogous manner to Example A.1 with the following exceptions:

3-methoxy-4-methylcinnamic acid was substituted for methoxy-cinnamic acid (see synthesis of Intermediate 1 above). 3-methoxy-4-methylcinnamic acid was synthesized as follows:

Intermediate 12 - **3-methoxy-4-methylcinnamic acid.** 3-(3-methoxy-4-methyl-phenyl)-acrylic acid starting material was prepared according to the following reaction: 3-methoxy-4-methylbenzaldehyde (20.0 mL, 137 mmol) was refluxed with malonic acid (27.2 g, 206 mmol) in a mixture of piperidine (6 mL) and pyridine (200 mL) for 2.5 hours. The mix was concentrated to one half volume. H₂O (~20 mL) and 1N HCl (~6 mL) were added to give a solid precipitate. The solid was filtered and rinsed with 1N HCl and then H₂O and then was dried *en vacuo* to give 3-methoxy-4-methylcinnamic acid (Intermediate 12) in quantitative yield. MS: M+1=193.1 (APCI). ¹H-NMR (400 MHz, CDCl₃) δ 7.72 (d, *J*= 16 Hz, 1H), 7.37 (m, 2H), 6.83 (d, *J* = 8.8 Hz, 1H), 6.31 (d, *J* = 15.6 Hz, 1H), 3.87 (s, 3H), 2.23 (s, 3H).

In the analogous reaction to the synthesis of Intermediate 2, isopropanol and triethylamine were substituted for phenol and pyridine, respectively to afford the isopropyl ester instead of the phenyl ester of Intermediate 2.

The rest of the steps in the synthesis of B.1 were carried out as in an analogous fashion to Example A.1 up to the 5-amino-tetrazole coupling step. The final step 5-amino tetrazole coupling was carried out under the following conditions: 5-Methoxy-6-methyl-3-phenoxy-benzo[*b*]thiophene-2-carboxylic acid (0.379 g, 1.21 mmol) was dissolved in anhydrous CH₂Cl₂ (6 mL) in an argon-purged flask. A catalytic drop of DMF followed by oxalyl chloride (0.158 mL, 1.82 mmol) were added via syringe. The reaction was stirred at room temperature for 30 minutes. Acetonitrile (6 mL) and then 5-aminotetrazole (0.155 g, 1.82 mmol) and triethylamine (0.506 mL, 3.64 mmol) were added. The reaction was stirred at reflux for 30 minutes and then was allowed to cool to room temperature. The reaction was diluted with H₂O and acidified with 1N HCl until a solid precipitated. The solid was filtered and rinsed with H₂O. The filter cake slurried in a minimum MeOH and filtered again and dried *en vacuo* to give 5-Methoxy-6-methyl-3-phenoxy-benzo[b]thiophene-2-carboxylic acid (2H-tetrazol-5-yl)-amide (Example B.1, Intermediate 13, 0.299 g, 65 % yield). MS: M+1=382.1 (APCI). ¹H-NMR (400 MHz, DMSO) δ 16.12 (s, 1H), 11.66 (s, 1H), 7.86 (s, 1H), 7.33 (t, *J* = 8.4 Hz, 2H), 7.08 (t, *J* = 7.4 Hz, 1H), 7.03 (d, *J* = 8.8 Hz, 2H), 6.74 (s, 1H), 3.59 (s, 3H), 2.24 (s, 3H).

**Example B.2. 3-(3-Ethyl-phenoxy)-5-Methoxy-6-methyl-benzo[*b*]thiophene-2-carboxylic acid (2H-tetrazol-5-yl)-amide** was synthesized in an analogous manner to Example A.9 starting from Intermediate 12 and replacing 3,5-dimethylphenol with 3-ethyl-phenol.

The title compounds of Examples B.3, and B.5-B.35 were synthesized in a manner analogous to Example B.2 by replacing 3-ethyl-phenol with an appropriately substituted phenol or other alcohol.

The title compounds of Examples B.4, and B.36-B.75 were synthesized in a manner analogous to Example B.1 by replacing phenol as R¹-L-OH with an appropriately substituted phenol or other alcohol.
Example B.3. **5-Methoxy-6-methyl-3-(naphthalene-2-yloxy)-benzo[*b*]thiophene-2-carboxylic acid (2H-tetrazol-5-yl)-amide.**
Example B.4. **3 -(4-Cyclohexyl-phenoxy)-5-methoxy-6-methyl-benzo[*b*]thiophene-2-carboxylic acid (2H-tetrazol-5-yl)-amide.**
Example B.5. **3-(6-Bromo-naphthalen-2-yloxy)-5-methoxy-6-methyl-benzo[*b*]thiophene-2-carboxylic acid (2H-tetrazol-5-yl)-amide.**
Example B.6. **{4-[5-Methoxy-6-methyl-2-(2H-tetrazol-5-ylcarbamoyl)-benzo[*b*]thiophene-3-yloxy]-phenyl}-acetic acid ethyl ester.**
Example B.7. **5-Methoxy-6-methyl-3-[4-(2-oxo-2-piperidin-1-yl-ethyl)-phenoxy]-benzo[*b*]thiophene-2-carboxylic acid (2H-tetrazol-5-yl)-amide.**
Example B.8. **-5-Methoxy-6-methyl-3-[4-(3-oxo-3-piperidin-1-yl-propyl)-phenoxy]-benzo[*b*]thiophene-2-carboxylic acid (2H-tetrazol-5-yl)-amide.**
Example B.9. **3-(3-Cyclohexyl-phenoxy)-5-methoxy-6-methyl-benzo[*b*]thiophene-2-carboxylic acid (2H-tetrazol-5-yl)-amide.**
Example B.10. **3-[4-(2-Bromo-ethyl)-phenoxy]-5-methoxy-6-methyl-benzo[*b*]thiophene-2-carboxylic acid (2H-tetrazol-5-yl)-amide.**
Example B.11. **5-Methoxy-6-methyl-3-(5,6,7,8-tetrahydro-naphthalen-2yloxy)-benzo[*b*]thiophene-2-carboxylic acid (2H-tetrazol-5-yl)-amide.**
Example B.12. **3-(4-Isopropyl-phenoxy)-5-methoxy-6-methyl-benzo[*b*]thiophene-2-carboxylic acid (2H-tetrazol-5-yl)-amide.**
Example B.13. **3-(4-Butyl-phenoxy)-5-methoxy-6-methyl-benzo[*b*]thiophene-2-carboxylic acid (2H-tetrazol-5-yl)-amide.**
Example B.14. **3-(4-Hexyloxy-phenoxy)-5-methoxy-6-methyl-benzo[*b*]thiophene-2-carboxylic acid (2H-tetrazol-5-yl)-amide.**
Example B.15. **3-(4-Benzyloxy-phenoxy)-5-methoxy-6-methyl-benzo[*b*]thiophene-2-carboxylic acid (2H-tetrazol-5-yl)-amide.**
Example B.16. **3-(4-Cyclohexylmethoxy-phenoxy)-5-methoxy-6-methyl-benzo[*b*]thiophene-2-carboxylic acid (2H-tetrazol-5-yl)-amide.**
Example B.17**. 3-(4-Cyclopentyloxy-phenoxy)-5-methoxy-6-methyl-benzo[*b*]thiophene-2-carboxylic acid (2H-tetrazol-5-yl)-amide.**
Example B.18. **3-(3-tert-Butyl-phenoxy)-5-methoxy-6-methyl-benzo[*b*]thiophene-2-carboxylic acid (2H-tetrazol-5-yl)-amide.**
Example B.19**. 3-(4-tert-Butyl-phenoxy)-5-methoxy-6-methyl-benzo[*b*]thiophene-2-carboxylic acid (2H-tetrazol-5-yl)-amide.**
Example B.20. **3-(Biphenyl-4-yloxy)-5-methoxy-6-methyl-benzo[b]thiophene-2-carboxylic acid -(2H-tetrazol-5-yl)-amide.**
Example B.21. **3-(4-Cycloheptyloxy-phenoxy)-5-methoxy-6-methyl-benzo[*b*]thiophene-2-carboxylic acid (2H-tetrazol-5-yl)-amide.**
Example B.22. **3-(4-Cyclohexyloxy-phenoxy)-5-methoxy-6-methyl-berizo[*b*]thiophene-2-carboxylic acid (2H-tetrazol-5-yl)-amide.**
Example B.23. **3-(3,4-Di-tert-butyl-phenoxy)-5-methoxy-6-methyl-benzo[*b*]thiophene-2-carboxylic acid (2H-tetrazol-5-yl)-amide.**
Example B.24. **5-Methoxy-6-methyl-3-(4-phenoxy-phenoxy)-benzo[*b*]thiophene-2-carboxylic acid (2H-tetrazol-5-yl)-amide.**
**Example B.25. 3-[4-(2-tert-Butylsulfanyl-ethyl)-phenoxy)-5-methoxy-6-methyl-benzo[*b*]thiophene-2-carboxylic acid (2H-tetrazol-5-yl)-amide.**
Example B.26. **5-Methoxy-6-methyl-3-[4-(1-methyl-cyclohexyl)-phenoxy]-benzo[*b*]thiophene-2-carboxylic acid (2H-tetrazol-5-yl)-amide.**
Example B.27**. 3-[4-(1,4-Dimethyl-cyclohexyl)-phenoxy]-5-methoxy-6-methyl-benzo[*b*]thiophene-2-carboxylic acid (2H-tetrazol-5-yl)-amide.**
Example B.28**. 3-(4-Cyclohexyl-2-methyl-phenoxy)-5-methoxy-6-methyl-benzo[*b*]thiophene-2-carboxylic acid (2H-tetrazol-5-yl)-amide.**
Example B.29. **3-(4-Cyclohexyl-3-methyl-phenoxy)-5-methoxy-6-methyl-benzo[*b*]thiophene-2-carboxylic acid (2H-tetrazol-5-yl)-amide.**
Example B.30. **5-Methoxy-6-methyl-3-[4-(1-phenyl-ethyl)-phenoxy]-benzo[*b*]thiophene-2-carboxylic acid (2H-tetrazol-5-yl)-amide.**
Example B.31. **5-Methoxy-6-methyl-3-(4-piperidin-1-yl-phenoxy)-benzo[*b*]thiophene-2-carboxylic acid (2H-tetrazol-5-yl)-amide.**
Example B.32. **3-(4-Cyclopentylmethyl-phenoxy)-5-methoxy-6-methyl-benzo[*b*]thiophene-2-carboxylic acid (2H-tetrazol-5-yl)-amide.**
**Example B.33. 3-(4-Cyclobutoxy-phenoxy)-5-methoxy-6-methyl-benzo[*b*]thiophene-2-carboxylic acid (2H-tetrazol-5-yl)-amide.**
Example B.34. **3-(3-Cyclopentyloxy-phenoxy)-5-methoxy-6-methyl-benzo[*b*]thiophene-2-carboxylic acid (2H-tetrazol-5-yl)-amide.**
Example B.35. **3-(4-Benzoyl-phenoxy)-5-methoxy-6-methyl-benzo[*b*]thiophene-2-carboxylic acid (2H-tetrazol-5-yl)-amide (Intermediate** 14).
Example B.36. **3-[(4-Hydroxy-phenyl-methyl)-phenoxy]-5-methoxy-6-methyl-benzo[*b*]thiophene-2-carboxylic acid (2H-tetrazol-5-yl)-amide.** Sodium borohydride (0.0055 g, 0.146 mmol) was added to a solution of Intermediate 14 (0.058 g, 0.12 mmol) in methanol (0.6 mL) and THF (0.1 mL). The reaction was stirred at room temperature for ten minutes and then was quenched with aq. 1N HCl (~1 mL). The quenched reaction was diluted with H₂O (~5 mL). The resulting solid precipitate was filtered, rinsed with H₂O, and dired *en vacuo* to give 3-[(4-Hydroxy-phenyl-methyl)-phenoxy]-5-methoxy-6-methyl-benzo[*b*]thiophene-2-carboxylic acid (2H-tetrazol-5-yl)-amide (0.057 g, 98% yield).
Example B.37. **5-Methoxy-6-methyl-3-(4-piperidin-1-yl-pyrimidin-5-yloxy)-benzo[*b*]thiophene-2-carboxylic acid (2H-tetrazol-5-yl)-amide.**
Example B.38. **3-(4-Bromo-phenoxy)-5-methoxy-6-methyl-benzo[*b*]thiophene-2-carboxylic acid (2H-tetrazol-5-yl)-amide.**
Example B.39. **3-(4-Fluoro-phenoxy)-5-methoxy-6-methyl-benzo[*b*]thiophene-2-carboxylic acid (2H-tetrazol-5-yl)-amide.**
Example B.40. **3-(4-Chloro-2-fluoro-phenoxy)-5-methoxy-6-methyl-benzo[*b*]thiophene-2-carboxylic acid (2H-tetrazol-5-yl)-amide.**
Example B.41. **3-(4-Chloro-3-fluoro-phenoxy)-5-methoxy-6-methyl-benzo[*b*]thiophene-2-carboxylic acid (2H-tetrazol-5-yl)-amide.**
Example B.42. **5-Methoxy-6-methyl-3-(3-trifluoromethyl-phenoxy)-benzo[*b*]thiophene-2-carboxylic acid (2H-tetrazol-5-yl)-amide.**
Example B.43. **5-Methoxy-6-methyl-3-(4-nitro-phenoxy)-benzo[*b*]thiophene-2-carboxylic acid (2H-tetrazol-5-yl)-amide.**
Example B.44. **3-(3,4-Dichloro-phenoxy)-5-methoxy-6-methyl-benzo[*b*]thiophene-2-carboxylic acid (2H-tetrazol-5-yl)-amide.**
Example B.45. **5-Methoxy-6-methyl-3-(4-trifluoromethoxy-phenoxy)-benzo[*b*]thiophene-2-carboxylic acid (2H-tetrazol-5-yl)-amide.**
Example B.46. **5-Methoxy-6-methyl-3-(pyridin-3-yloxy)-benzo[*b*]thiophene-2-carboxylic acid (2H-tetrazol-5-yl)-amide.**
Example B.47**. 3-(4-Methanesulfonyl-phenoxy)-5-methoxy-6-methyl-benzo[*b*]thiophene-2-carboxylic acid (2H-tetrazol-5-yl)-amide.**
Example B.48. **3-[4-(Cyclohexyl-piperidin-1-yl-methyl)-phenoxy]-5-methoxy-6-methyl-benzo[*b*]thiophene-2-carboxylic acid (2H-tetrazol-5-yl)-amide.**
Example B.49. **5-Methoxy-6-methyl-3-[4-(3-methyl-cyclopentyl)-phenoxy]-benzo[*b*]thiophene-2-carboxylic acid (2H-tetrazol-5-yl)-amide.**
Example B.50. **5-Methoxy-6-methyl-3-[4-(3-methyl-cyclopentyloxy)-phenoxy]-benzo[*b*]thiophene-2-carboxylic acid (2H-tetrazol-5-yl)-amide.**
Example B.51. **3-(4-Chloro-phenoxy)-5-Methoxy-6-methyl-benzo[*b*]thiophene-2-carboxylic acid (2H-tetrazol-5-yl)-amide.**
Example B.52. **3-[4-(3-Butyl-cyclohexyl)-phenoxy]-5-methoxy-6-methyl-benzo[b]thiophene-2-carboxylic acid (2H-tetrazol-5-yl)-amide.**
Example B.53**. 5-Methoxy-6-methyl-3-(4-piperdiny-4-yl-phenoxy)-benzo[b]thiophene-2-carboxylic acid(2H-tetrazole-5yl)-amide.**
Example B.54**. 5-Methoxy-6-methyl-3-[4-(3,3,5,5-tetramethyl-cyclohexyl)-phenoxy]-benzo[b]thiophene-2-carboxylic acid(2H-tetrazol-5yl)-amide.**
Example B.55. **3-[4-(4,4-Dimethyl-cyclohexyl)-phenoxy]-5-methoxy-6-methyl-benzo[b]thiophene-2-carboxylic acid (2H-tetrazol-5yl)-amide.**
Example B.56**. 5-Methoxy-6-methyl-3-(2-phenyl-propoxy)-benzo[b]thiophene-2-carboxylic acid(2H-tetrazol-5-yl)-amide amide.**
Example B.57. **3-(4-cycloheptyl-phenoxy)-5-methoxy-6-methyl-benzo[b]thiophene-2-carboxylic acid (2H-tetrazol-5-yl) amide.**
Example B.58. **3-(4-cyclopentyl-phenoxy)-5-methoxy-6-methyl-benzo[b]thiophene-2-carboxylic acid (2H-tetrazol-5-yl) amide.**
Example B.59. **5-Methoxy-3-[4-(1-methoxymethyl-cyclopentyl)-phenoxy]-6-methyl-benzo[b]thiophene-2-carboxylic acid (2H-tetrazol-5-yl)- amide.**
Example B.60**. 5-Methoxy-6-methyl-3-[4-(4-oxo-cyclohexyl)-phenoxy]-benzo[b]thiophene-2-carboxylic acid (2H-tetrazol-5-yl)-amide.**
Example B.61. **[2-(4-{4-[5-Methoxy-6-methyl-2-(2H-tetrazol-5-ylcarbamoyl)-benzo[b]thiophen-3-ylox-y]-phenyl}-piperidiny-1-yl)-1,1-dimethyl-2-oxoethyl]-carbamic acid tert-butyl ester.**
Example B.62. **3-[4-(1-Acetyl-piperdin-4-yl)-phenoxy]-5-methoxy-6-methyl-benzo[b]thiophene-2-carboxylic acid (2H-tetrazol-5-yl) amide.**
Example B.63**. 3-[4-(1-isobutyryl-piperdin-4-yl)-phenoxy]-5-methoxy-6-methyl-benzo[b]thiophene-2-carboxylic acid (2H-tetrazol-5-yl)-amide.**
Example B.64. **3-[4-(1-carbamoyl-cyclopentyl)-phenoxy]-5-methoxy-6-methyl-benzo[b]thiophene-2-carboxylic acid (2H-tetrazol-5-yl)-amide.**
Example B.65. **5-methoxy-6-methyl-3-[4-(tetrahydro-pyran-4-yl)-phenoxy]-benzo[b]thiophene-2-carboxylic acid(2H-tetrazol-5-yl)-amide.**
**Example B.66. 3-[4-(3-Hydroxy-cyclohexyl)-phenoxy]-5-methoxy-6-methyl-benzo[b]thiophene-2-carboxylic acid(2H-tetrazol-5-yl)-amide.**
Example B.67. **5-methoxy-6-methyl-3-[4-(tetrahydro-thiopyran-4-yl)-phenoxy]-benzo[b]thiophene-2-carboxylic acid(2H-tetrazol-5-yl)-amide.**
Example B.68. **3-[4-(1,4-Dioxa-spiro[4.5]dec-S-yl)-phenoxy]-5-methoxy-6-methyl-benzo[b]thiophene-2-carboxylic acid (2H-tetrazol-5-yl)-amide.**
Example B.69. **3-[4-(1,1-Dioxo-hexahydro-1λ⁶-thiopyran-4-yl)-phenoxy]-5-methoxy-6-methyl-benzo[b]thiophene-2-carboxylic acid (2H-tetrazol-5-yl)-amide.**
Example B.70. **5-Methoxy-6-methyl-3-(2-nitro-4-cyclohexyl-phenoxy)-benzo[b]thiophene-2-carboxylic acid (2H-tetrazol-5-yl)-amide.**
Example B.71. **3-(2-Amino-4-cyclohexyl-phenoxy)-5-methoxy-6-methyl-benzo[b]thiophene-2-carboxylic acid (2H-tetrazol-5-yl)-amide.**
Example B.72. **3-(2-Methoxy-4-cyclohexyl-phenoxy)-5-methoxy-6-methyl-benzo[b]thiophene-2-carboxylic acid (2H-tetrazol-5-yl)-amide.**
Example B.73. **3-(2-Chloro-4-cyclohexyl-phenoxy)-5-methoxy-6-methyl-benzo[b]thiophene-2-carboxylic acid (2H-tetrazol-5-yl)-amide.**
Example B.74. **3-(2-Bromo-4-cyclohexyl-phenoxy)-5-methoxy-6-methyl-benzo[b]thiophene-2-carboxylic acid (2H-tetrazol-5-yl)-amide.**
Example B.75. **3-(2-Cyano-4-cyclohexyl-phenoxy)-5-methoxy-6-methyl-benzo[b]thiophene-2-carboxylic acid (2H-tetrazol-5-yl)-amide.**

### EXAMPLES C.1-C.35

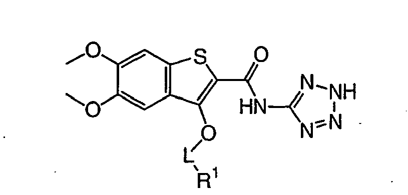

| Ex. | -L-R¹ | MS (M+1) | ¹H-NMR*^{a}* or Microanalysis or Melting Point (°C) |
|---|---|---|---|
| C.1 | 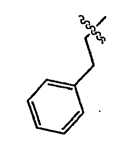 | 426.2 | δ 16.09 (bs, 1H), 11.08 (bs, 1H), 7.57 (s, 1H), 7.37 (d, *J* = 7.3, 2H), 7.26 (t, *J* = 7.4, 2H), 7.17 (t, *J* = 7.3, 1H), 6.98 (s, 1H), 4.66-4.62 (m, 2H), 3.83 (s, 3H), 3.72 (s, 3H), 3.17-3.14 (m, 2H) |
| C.2 | 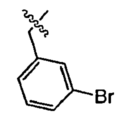 | 492.1 | δ 7.62 (s, 1H), 7.36 6.89 (m, H), 5.22 (s, 2H), 3.85 (s, 3H), 3.76. |
| C.3 | 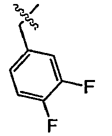 | 448.2 | δ 10.11 (s, 1H), 7.11-7.00 (m, 5H), 5.24 (s, 2H), 3.86 (s, 3H), 3.80 (s, 3H) |
| C.4 | 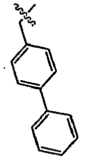 | 488.0 | δ 7.57-745. (m, 1H), 7.29-7.03 (11H), 6.63 (s, 1H), 5.33 (s, 2H), 3.86 (s, 3H), 3.59 (s, 3H) |
| C.5 | 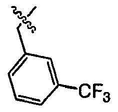 | 480.1 | δ 7.84 (s, 1H), 7.76-7.66 (m, 2H), 7.59-7.55 (m, 2H), 7.25 (m, 1H), 5.52 (s, 2H), 3.83 (s, 3H), 3.80 (s, 3H) |
| C.6 | 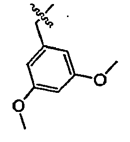 | 472.1 | Microanalysis: Calculated.: C, 53.50; H4.49; N14.85. Found: C, 53.86; 4.49; 14.56 |
| C.7 | 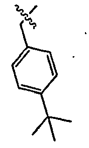 | 468.2 | δ15.90 (s, 1H),10.72 (s, 1H), 7.59 (s, 1H), 7.39-7.24 (m, 4H), 7.21 (s, 1H), 5.48 (s, 2H), 3.83 (s. 3H), 3.80 (s, 3H), 1.08 (s, 9H) |
| C.8 | 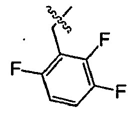 | 466.1 | 10.11 (s, 1H), 7.28-7.03 (m, 3H), 6.78-6.82 (m, 1H), 5.28 (s, 2H), 3.83 (s, 3H), 3.82 (s, 3H) |
| C.9 | 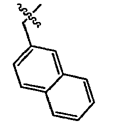 | 460.2 APCI | Melting Point = 204 °C |
| C.10 | 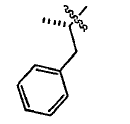 | 440.3 | |
| C.11 | 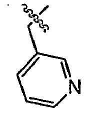 | 413.2 | |
| C.12 | 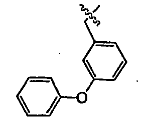 | 504.2 | |
| C.13 | 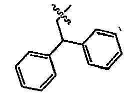 | 502.3 | |
| C.14 | 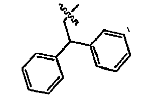 | 413.2 | |
| C.15 | 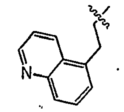 | 477.3 | |
| C.16 | 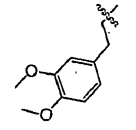 | 486.3 | |
| C.17 | 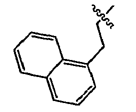 | 476.3 | |
| C.18 | 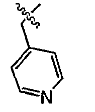 | 413.2 | |
| C.19 | 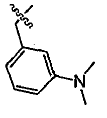 | 455.3 | |
| C.20 | 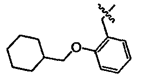 | 524.3 | |
| C.21 | 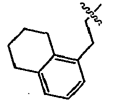 | 480.2 | |
| C.22 | 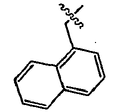 | 462.3 | |
| C.23 | 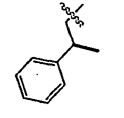 | 440.3 | |
| C.24 | 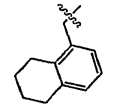 | 466.2 | |
| C.25 | 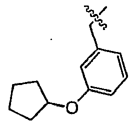 | 496.1 | |
| C.26 | 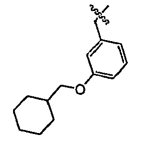 | 524.3 | |
| C.27 | 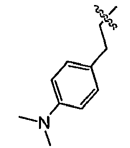 | 469.2 | |
| C.28 | 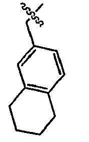 | 466.1 | |
| C.29 | 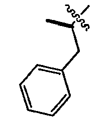 | 440.3 | |
| C.30 | 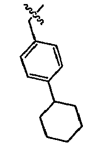 | 494.1 | |
| C.31 | 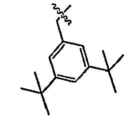 | 524.2 | |
| C.32 | 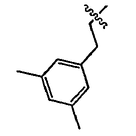 | 454.2 | |
| C.33 | 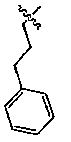 | 438.3 (M-1) | |
| C.34 | 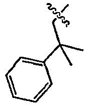 | 454.3 | |
| C.35 | 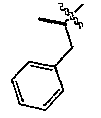 | 440.3 | |

| | | | |
|---|---|---|---|
| ^{a 1}H-NMR (400 MHz, CDCl₃) | | | |

Intermediate 16. **5,6-dimethoxy-3-phenethyloxy-benzo[*b*]thiophene-2-carboxylic acid methyl ester.** To a solution of 3-hydroxy-5,6-dimethoxy-benzo[b]thiophene-2-carboxylic acid methyl ester (Intermediate 15, 0.2 g, 0.75 mmol) (prepared as described in U.S. Patent No. 3,954,748) in THF (28mL) under N₂ was added PS-triphenylphosphine (polystyrene-triphenylphosphine) (1.38 g, 2.23 mmol) and the suspension stirred for 15 min then cooled to 0°C. Diethylazodicarboxylate (DEAD) (0.37 mL, 2.35 mmol) was added and reaction mixture stirred 25 min followed by addition of phenethyl alcohol (0.25 mL, 2.09 mmol). The reaction was stirred for 5 min then warmed to room temperature and stirred 2.5 h. The resin was filtered, and washed with alternating diethyl ether and methylene chloride (3 x 50mL each). The resulting filtrates were concentrated to a crude bright yellow paste (1.04 g) of the title product which was used without further purification in the next step.

Intermediate 17. **5,6-dimethoxy-3-phenethyloxy-benzo[*b*]thiophene-2-carboxylic acid.** Potassium hydroxide (0.746 g, 13.32 mmol) was added to a solution of Intermediate 16 (1.48 mmol) in methanol (42 ml). The reaction mixture was heated to reflux forming an orange solution and stirred overnight. After cooling to room temperature, the solution was acidified to pH ~ 3 with concentrated HCl then diluted with excess water to obtain a beige precipitate. The solid was filtered, rinsed with water and dried to provide 0.489 g of the title product.

Example C.1. **5,6-Dimethoxy-3-phenethyloxy-benzo[b]thiophene-2-carboxylic acid (1H-tetrazol-5-yl)-amide.** Intermediate 17 was converted to the title product (0.0449 g, 73% yield) in an analogous manner to the synthesis of Intermediate 7.

Intermediate 18. **3-(3-Bromo-benzyloxy)-5,6-dimethoxy-benzo[b]thiophene-2-carboxylic acid methyl ester.** To Intermediate 15 (0.2 g, 0.75 mmol) in acetonitrile (8 ml) was added di-isopropyl ethyl amine (Hunig's base) (0.097g, 0.75 mmol) followed by 1-bromo-3-bromomethyl-benzene (0.19g, 0.76 mmol). The resulting mixture was stirred at 70 °C overnight. The mixture was cooled and partitioned between Et₂O and H₂O. The layers were separated and the organic phase was washed with satd. aqu. Na₂CO₃, saturated aqueous NaCl and dried over MgSO₄. Recrystallization from Et₂O provided 0.08g (24%) of the title product. MS: M+1=437.0 (APCI). ¹H-NMR (400 MHz, CDCl₃) □ 7.71 (s, 1H), 7.46 (m, 2H), 7.38-7.0 (m, 3H), 5.51 (s, 2H), 3.98 (s, 3H), 3.96, (s, 3H), 3.87, (s, 3H).

Intermediate 19. **3-(3-Bromo-benzyloxy)-5,6-dimethoxy-benzo[b]thiophene-2-carboxylic acid.** Intermediate 18 (0.0.07 g, 0.16 mmol) was stirred with 1.0 M NaOH aq. (0.48 mL, 0.48 mmol), MeOH (5.0 mL) at room temperature for 16 hours. The mix was diluted with saturated aqueous sodium bicarbonate and washed twice with diethyl ether. The aqueous portion was acidified with 6 N HCl. The acid was extracted with 1:1 EtOAc/ Et₂O. The organic extracts were washed with brine, dried over Mg₂SO₄ to give the title product (0.03 g, 43 % yield). MS: M-CO₂=375.9 (APCI). ¹H-NMR (400 MHz, CDCl₃) δ 7.62 (s, 1H), 7.36 6.89 (m, 5H), 5.22 (s, 2H), 3.85 (s, 3H), 3.76.

Example C.2. **3-(3-Bromo-benzyloxy)-5,6-dimethoxy-benzo[b]thiophened-2-carboxylic acid (1H-tetrazol-5-yl)-amide.** Intermediate 19 (0.02 g, 0.05 mmol) in DMF (5 ml) was treated with EDAC HCl (0.011 g, 0.055 mmol), HOBT (0.0074 g, 0.055 mmol) and 5-aminotetrazole (0.006 g, 0.055 mmol). The resulting mixture was stirred overnight at room temperature. Water was added and the product was extracted twice with 1:1 Et₂O/ EtOAc. The combined organic extracts were washed three times with saturated aqueous NaCl and then dried. The title product was recrystallized from methanol to afford 0.005 g (20 %). Microanalysis: Calcd: C, 46.54; H, 3.29; N, 14.28. Found: C, 46.36; H, 3.26; N, 13.88. MS: M+1= 492.1 (APCI). ¹H-NMR (400 MHz, CDCl₃) δ 7.62 (s, 1H), 7.36 6.89 (m, H), 5.22 (s, 2H), 3.85 (s, 3H), 3.76.

The title compounds of Examples C.10-C.35 were synthesized in a manner analogous to Example C.1 by substituting an appropriately substituted alcohol for phenethyl alcohol as in the synthesis of Intermediate 17.
Examples C.3-C.9 were synthesized in a manner analogous to Example C.2 by substituting an appropriately substituted bromomethylbenzene or bromomethylnaphthalene for 1-bromo-3-bromomethyl-benzene.
Example C.3. **3-(3,4-Difluoro-benzyloxy)-5,6-dimethoxy-benzo[b]thiophene-2-carboxylic acid (1H-tetrazol-5-yl)-amide.**
Example C.4. **3-(Biphenyl-4-ylmethoxy)-5,6-dimethoxy-benzo[b]thiophene-2-carboxylic acid (1H-tetrazol-5-yl)-amide.**
Example C.5. **5,6-Dimethoxy-3-(3-trifluoromethyl-benzyloxy)-benzo[b]thiophene-2-carboxylic acid (1H-tetrazol-5- yl)-amide.**
Example C.6. **3-(3,5-Dimethoxy-benzyloxy)-5,6-dimethoxy-benzo[b]thiophene-2-carboxylic acid (1H-tetrazol-5-yl)-amide.**
Example C.7. **3-(4-tert-Butyl-benzyloxy)-5,6-dimethoxy-benzo[b]thiophene-2-carboxylic acid (1H-tetrazol-5-yl)-amide.**
Example C.8. **5,6-Dimethoxy-3-(2,3,6-trifluoro-benzyloxy)-benzo[b]thiophene-2-carboxylic acid (1H-tetrazol-5-yl)-amide.**
Example C.9. 5,6-**Dimethoxy-3-(naphthalen-2-ylmethoxy)-benzo[b]thiophene-2-carboxylic acid (1H-tetrazol-5-yl)-amide.**
Example C.10. **5,6-Dimethoxy-3-((R)-1-methyl-2-phenyl-ethoxy)-benzo[b]thiophene-2-carboxylic acid (1H-tetrazol-5-yl)-amide.**
Example C.11. **5,6-Dimethoxy-3-(pyridin-3-ylmethoxy)-benzo[b]thiophene-2-carboxylic acid (1H-tetrazol-5-yl)-amide.**
Example C.12**. 5,6-Dimethoxy-3-(3-phenoxy-benzyloxy)-benzo[b]thiophene-2-carboxylic acid (1H-tetrazol-5-yl)-amide.**
Example C.13. **3-(2,2-Diphenyl-ethoxy)-5,6-dimethoxy-benzo[b]thiophene-2-carboxylic acid (1H-tetrazol-5-yl)-amide.**
**Example C.14. 5,6-Dimethoxy-3-(pyridin-2-ylmethoxy)-benzo[b]thiophene-2-carboxylic acid (1H-tetrazol-5-yl)-amide.**
Example C.15. **5,6-Dimethoxy-3-(2-quinolin-5-yl-ethoxy)-benzo[b]thiophene-2-carboxylic acid (1H-tetrazol-5-yl)-amide.**
Example C.16. **3-[2-(3,4-Dimethoxy-phenyl)-ethoxy]-5,6-dimethoxy-benzo[b]thiophene-2-carboxylic acid (1H-tetrazol-5-yl)-amide.**
Example C.17. **5,6-Dimethoxy-3-(2-naphthalen-1-yl-ethoxy)-benzo[b]thiophene-2-carboxylic acid (1H-tetrazol-5-yl)-amide.**
Example C.18. **5,6-Dimethoxy-3-(pyridin-4-ylmethoxy)-benzo[b]thiophene-2-carboxylic acid (1H-tetrazol-5-yl)-amide.**
Example C.19. **3-(3-Dimethylamino-benzyloxy)-5,6-dimethoxy-benzo[b]thiophene-2-carboxylic acid (1H-tetrazol-5-yl)-amide.**
Example C.20. **3-(2-Cyclohexylmethoxy-benzyloxy)-5,6-dimethoxy-benzo[b]thiophene-2-carboxylic acid (1H-tetrazol-5-yl)-amide.**
Example C.21. **5,6-Dimethoxy-3-[2-(5,6,7,8-tetrahydro-naphthalen-1-yl)-ethoxy]-benzo[b]thiophene-2-carboxylic acid (1H-tetrazol-5-yl)-amide.**
Example C.22. **5,6-Dimethoxy-3-(naphthalen-1-ylmethoxy)-benzo[b]thiophene-2-carboxylic acid (1H-tetrazol-5-yl)-amide.**
Example C.23. **5,6-Dimethoxy-3-((S)-2-phenyl-propoxy)-benzo[b]thiophene-2-carboxylic acid (1H-tetrazol-5-yl)-amide.**
Example C.24. **5,6-Dimethoxy-3-(5,6,7,8-tetrahydro-naphthalen-1-ylmethoxy)-benzo[b]thiophene-2-carboxylic acid (1H-tetrazol-5-yl)-amide.**
Example C.25. **3-(3-Cyclopentyloxy-benzyloxy)-5,6-dimethoxy-benzo[b]thiophene-2-carboxylic acid (1H-tetrazol-5-yl)-amide.**
Example C.26. **3-(3-Cyclohexylmethoxy-benzyloxy)-5,6-dimethoxy-benzo[b]thiophene-2-carboxylic acid (1H-tetrazol-5-yl)-amide.**
Example C.27. **3-[2-(4-Dimethylamino-phenyl)-ethoxy]-5,6-dimethoxy-benzo[b]thiophene-2-carboxylic acid (1H-tetrazol-5-yl)-amide.**
Example C.28. **5,6-Dimethoxy-3-(5,6,7,8-tetrahydro-naphthalen-2-ylmethoxy)-benzo[b]thiophene-2-carboxylic acid (1H -tetrazol-5-yl)-amide.**
Example C.29. **5,6-Dimethoxy-3-((S)-1-methyl-2-phenyl-ethoxy)-benzo[b]thiophene-2-carboxylic acid (1H-tetrazol-5-yl)-amide.**
Example C.30. **3-(4-Cyclohexyl-benzyloxy)-5,6-dimethoxy-benzo[b]thiophene-2-carboxylic acid (1H-tetrazol-5-yl)-amide.**
Example C.31. **3-(3,5-Di-tert-butyl-benzyloxy)-5,6-dimethoxy-benzo[b]thiophene-2-carboxylic acid (1H-tetrazol-5-yl)-amide.**
Example C.32. **3-[2-(3,5-Dimethyl-phenyl)-ethoxy]-5,6-dimethoxy-benzo[b]thiophene-2-carboxylic acid (1H-tetrazol-5-yl)-amide.**
Example C.33. **5,6-Dimethoxy-3-(3-phenyl-propoxy)-benzo[b]thiophene-2-carboxylic acid (1H-tetrazol-5-yl)-amide.**
Example C.34. **5,6-Dimethoxy-3-(2-methyl-2-phenyl-propoxy)-benzo[b]thiophene-2-carboxylic acid (1H-tetrazol-5-yl)-amide.**
Example C.35. **5,6-Dimethoxy-3-((R)-2-phenyl-propoxy)-benzo[b]thiophene-2-carboxylic acid (1H-tetrazol-5-yl)-amide.**

### EXAMPLES D.1-D.9

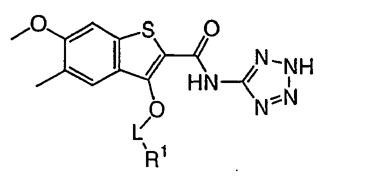

| Ex. | L-R¹ | MS (M+1) | NMR*^{a}* |
|---|---|---|---|
| D.1 | 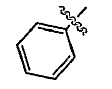 | 382.0 | |
| D.2 | 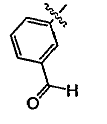 | 410.1 | |
| D.3 | 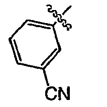 | 407.1 | |
| D.4 | 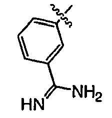 | 422.1 | |
| D.5 | 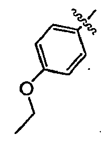 | 426 | 16.06 (s, 1H), 11.27 (s, 1H), 7.64 (s, 1H), 7.14 (s, 1H), 6.94-7.04 (m, 2H), 6.83-6.91 (m, 2H), 3.94 (q, *J* = 7.0 Hz, 2H), 3.88 (s, 3H), 2.11 (s, 3H), 1.27 (t, *J* = 6.9 Hz, 3H). |
| D.6 | 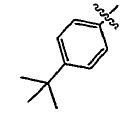 | 438 | 16.02 (s, 1H), 11.45 (s, 1H), 7.62 (s, 1H), 7.26-7.34 (m, 2H), 7.15 (s, 1H), 6.86-6.92 (m, 2H), 3.85 (s, 3H), 2.09 (s, 3H), 1.18 (s, 9H) |
| D.7 | 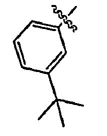 | 438 | 15.99 (s, 1H), 11.47 (s, 1H), 7.61 (s, 1H), 7.04-7.23 (m, 4H), 6.63 (dd, *J* = 8.0, 1.7 Hz, 1H), 3.85 (s, 3H), 2.08 (s, 3H), 1.17 (s, 9H) |
| D.9 | 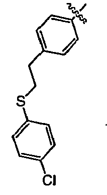 | 552.0 | |
| D.10 | 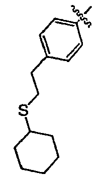 | 522.0 | |

Example D.1. **6-Methoxy-5-methyl-3-phenoxy-benzo[*b*]thiophene-2-carboxylic acid (2H-tetrazol-5-yl)-amide** was synthesized in an analogous manner to Example A.1 with the following exceptions: 3-(3-methyl-4-methoxy-phenyl)-acrylic acid (Intermediate 20) starting material was prepared from 3-methyl-4-methyloxybenzaldehyde in an analogous manner to the synthesis of Intermediate 12. MS: M+1=193.1 (APCI). ¹H-NMR (400 MHz, CDCl₃) δ 7.72 (d, *J* = 16 Hz, 1H), 7.37 (m, 2H), 6.83 (d, *J* = 8.8 Hz, 1H), 6.31 (d, *J* =15.6 Hz, 1H), 3.87 (s, 3H), 2.23 (s, 3H).

Examples D.2 to D.9 were synthesized in a manner analogous to Example D.1 by by replacing phenol as R¹-L-OH with an appropriately substituted phenol or other alcohol.
Example D.2. **3-(3-Formyl-phenoxy)-6-methoxy-5-methyl-benzo[*b*]thiophene-2-carboxylic acid (2H-tetrazol-5-yl)-amide.**
Example D.3. **3-(3-Cyano-phenoxy)-6-methoxy-5-methyl-benzo[*b*]thiophene-2-carboxylic acid (2H-tetrazol-5-yl)-amide.**
Example D.4. **3-(3-Carbamimidoyl-phenoxy)-6-methox-y-5-methyl-benzo[*b*]thiophene-2-carboxylic acid (2H-tetrazol-5-yl)-amide. Anhydrous**

HCl gas was bubbled through a chilled (-78 °C) solution of Example D.3 (0.034 g, 0.084 mmol) in anhydrous ethanol (4 mL) for five minutes. The reaction was then stirred at room temperature for 16 hours and then was concentrated. The residue was then stirred in 2.0M NH₃ in ethanol (4 mL) at room temperature for 16 hours. The mix was concentrated to give the title compound (0.024 g, 69% yield). MS: M-1 = 422.1 (APCI).
Example D.5. **3-(4-Ethoxy-phenoxy)-6-methoxy-5-methyl-benzo[*b*]thiophene-2-carboxylic acid (2H-tetrazol-5-yl)-amide.**
Example D.6. **3-(4-*ter*t-Butyl-phenoxy)-6-methoxy-5-methyl-benzo[*b*]thiophene-2-carboxylic acid (2H-tetrazol-5-yl)-amide.**
Example D.7. **3-(3-t*ert*-Butyl-phenoxy)-6-methoxy-5-methyl-benzo[*b*]thiophene-2-carboxylic acid (2H-tetrazol-5-yl)-amide.**
Example D.8. **3-{4-[3-(4-Chlorophenenyl)sulfanylpropyl]phenoxy}-6-methoxy-5-methylbenzo[*b*]thiophene-2-caboxylic acid(1H-tetrazolyl)amide trifluroacetic acid salt.** Intermediate 20 was converted to 3-chloro-6-methoxy-5-methyl-benzo[b]thiophene-2-carbonyl chloride (Intermediate 21) under the same conditions used to prepare Intermediate 1. 3-{4-[3-(4-Chorophenenyl) sulfanylpropyl]phenoxy}-6-methoxy-5-methylbenzo[*b*]thiophene-2-caboxylic acid(1H-tetrazolyl)amide trifluroacetic acid salt (Example D.8) was prepared from Intermediate 21 in an analogous manner to Example A.9 by substituting 4-[2-(4-Chloro-phenylsulfanyl)-ethyl]-phenol for phenol.
Example D.9. **3-{4-[3-(4-Cyclohexyl)sulfanylpropyl]phenoxy}-5-methoxy-6-methylbenzo[*b*]thiophene-2-carboxylic acid(1H-tetrazolyl) amide trifluroacetic acid salt** was synthesized in an analogous manner to Example D.8 by substituting 4-(2-Cyclohexylsulfanyl-ethyl)-phenol for 4-[2-(4-Chloro-phenylsulfanyl)-ethyl]-phenol.

### EXAMPLES H.1 AND H.2

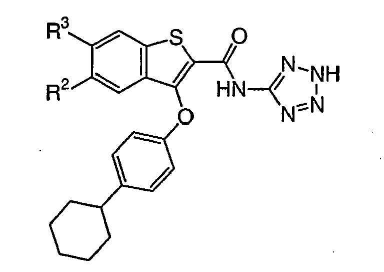

| Ex. | R² | R³ | MS (M+1) |
|---|---|---|---|
| H.1 | HO- | CH₃- | 450.1 |
| H.2 | CHF₂O- | CH₃- | 500.1 |

Example H.1. **3-(4-Cyclohexyl-phenoxy)-5-hydroxy-6-methyl-benzo[*b*]thiophene-2-carboxylic acid (2H-tetrazol-5-yl)-amide.** Boron tribromide (0.837 mL, 8.85 mmol) was added dropwise to a stirring -78°C solution of 3-(4-Cyclohexyl-phenoxy)-5-methoxy-6-methyl-benzo[*b*]thiophene-2-carboxylic acid (as prepared in the synthesis of Example B.4, 0.700 g, 1.77 mmol) in anhydrous CH₂Cl₂ (9 mL). The reaction was stirred at -78 °C for ten minutes and then at room temperature for 45 minutes. More boron tribromide (0.800 mL, 8.46 mmol) was added and the reaction was stirred at room temperature for 40 minutes. The reaction was carefully quenched dropwise with 1:1 MeOH-H₂O. The quenched mix was diluted with 1N HCl and stirred at room temperature for one hour. The mix was extracted with CH₂Cl₂. The extracts were filtered and concentrated. The residue was dissolved in a minimum of CH₂Cl₂ and titurated with hexanes to give a solid precipitate. The solid was filtered and dried to give 3-(4-Cyclohexyl-phenoxy)-5-hydroxy-6-methyl-benzo[*b*]thiophene-2-carboxylic acid (Intermediate 21,0.510 g, 75% yield). MS: M+1 = 383.1 (APCI). Intermediate 21 was coupled with 5-aminotetrazole as in the synthesis of intermediate 13 to give the title product.

Example H.2. **3-(4-Cyclohexyl-phenoxy)-5-difluoromethoxy-6-methyl-lienzo[*b*]thiophene-2-carboxylic acid (2H-tetrazol-5-yl)-amide.** NaOH (1.6g dissolved in 1.6 mL H₂O) was added to a 0 °C solution of Intermediate 21 (0.310 g, 0.811 mmol) in dioxane (8 mL) in a three necked flask fitted with a cold finger chilled to -78 °C. Chlorodifluoromethane (~10 mL) was condensed into the flask. The reaction was heated to 75 °C (with the cold finger maintained at -78 °C) for four hours. The reaction was allowed to cool to room temperature and then was acidified with 1N HCl. The reaction was extracted several times with EtOAc. The extracts were washed with brine, dried over Na₂SO₄, filtered through celite, and concentrated.. The product was purified by silica gel flash chromatography (0-100% acetone-CH₂Cl₂ gradient elution) to give 3-(4-Cyclohexyl-phenoxy)-5-difluoromethoxy-6-methyl-benzo[*b*]thiophene-2-carboxylic acid (0.202 mg, 57% yield). MS: M+1=433.0 (APCI). 3-(4-Cyclohexyl-phenoxy)-5-difluoromethoxy-6-methyl-benzo[*b*]thiophene-2-carboxylic acid was coupled to 5-aminotetrazole as in the synthesis of intermediate 13 to give the title product.

### EXAMPLES K.1-K.2

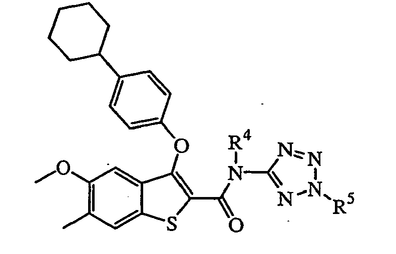

| Ex. | R⁴ and R⁵ | MS | Micoranalysis or Melting Point (°C) |
|---|---|---|---|
| K.1 | R⁴ is hydrogen and R⁵ is methyl | 478.3 | Microanalysis: C₂₅H₂₇N₅O₃S: predicted - C, 62.87; H, 5.70; N, 14.66; O, 10.05; S, 6.71. Found -C, 62.19; H, 5.57; N, 14.38 |
| K.2 | R⁴ is methyl and R⁵ is hydrogen | 476.2 (M-1) | MP: 197-198°C |

Example K.1. **3-(4-Cyclohexyl-phenoxy)-5-methoxy-6-methyl-benzo[*b*]thiophene-2-carboxylic acid (2-methyl-2H-tetrazol-5-yl)-amide.** 3-(4-Cyclohexyl-phenoxy)-5-methoxy-6-methyl-benzo[*b*]thiophene-2-carboxylic acid (2H-tetrazol-5-yl)-amide (0.400 g, 0.863 mmol, Example B.4) was stirred with diisopropyl ethyl amine (0.420mL, 2.41 mmol) in dichloromethane (6mL) at room temperature for one hour. The mix was chilled to 0 °C and methyl iodide (0.096mL, 1.90 mmol) was added. The cold bath was removed and the reaction was stirred at room temperature for 3.5 hours. Diisopropyl ethyl amine (0.060mL) and methyl iodide (0.020mL) were added and the mix was stirred at room temperature for 18 hours. More diisopropyl ethyl amine (0.060mL) and methyl iodide (0.020mL) were added and the reaction was stirred at room temperature another 18 hours. The reaction was quenched with 1M HCl and was extracted three times with ethyl acetate. The organic extracts were washed with H₂O then brine and then were concentrated. The crude product was passed through a silica gel column with 45% diethyl ether/hexanes. The collected material was concentrated and triturated with diethyl ether to give the title product (0.070 g, 37% yield).

Example K.2. **3-(4-Cyclohexyl-phenoxy)-5-methoxy-6-methyl-benzo[*b*]thiophene-2-carboxylic acid methyl-(2H-tetrazol-5-yl)-amide.** 3-(4-Cyclohexyl-phenoxy)-5-methoxy-6-methyl-benzo[b]thiophene-2-carboxylic acid (as prepared in Example B.4) was converted to 3-(4-cyclohexyl-phenoxy)-5-methoxy-6-methyl-N-cyano-benzo[b]thiophene-2-carboxamide in a manner analogous to the 5-amino-tetrazole coupling reaction of Example B.1, with the exception that cyanamide was substituted for amino-tetrazole.

3-(4-cyclohexyl-phenoxy)-5-methoxy-6-methyl-N-cyano-benzo[b]thiophene-2-carboxamide (0.115g, 0.273 mmol) was refluxed with sodium hydride (0.008g, 0.273 mmol) and methyl iodide (0.069mL, 1.37 mmol) in DMF for two hours. The reaction was blown dry under a constant nitrogen stream for 16 hours. The residue was purified by flash chromatography (SiO₂, 10%Et₂O-Hexanes → 20% Et₂O-Hexanes) to give 3-(4-cyclohexyl-phenoxy)-5-methoxy-6-methyl- N-cyano-N-methyl-benzo[b]thiophene-2-carboxamide (0.110 g, 93% yield).

3-(4-cyclohexyl-phenoxy)-5-methoxy-6-methyl- N-cyano-N-methyl-benzo[b]thiophene-2-carboxamide (0.158g, 0.364 mmol) was dissolved in toluene (5mL). Sodium azide (0.071g, 1.09 mmol) and then triethyl ammonium chloride (0.150 g, 1.09 mmol) were added. The reaction was heated to reflux for 2.5 hours. The mix was allowed to cool to room temperature and was diluted with H₂O. Several drops of concentrated HCl were added and the mix was extracted twice with ethyl acetate. The organic extracts were washed with H₂O, washed with brine, dried over MgSO₄, and concentrated. Analysis indicated residual unreacted starting material. The crude product was resubmitted to identical reaction conditions and then worked up as before. The crude product was slurried in a minimum of MeOH and filtered. The filter cake was rinsed sparingly with MeOH. The solid filter cake material was collected and dried under high vacuum to give the title compound as a white solid (0.075g, 43% yield).

### Example L.1:

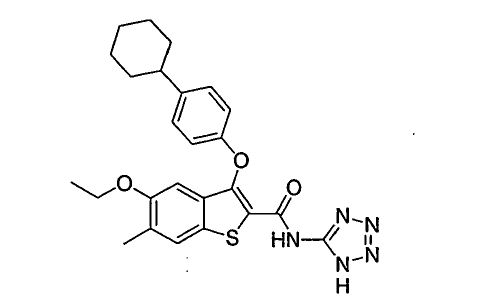

Intermediate 22- **3-(4-Cyclohexyl-phenoxy)-5-hydroxy-6-methyl-benzo[*b*]thiophene-2-carboxylic acid.** To an ice-cold solution of 3-(4-Cyclohexyl-phenoxy)-5-methoxy-6-methyl-benzo[b]thiophene-2-carboxylic acid (0.25 g, 0.630 mmol) in CH₂Cl₂ (70 mL) was added BBr₃ dropwise via syringe. The mixture was stirred at 0°C, and was allowed to warm to ambient temperature. After 12 hours, the reaction was quenched by the addition of water (10 mL). The solution was cooled to 0°C, and solid precipitated. The solid was filtered washed with water, dried under high vacuum pump to give the title product (0.22 g, 95 % yield) as an off white solid. MS: M+1= 365.1(-OH) (APCI). H¹-NMR (400 MHz, DMSO) δ 10.15 (s, 1H), 7.99 (s, 1H), 7.85 (s, 1H), 7.78 (s, 3H), 7.50 (s, 1H), 2.71(m, 1H), 2.32 (s, 3H), 1.93-1.68 (m, 4H), 1.56-1.12 (m, 6H).

Intermediate 23- **3-(4-cyclohexyl-phenoxy)-5-ethoxy-6-methyl-benzo[*b*]thiophene-2-carboxylic acid ethyl ester.** To a stirring solution of intermediate 22 (0.22 g, 0.57 mmol) in DMF (20 mL), and cesium carbonate (0.47 g, 1.43 mmol) was added iodoethane (0.50 mL, 5.70 mmol). The reaction mixture was stirred at room temperature. After 12 hours, the reaction was quenched by the addition of water (20 mL). The solution was cooled to 0°C, and solid precipitated. The solid was filtered washed with water, dried under high vacuum pump to give the title product (0.24 g, 95 % yield) as an off white. MS: M+1= 439.1 (APCI). H¹-NMR (400 MHz, CDCl₃) δ 8.14 (s, 1H), 7.58 (d, 4H), 7.41 (s, 1H), 4.17 (q, 2H), 2.62 (m, 1H), 2.38 (s, 3H), 2.32 (s, 3H), 1.96-1.72 (m, 6H), 1.38-1.20 (m, 9H).

Intermediate 24**-3-(4-cyclohexyl-phenoxy)-5-ethoxy-6-methyl-benzo[*b*]thiophene-2-carboxylic acid.** A solution of intermediate 23 (0.24 g, 0.55 mmol) and KOH (0.30 g, 5.50 mmol) in EtOH (40 mL) was heated to reflux for 2 hours. The mixture was cooled to ambient temperature and concentrated under reduced pressure. The residue was diluted with water (10 mL) and washed with diethyl ether. The aqueous portion was acidified with IN HCl. The solid was precipitated out upon cooling the filtered and washed with water, dried under high vacuum pump to give the title product (0.21 g, 95%). MS: M+1= 411.1 (APCI). H¹-NNM (400 MHz, CDCl₃) δ 7.56 (s, 1H), 7.06 (d, 2H), 6.06 (d, 2H), 6.73 (s, 1H), 3.83 (q, 2H), 2.41 (m, 1H), 2.27 (s, 3H), 1.82-1.66 (m, 5H), 1.39-1.17 (m, 8H).

Example L.1- **3-(4-cyclohexyl-phenoxy)-5-ethoxy-6-methyl-benzo[*b*]thiophene-2-carboxylic acid (1H-tetrazol-5-yl)-amide.** A solution of intermediate 24 (0.15 g, 0.365 mmol) and oxalylchloride (0.13 mL, 1.46 mmol) in CH₂Cl₂ (20 mL) was added 1 drop of DMF. The mixture was stirred at ambient temperature for two hours and then concentrated under reduced pressure. The resulting residue was diluted with CH₂Cl₂ (20 mL) then were added triethyl amine (0.13 mL, 0.91 mmol) and 5-aminotetrazole (0.08 g, 0.91 mmol). The reaction mixture was stirred at ambient temperature. After 12 hours, the mixture was concentrated under reduced pressure. The residue was dissolved in ethyl acetate (100 mL) then washed with 1N HCl (30 mL), then brine, dried over MgSO₄, filtered, and evaporated to dryness under reduced pressure to give oil which solidified upon standing. This solid was purified by prep HPLC (gradient 20% H₂O to 60% CH₃CN in 1% TFA) to give the title product as a white solid (30 mg, 17%). MS: M+1= 478.1 (APCI). H¹-NMR (400 MHz, CDCl₃) δ 10.35 (s, 1H), 7.65 (s, 1H), 7.21 (d, 2H), 7.05 (d, 2H), 6.39 (s, 1H), 3.63 (q, 2H), 2.51 (m, 1H), 2.29 (s,3H), 1.82-1.71 (m, 5H), 1.53-1.21 (m, 8H).

### BIOLOGICAL EXAMPLE 1

### PI3Kγ Protein Expression and Purification Protocol

Spodtera frugiperda cells, grown in ESF921 media, were coinfected with baculovirus expressing a glu-tagged p101 and baculovirus expressing an HA-tagged p110γ, at a 3:1 ratio of p101 baculovirus to p110γ baculovirus. Sf9 cells were grown to 1 × 10⁷ total cells/mL in 10L bioreactors and harvested 48-72 hours post infection. Samples of infected cells were then tested for expression of p101/p110γ PI3 kinase by immunoprecipitation and Western Blot analysis methods (see below).

To purify PI3Kγ, 4 volumes of room temperature hypotonic lysis buffer (1 mM MgCl₂, 1 mM DTT, 5 mM EGTA, 1 mM Pefabloc, 0.5 µM aprotinin, 5 µM leupeptin, 2 µM pepstatin, 5 µM E64, pH 8) per gram of cell paste, was poured onto frozen cell pellets with stirring, then lysed in a nitrogen "bomb" at 400 psi (599HC T316, Parr Instrument Co, Moline, IL). NaCl was added to 150 mM, and sodium cholate was added to 1% and mixed for another 45 minutes. The lysates were clarified by centrifugation for 25 minutes at 14,000 rpm. The lysates were then loaded over anti-glu-linked Protein-G Sepaharose beads (Covance Research Products, Richmond, CA) using 20 mL resin/50 g cell paste. The column was washed with 15 volumes of wash buffer (1 mM DTT, 0.2 mM EGTA, 1 mM Pefabloc, 0.5 µM aprotinin, 5 µM leupeptin, 2 µM pepstatin, 5 µM E64, 150 mM NaCl, 1% sodium cholate, pH 8). PI3Kγ was eluted with 6 column volumes of wash buffer that contain 100 µg/mL of a peptide that competes for binding of the glu tag. The column fractions with the eluted protein (determined by taking OD₂₈₀ readings) were collected and dialyzed in 0.2 mM EGTA, 1 mM DTT, 1 mM Pefabloc, 5 *µ*M leupeptin, 0.5% sodium cholate, 150 mM NaCl, and 50% glycerol, pH 8. The fractions were stored at -80°C until further use.

### BIOLOGICAL EXAMPLE 2

### G Protein Subunits Expression

Spodtera frugiperda cells were coinfected with baculovirus expressing a glu-tagged G protein β₁ and baculovirus expressing a G protein β₂, at a 1:1 ratio of glu-tagged G protein β₁ baculovirus to G protein β₂ baculovirus. Sf9 cells are grown in 10 L bioreactors and harvested 48-72 hours post infection. Samples of infected cells were tested for G protein β₁/β₂ expression by Western Blot analysis, as described below. Cell lysates were homogenized and loaded onto a column of glu-tagged beads as in Biological Example 1 and competed off the column with a glu peptide and processed as described in Biological Example 1.

### BIOLOGICAL EXAMPLE 3

### Western Blot Analysis

Protein samples were run on an 8% Tris-Glycine gel and transferred to a 45 µM nitrocellulose membrane. The blots were then blocked with 5% bovine serum albumin (BSA) and 5% ovalbumin in TBST (50 mM Tris, 200 mM NaCl, 0.1% Tween 20, ph 7.4) for 1 hour at room temperature, and incubated overnight at 4°C with primary antibody diluted 1:1000 in TBST with 0.5% BSA: The primary antibodies for the p110γ, p110α, p110β, p85α, G protein β₁, and G protein γ₂ subunits were purchased from Santa Cruz Biotechnology, Inc., Santa Cruz, CA. The p101 subunit antibodies were developed at Research Genetics, Inc., Huntsville, AL based on a p101 peptide antigen.

After incubation with the primary antibody, the blots were washed in TBST and incubated for 2 hours at room temperaure with goat-anti-rabbit HRP conjugate (Bio-Rad Laboratories, Inc., Hercules, CA, product Number 170-6515), diluted 1:10,000 in TBST with 0.5% BSA. The antibodies were detected with ECL^{™} detection reagents (Amersham Biosciences Corp., Piscataway, New Jersey) and quantified on a Kodak ISO400F scanner.

### BIOLOGICAL EXAMPLE 4

### Immunoprecipitation

100 µL of cell paste from Biological Example 1 or 2 was thawed and lysed on ice with 400 µL of hypotonic lysis buffer (25 mM tris, 1 mM DTT, 1 mM EDTA, 1 mM Pefabloc, 5 µM leupeptin, 5 µM E-64 (Roche), 1% Nonidet P40, pH 7.5-8). The lysate was incubated for 2 hours at room temperature with glutagged beads (Covance Research Products, Cambridge, England, product Number AFC-115P). The beads were washed 3 times in wash buffer (20 mM Tris, pH 7.8-8, 150 mM NaCl₂, 0.5% NP40) and the protein eluted off the beads by heating in 2 times sample buffer (Invitrogen Corporation, Carlsbad, CA, product Number LC1676).

### BIOLOGICAL EXAMPLE 5

### PI3Kγ In Vitro Kinase Assay

The inhibitory properties of the compounds in Table 1 were assayed in an in vitro PI3K assay. In a 96-well polypropylene plate, each well was spotted with 2 µL of 50 times the desired final concentration of compound in DMSO. Purified recombinant p101/p110γ protein (0.03 µg; -2.7 nM) and G protein β₁/_{γ2} subunits (0.09 µg; ~57.7 nM) for each reaction was combined in the assay buffer (30 mM . HEPES, 100 mM NaCl, 1 mM EGTA, and 1 mM DTT). ATP and [γ-³²P-ATP] (0.09 µCi) were added to this mixture so that the final ATP concentration in the reaction was 20 µM. Lipid micelles were formed by sonicating phosphatidylinositol-4,5-diphosphate (PIP₂), phosphatidylethanolamine (PE), and Na-cholate in the assay buffer for 10 minutes, adding MgCl₂ and incubating on ice for 20 minutes, for final concentrations of 25 µM PIP₂, 300 µM PE, 0.02% Na-cholate, and 10 mM MgCl₂ in the reaction. The reactions were started by adding equal volumes lipid and enzyme mixture in a total volume of 50 µL, allowed to run for 20 minutes at room temperature, and stopped with 100 µL 75 mM H₃PO₄. The lipid product was transferred to a glass fiber filter plate and washed with 75 mM H₃PO₄ several times. The presence of radioactive lipid product (PIP₃) was measured by adding Wallac Optiphase mix to each well and counting in a Wallac 1450 Trilux plate reader (PerkinElmer Life Sciences Inc., Boston, MA 02118). The IC₅₀ for each compound tested is reported in µM in Table 1:

**TABLE 1**

| Example No. | IC₅₀ (µM) |
|---|---|
| A.1 | 0.250 |
| A.2 | 0.400 |
| A.3 | 0.415 |
| A.4 | 1.365 |
| A.5 | 1.055 |
| A.6 | 0.860 |
| A.7 | 0.170 |
| A.8 | 0.215 |
| A.9 | 1.200 |
| A.10 | 0.390 |
| A.11 | 0.160 |
| A.12 | 0.495 |
| A.13 | 0.099 |
| A.14 | 0.268 |
| A.15 | 0.119 |
| A.16 | 0.026 |
| B.1 | 0.027 |
| B.2 | 0.018 |
| B.3 | 0.079 |
| B.4 | 0.037 |
| B.5 | 0.520 |
| B.6 | 0.012 |
| B.7 | 0.028 |
| B.8 | 0.033 |
| B.9 | 0.063 |
| B.10 | 0.015 |
| B.11 | 0.086 |
| B.12 | 0.017 |
| B.13 | 0.031 |
| B.14 | 0.365 |
| B.15 | 0.076 |
| B.16 | 0.126 |
| B.17 | 0.004 |
| B.18 | 0.029 |
| B.19 | 0.009 |
| B.20 | 0.077 |
| B.21 | 0.043 |
| B.22 | 0.018 |
| B.23 | 2.335 |
| B.24 | 0.008 |
| B.25 | 0.025 |
| B.26 | 0.067 |
| B.27 | 0.125 |
| B.28 | 0.061 |
| B.29 | 0.275 |
| B.30 | 0.029 |
| B.31 | 0.087 |
| B.32 | 0.036 |
| B.33 | 0.007 |
| B.34 | 0.110 |
| B.35 | 0.027 |
| B.36 | 0.008 |
| B.37 | 0.089 |
| B.38 | 0.016 |
| B.39 | 0.018 |
| B.40 | 0.022 |
| B.41 | 0.135 |
| B.42 | 0.180 |
| B.43 | 0.056 |
| B.44 | 0.165 |
| B.45 | 0.017 |
| B.46 | 0.120 |
| B.47 | 0.058 |
| B.48 | 0.715 |
| B.49 | 0.071 |
| B.50 | 0.051 |
| B.51 | 0.061 |
| B.52 | 1.310 |
| B.53 | 0.365 |
| B.54 | 0.325 |
| B.55 | 0.102 |
| B.56 | 0.056 |
| B.57 | 0.040 |
| B.58 | 0.035 |
| B.59 | 0.025 |
| B.60 | 0.022 |
| B.61 | 0.020 |
| B.62 | 0.014 |
| B.63 | 0.011 |
| B.64 | 0.009 |
| B.65 | 0.009 |
| B.66 | 0.008 |
| B.67 | 0.008 |
| B.68 | 0.008 |
| B.69 | 0.004 |
| B.70 | 0.017 |
| B.71 | 0.275 |
| B.72 | 0.160 |
| B.73 | 0.013 |
| B.74 | 0.012 |
| B.75 | 0.006 |
| C.1 | 0.185 |
| C.2 | 0.346 |
| C.3 | 3.630 |
| C.4 | 2.650 |
| C.5 | 1.617 |
| C.6 | 1.523 |
| C.7 | 0.986 |
| C.8 | 0.564 |
| C.9 | 0.210 |
| C.10 | 1.800 |
| C.11 | 1.480 |
| C.12 | 1.155 |
| C.13 | 0.970 |
| C.14 | 0.885 |
| C.15 | 0.757 |
| C.16 | 0.740 |
| C.17 | 0.715 |
| C.18 | 0.450 |
| C.19 | 0.450 |
| C.20 | 0.410 |
| C.21 | 0.300 |
| C.22 | 0.290 |
| C.23 | 0.255 |
| C.24 | 0.255 |
| C.25 | 0.230 |
| C.26 | 0.215 |
| C.27 | 0.185 |
| C.28 | 0.185 |
| C.29 | 0.145 |
| C.30 | 0.134 |
| C.31 | 0.123 |
| C.32 | 0.117 |
| C.33 | 0.115 |
| C.34 | 0.084 |
| C.35 | 0.042 |
| D.1 | 0.041 |
| D.2 | 0.150 |
| D.3 | 0.275 |
| D.4 | 0.388 |
| D.5 | 0.235 |
| D.6 | 0.890 |
| D.7 | 0.230 |
| D.8 | 2.610 |
| D.9 | 0.240 |
| H.1 | 0.028 |
| H.2 | 0.230 |
| K.1 | 1.930 |
| K.2 | 1.610 |
| L.1 | 0.115 |

## Claims

1. A compound of Formula I: 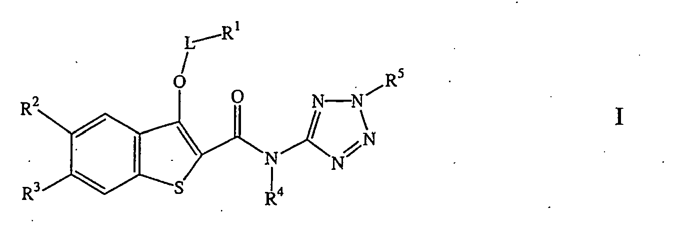 or a pharmaceutically acceptable salt thereof;
wherein R² and R³ are selected from the group consisting of:
(i) R² is methoxy and R³ is selected from the group consisting of H, methyl, and methoxy;
(ii) R² is methyl and R³ is methoxy;
(iii) R² is -O-CHF₂ and R³ is methyl;
(iv) R² is -OH and R³ is methyl;
(v) R² is cyclopropyloxy and R³ is selected from the group consisting of H, methyl, and methoxy;
(vi) R² is -O-CHF₂, and R³ is cyclopropyloxy; and
(vii) R² is ethoxy and R³ is methyl;
wherein R⁴ is H or CH₃;
wherein R⁵ is H or CH₃;
wherein L is absent, a C₁-C₄ alkylene, or a C₁-C₄ alkylene-C(O)-;
wherein R¹ is a substituted phenyl that is substituted as in group (a), (b), (c), or (d); or
R¹ is an optionally substituted group selected from the group consisting of: naphthalenyl, a 5-membered heteroaryl, 6-membered heteroaryl, pyrimidinyl, pyridinyl, quinolinyl, and indanyl, wherein said optionally substituted group can be substituted as in (a), (b), (c), or (d);
wherein said group (a), (b), (c), or (d) are:
(a) 1 to 3 substituents independently selected from the group consisting of:
Br, F, Cl, -CN, -NO₂, -CF₃, -OH, -OCF₃, -SO₂-CH₃, C₁-C₄ alkyl, -CH₂CH₂-Br, -CH₂CH₂-S-(t-butyl), O-C₁-C₆alkyl, -C(NH)(NH₂), -NH-C(O)-CH₃, NH₂, N(CH₃)₂; -CH₂-C(O)-O-CH₂CH₃, C(O)-C₁-C₄ alkyl, and C(O)H;
(b) 1 substituent of J-R⁸,
wherein J is absent, -O-, C₁-C₄-alkylene, O-C₁-C₄-alkylene, C₁-C₄-alkylene-C(O)-, or C₁-C₄-alkyleneS-,
wherein R⁸ is an optionally substituted group selected from the group consisting of:
phenyl, piperidinyl, morpholinyl, tetrahydropyranyl, tetrahydrothiopyranyl, 1,1-dioxo-hexahydro-1λ⁶-thiopyranyl, a 5-membered heterocycloalkyl, or a 6-membered heterocycloalkyl, that are optionally substituted with 1 to 3 groups independently selected from the group consisting of:
Br, F, Cl, -CN, -NO₂, -CF₃, -OH, -OCF₃, -SO₂-CH₃, C₁-C₄ alkyl, -O-C₁-C₆alkyl, - C(NH)(NH₂), NH-C(O)-CH₃, NH₂, N(CH₃)₂, - C(O)-NH₂, C(O)=CH₃, -C(O)-C₁-C₄ alkyl, C(O)H, and C(O)-C(CH₃)₂-NH-C(O)-O-t-butyl;
(c) 1 substituent of Z-R⁹,
wherein Z is absent, -O-, -C ₁-C₆alkylene, -O-C ₁-C₆alkylene, -C(O)-, or -CH(OH)-, -C ₁-C₄alkylene-S-, -C₁-C₆alkylene-O-, or C₁-C₄-alkylene-C(O)-;
wherein R⁹ is a C₄-C₇ cycloalkyl optionally substituted with 4 methyl groups, or 1 to 3 groups independently selected from the group consisting of:
=O, Br, F, Cl, -CF₃, -OH, -OCF₃, -SO₂-CH₃, C₁-C₄ alkyl, -C(O)-NH₂, CH₂-O-CH₃, piperidinyl, and 1,3-dioxolan-2-yl; and
(d) 1 or 2 substituents independently selected from (a) and 1 substituent from (b) or (c).

2. The compound of claim 1, wherein R² is methoxy, ethoxy, or -O-CHF₂ and R³ is methyl.

3. The compound of claim 3, wherein R¹ is a substituted phenyl that is substituted as in group (a), (b), (c), or (d); wherein said group (a), (b), (c), or (d) are:
(a) 1 to 3 substituents independently selected from the group consisting of:
Br, F, Cl, -CN, -NO₂, -CF₃, -OH, -OCF₃, -SO₂-CH₃, C₁-C₄ alkyl, -CH₂CH₂-Br, -CH₂CH₂-S-(t-butyl), O-C₁-C₆alkyl;
- CH₂-C(O)-O-CH₂CH₃, and C(O)-C₁-C₄ alkyl;
(b) 1 substituent of J-R⁸,
wherein J is absent, -O-, C₁-C₄-alkylene, O-C₁-C₄-alkylene, C₁-C₄-alkylene-C(O)-, or C₁-C₄-alkylene-S-,
wherein R⁸ is an optionally substituted group selected from the group consisting of:
piperidinyl, morpholinyl, tetrahydropyranyl, tetrahydrothiopyranyl, 1,1-dioxo-hexahydro-1λ⁶-thiopyranyl, that are optionally substituted with 1 to 3 groups independently selected from the group consisting of:
Br, F, Cl, -CN, -NO₂, -CF₃, -OH, -OCF₃, -SO₂-CH₃, C₁-C₄ alkyl, -O-C ₁-C₆alkyl, and -C(O)-NH₂;
(c) 1 substituent of Z- R⁹,
wherein Z is absent, -O-, -C₁-C₆alkylene, -O-C ₁-C₆alkylene, -C ₁-C₆alkylene-O-, or C₁-C₄-alkylene-C(O)-;
wherein R⁹ is a C₄-C₇ cycloalkyl optionally substituted with 1 to 3 groups independently selected from the group consisting of: =O, Br, F, Cl, -CF₃, -OH, -OCF₃, -SO₂-CH₃, C₁-C₄ alkyl, and -C(O)-NH₂; and
(d) 1 or 2 substituents independently selected from (a) and 1 substituent from (b) or (c).

4. The compound of claim 3, wherein said compound is selected from the group consisting of:
{4-[5-Methoxy-6-methyl-2-(2H-tetrazol-5-ylcarbamoyl)-benzo[*b*]thiophene-3-yloxy]-phenyl}-acetic acid ethyl ester;
3-(4-Isopropyl-phenoxy)-5-methoxy-6-methyl-benzo[*b*]thiophene-2-carboxylic acid (2H-tetrazol-5-yl)-amide;
3-(4-Cyclopentyloxy-phenoxy)-5-methoxy-6-methyl-benzo [*b*] thiophene-2-carboxylic acid (2H-tetrazol-5-yl)-amide;
3-(4-tert-Butyl-phenoxy)-5-methoxy-6-methyl-benzo[*b*]thiophene-2-carboxylic acid (2H-tetrazol-5-yl)-amide;
3-(4-Bromo-phenoxy)-5-methoxy-6-methyl-benzo[*b*]thiophene-2-carboxylic acid (2H-tetrazol-5-yl)-amide;
3-(4-Fluoro-phenoxy)-5-methoxy-6-methyl-benzo[*b*]thiophene-2-carboxylic acid (2H-tetrazol-5-yl)-amide;
3-(4-Chloro-2-fluoro-phenoxy)-5-methoxy-6-methyl-benzo[*b*]thiophene-2-carboxylic acid (2H-tetrazol-5-yl)-amide;
5-Methoxy-6-methyl-3-(4-trifluoromethoxy-phenoxy)-benzo[*b*]thiophene-2-carboxylic acid (2H-tetrazol-5-yl)-amide;
3-[4-(1-carbamoyl-cyclopentyl)-phenoxy]-5-methoxy-6-methyl-benzo[b]thiophene-2-carboxylic acid (2H-tetrazol-5-yl)-amide;
5-methoxy-6-methyl-3-[4-(tetrahydro-pyran-4-yl)-phenoxy]-benzo[b]thiophene-2-carboxylic acid(2H-tetrazol-5-yl)-amide;
3-[4-(1,1-Dioxo-hexahydro-1λ⁶-thiopyran-4-yl)-phenoxy]-5-methoxy-6-methyl-benzo[b]thiophene-2-carboxylic acid (2H-tetrazol-5-yl)-amide;
5-Methoxy-6-methyl-3-(2-nitro-4-cyclohexyl-phenoxy)-benzo[b]thiophene-2-carboxylic acid (2H-tetrazol-5-yl)-amide;
3-(2-Chloro-4-cyclohexyl-phenoxy)-5-methoxy-6-methyl-benzo[b]thiophene-2-carboxylic acid (2H-tetrazol-5-yl)-amide; and
3-(2-Cyano-4-cyclohexyl-phenoxy)-5-methoxy-6-methyl-benzo[b]thiophene-2-carboxylic acid (2H-tetrazol-5-yl)-amide.

5. The compound of claim 1, wherein R² is methoxy, methyl, or -OH and R³ is methoxy.

6. The compound of claim 5, wherein R¹ is a substituted phenyl that is substituted as in group (a), (b), (c), or (d); wherein said group (a), (b), (c), or (d) are:
(a) 1 to 3 substituents independently selected from the group consisting of:
Br, F, Cl, -CN, -NO₂, -CF₃, -OH, -OCF₃, -SO₂-CH₃, C₁-C₄ alkyl, -CH₂CH₂-Br, -CH₂CH₂-S-(t-butyl), O-C₁-C₆alkyl, -CH₂-C(O)-O-CH₂CH₃, and C(O)-C₁-C₄ alkyl;
(b) 1 substituent of J-R⁸,
wherein J is absent, -O-, C₁-C₄-alkylene, O-C₁-C₄-alkylene, C₁-C₄-alkylene-C(O)-, or C₁-C₄-alkylene-S-,
wherein R⁸ is an optionally substituted group selected from the group consisting of:
piperidinyl, morpholinyl, tetrahydropyranyl, tetrahydrothiopyranyl, 1,1-dioxo-hexahydro-Iλ⁶-thiopyranyl, that are optionally substituted with 1 to 3 groups independently selected from the group consisting of:
Br, F, Cl, -CN, -NO₂, -CF₃, -OH, -OCF₃, -SO₂-CH₃, C₁-C₄ alkyl, -O-C₁-C₆alkyl, and -C(O)-NH₂;
(c) 1 substituent of Z- R⁹,
wherein Z is absent, -O-, -C₁-C₆alkylene, -O-C₁-C₆alkylene, -C₁-C₆alkylene-O-, or C₁-C₄-alkylene-C(O)-;
wherein R⁹ is a C₄-C₇ cycloalkyl optionally substituted with 1 to 3 groups independently selected from the group consisting of: =O, Br, F, Cl, -CF₃, -OH, -OCF₃, -SO₂-CH₃, C₁-C₄ alkyl, and -C(O)-NH₂; and
(d) 1 or 2 substituents independently selected from (a) and 1 substituent from (b) or (c).

7. The compound of claim 6, wherein said compound is:
3-(2-Cyclohexylmethoxy-benzyloxy)-5,6-dimethoxy-benzo[b]thiophene-2-carboxylic acid (1H-tetrazol-5-yl)-amide; or
3-(4-CycloheXyl-phenoxy)-5-hydroxy-6-methyl-benzo[b]thiophene-2-carboxylic acid (2H-tetrazol-5-yl)-amide.

8. The compound of claim 1, wherein R² is methoxy, and R³ is H.

9. Use of a compound of claim 1 for the preparation of a medicament for treating a subject comprising:
administering, to a subject suffering from a disease selected from the group consisting of: rheumatoid arthritis, osteoarthritis, psoriatic arthritis, psoriasis, inflammatory diseases, autoimmune diseases, respiratory diseases, bronchitis, asthma, and chronic obstructive pulmonary disease, a pharmaceutical composition comprising a therapeutically effective amount of a compound of claim 1 and a pharmaceutically acceptable carrier.

10. Use of a compound of claim 1 for the preparation of a medicament for treating a subject comprising:
administering, to a subject suffering from a disease selected from the group consisting of: cancer, colon cancer, glioblastoma, endometrial carcinoma, hepatocellular cancer, lung cancer, melanoma, renal cell carcinoma, thyroid carcinoma, cell lymphoma, lymphoproliferative disorders, small cell lung cancer, squamous cell lung carcinoma, glioma, breast cancer, prostate cancer, ovarian cancer, cervical cancer, and leukemia, a pharmaceutical composition comprising a therapeutically effective amount of a compound of claim 1 and a pharmaceutically acceptable carrier.

11. Use of a compound of claim 1 for the preparation of a medicament for treating a subject comprising:
administering, to a subject suffering from a disease selected from the group consisting of: cardiovascular diseases, atherosclerosis, hypertension, deep venous thrombosis, stroke, myocardial infarction, unstable angina, thromboembolism, pulmonary embolism, thrombolytic diseases, acute arterial ischemia, peripheral thrombotic occlusions, coronary artery disease, respiratory diseases, bronchitis, asthma, and chronic obstructive pulmonary disease, a pharmaceutical composition comprising a therapeutically effective amount of a compound of claim 1 and a pharmaceutically acceptable carrier.

12. The use of claim 9, 10, or 11, wherein said compound is a compound of any one of claims 1-8.

13. A pharmaceutical composition comprising:
a therapeutically effective amount of a compound of claim 1 and a pharmaceutically acceptable carrier.

14. A pharmaceutical composition comprising:
a therapeutically effective amount of a compound of any one of claims 1-8 and a pharmaceutically acceptable carrier.

## Patentansprüche

1. Verbindung der Formel I: 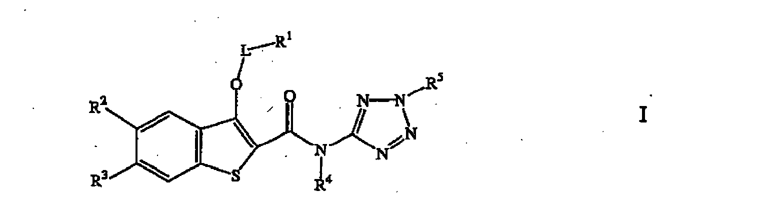 oder ein pharmazeutisch annehmbares Salz davon;
worin R² und R³ ausgewählt sind aus der Gruppe, bestehend aus:
(i) R² ist Methoxy und R³ ist ausgewählt aus der Gruppe, bestehend aus H, Methyl und Methoxy;
(ii) R² ist Methyl und R³ ist Methoxy;
(iii) R² ist -O-CHF₂ und R³ ist Methyl;
(iv) R² ist -OH und R³ ist Methyl;
(v) R² ist Cyclopropyloxy und R³ ist ausgewählt aus der Gruppe, bestehend aus H, Methyl und Methoxy;
(vi) R² ist -O-CHF₂ und R³ ist Cyclopropyloxy; und
(vii) R² ist Ethoxy und R³ ist Methyl;
worin R⁴ H oder CH₃ ist;
worin R⁵ H oder CH₃ ist;
worin L nicht vorhanden, ein C₁-C₄-Alkylen oder ein C₁-C₄-Alkylen-C(O)- ist;
worin R¹ ein substituiertes Phenyl ist, das substituiert ist wie in Gruppe (a), (b), (c) oder (d); oder
R¹ eine gegebenenfalls substituierte Gruppe ist, ausgewählt aus der Gruppe, bestehend aus: Naphthalenyl, einem 5-gliedrigen Heteroaryl', 6-gliedrigen Heteroaryl, Pyrimidinyl, Pyridinyl; Chinolinyl und Indanyl, wobei die gegebenenfalls substituierte Gruppe substituiert sein kann wie in (a), (b), (c) oder (d);
wobei die Gruppe (a), (b), (c) oder (d) ist:
(a) 1 bis 3 Substituenten, unabhängig ausgewählt aus der Gruppe, bestehend aus:
Br, F, Cl, -CN, -NO₂, -CF₃, -OH, -OCF₃, -SO₂-CH₃, C₁-C₄-Alkyl, -CH₂CH₂-Br, -CH₂CH₂-S-(t-Butyl), O-C₁-C₆-Alkyl, -C(NH)(NH₂), -NH-C(O)-CH₃, NH₂, N(CH₃)₂, -CH₂-C(O)-O-CH₂CH₃, C(O)-C₁-C₄-Alkyl und C(O)H;
(b) 1 Substituent von J-R⁸,
wobei J nicht vorhanden, -O-, C₁-C₄-Alkylen, O-C₁-C₄-Alkylen, C₁-C₄-Alkylen-C(O)- oder C₁-C₄-Alkylen-S- ist,
wobei R⁸ eine gegebenenfalls substituierte Gruppe ist, ausgewählt aus der Gruppe, bestehend aus:
Phenyl, Piperidinyl, Morpholinyl, Tetrahydropyranyl, Tetrahydrothiopyranyl, 1,1-Dioxohexahydro-1λ⁶-thiopyranyl, einem 5-gliedrigen Heterocycloalkyl und einem 6-gliedrigen Heterocycloalkyl, die gegebenenfalls substituiert sind mit 1 bis 3 Gruppen, unabhängig ausgewählt aus der Gruppe bestehend aus: Br, F, Cl, -CN, -NO₂, -CF₃, -OH, -OCF₃, -SO₂-CH₃, C₁-C₄-Alkyl, -O-C₁-C₆-Alkyl, -C(NH)(NH₂), NH-C(O)-CH₃, NH₂, N(CH₃)₂, -C(O)-NH₂, C(O)-CH₃, -C(O)-C₁-C₄-Alkyl, C(O)H und C(O)-C(CH₃)₂-NH-C(O)-O-t-Butyl;
(c) 1 Substituent von Z-R⁹,
wobei Z nicht vorhanden, -O-, -C₁-C₆-Alkylen, -O-C₁-C₆-Alkylen, -C(O) - oder -CH (OH) -, -C₁-C₄-Alkylen-S-, -C₁-C₆-Alkylen-O- oder C₁-C₄-Alkylen-C(O)- ist;
wobei R⁹ ein C₄-C₇-Cycloalkyl ist, gegebenenfalls substituiert mit 4 Methylgruppen oder 1 bis 3 Gruppen, unabhängig ausgewählt aus der Gruppe, bestehend aus:
=O, Br, F, Cl, -CF₃, -OH, -OCF₃, -SO₂-CH₃, C₁-C₄-Alkyl, -C(O)-NH₂, CH₂-O-CH₃, Piperidinyl und 1,3-Dioxolan-2-yl; und
(d) 1 oder 2 Substituenten, unabhängig ausgewählt aus (a) und 1 Substituent aus (b) oder (c).

2. Verbindung nach Anspruch 1, wobei R² Methoxy, Ethoxy oder -O-CHF₂ ist und R³ Methyl ist.

3. Verbindung nach Anspruch 3, wobei R¹ ein substituiertes Phenyl ist, das substituiert ist wie in Gruppe (a), (b), (c) oder (d); wobei die Gruppe (a), (b), (c) oder (d) ist:
(a) 1 bis 3 Substituenten, unabhängig ausgewählt aus der Gruppe, bestehend aus:
Br, F, Cl, -CN, -NO₂, -CF₃, -OH, -OCF₃, -SO₂-CH₃, C₁-C₄-Alkyl, -CH₂CH₂-Br, -CH₂CH₂-S-(t-Butyl), O-C₁-C₆-Alkyl , -CH₂-C(O)-O-CH₂CH₃ und C ( O ) -C₁-C₄-Alkyl ;
(b) 1 Substituent von J-R⁸,
wobei J nicht vorhanden, -O-, C₁-C₄-Alkylen, O-C₁-C₄-Alkylen, C₁-C₄-Alkylen-C(O)- oder C₁-C₄-Alkylen-S- ist, wobei R⁸ eine gegebenenfalls substituierte Gruppe ist, ausgewählt aus der Gruppe, bestehend aus:
Piperidinyl, Morpholinyl, Tetrahydropyranyl, Tetrahydrothiopyranyl, 1,1-Dioxohexahydro-1λ⁶-thiopyranyl, die gegebenenfalls substituiert sind mit 1 bis 3 Gruppen, unabhängig ausgewählt aus der Gruppe, bestehend aus:
Br, F, Cl, -CN, -NO₂, -CF₃, -OH, -OCF₃, -SO₂-CH₃, C₁-C₄-Alkyl, -O-C₁-C₆-Alkyl und -C(O)-NH₂;
(c) 1 Substituent von Z-R⁹, wobei Z nicht vorhanden, -O-, -C₁-C₆-Alkylen, -O-C₁-C₆-Alkylen, -C₁-C₆-Alkylen-O- oder C₁-C₄-Alkylen-C (O)-ist,
wobei R⁹ ein C₄-C₇-Cycloalkyl ist, gegebenenfalls substituiert mit 1 bis 3 Gruppen, unabhängig ausgewählt aus der Gruppe, bestehend aus:
=O, Br, F, Cl, -CF₃, -OH, -OCF₃, SO₂-CH₃, C₁-C₄-Alkyl und -C(O)-NH₂ ; und
(d) 1 oder 2 Substituenten, unabhängig ausgewählt aus (a) und 1 Substituent aus (b) oder (c).

4. Verbindung nach Anspruch 3, wobei die Verbindung ausgewählt ist aus der Gruppe, bestehend aus:
{4-[5-Methoxy-6-methyl-2-(2*H*-tetrazol-5-ylcarbamoyl)benzo[*b*]thiophen-3-yloxy]phenyl}essigsäureethylester;
3-(4-Isopropylphenoxy)-5-methoxy-6-methylbenzo[*b*]thiophen-2-carbonsäure(2*H*-tetrazol-5-yl)amid;
3-(4-Cyclopentyloxyphenoxy)-5-methoxy-6-methylbenzo[*b*]thiophen-2-carbonsäure(2*H*-tetrazol-5-yl)amid;
3-(4-tert.-Butylphenoxy)-5-methoxy-6-methylbenzo[*b*]thiophen-2-carbonsäure(2*H*-tetrazol-5-yl)amid;
3-(4-Bromphenoxy)-5-methoxy-6-methylbenzo[*b*]thiophen-2-carbonsäure(2*H*-tetrazol-5-yl)amid;
3-(4-Fluorphenoxy)-5-methoxy-6-methylbenzo[*b*]thiophen-2-carbonsäure(2*H*-tetrazol-5-yl)amid;
3-(4-Chlor-2-fluorphenoxy)-5-methoxy-6-methylbenzo[*b*]thiophen-2-carbonsäure(2*H*-tetrazol-5-yl)amid;
5-Methoxy-6-methyl-3-(4-trifluormethoxyphenoxy)benzo[*b*]thiophen-2-cärbonsäüre(2*H-*tetrazol-5-yl)amid;
3-[4-(1-Carbamoylcyclopentyl)phenoxy]-5-methoxy-6-methyl-benzo[*b*]thiophen-2-carbonsäure(2*H*-tetrazol-5-yl)amid;
5-Methoxy-6-methyl-3-[4-(tetrahydropyran-4-yl)phenoxy]benzo[*b*]thiophen-2-carbonsäure(2H-tetrazol-5-yl)amid;
3-[4-(1,1-Dioxohexahydro-1λ⁶-thiopyran-4-yl)phenoxy]-5-methoxy-6-methylbenzo[*b*]thiophen-2-carbonsäure(2*H*-tetrazol-5-yl)amid;
5-Methoxy-6-methyl-3-(2-nitro-4-cyclohexylphenoxy)benzo[*b*]thiophen-2-carbonsäure(2*H*-tetrazol-5-yl)amid;
3-(2-Chlor-4-cyclohexylphenoxy)-5-methoxy-6-methylbenzo[*b*]thiophen-2-carbonsäure(2*H*-tetrazol-5-yl)amid; und
3-(2-Cyano-4-cyclohexylphenoxy)-5-methoxy-6-methylbenzo[*b*]thiophen-2-carbonsäure(2H-tetrazol-5-yl)amid.

5. Verbindung nach Anspruch 1, wobei R² Methoxy; Methyl oder -OH ist und R³ Methoxy ist.

6. Verbindung nach Anspruch 5, wobei R¹ ein substituiertes Phenyl ist, das substituiert ist wie in Gruppe (a), (b), (c) oder (d); wobei die Gruppe (a), (b), (c) oder (d) ist:
(a) 1 bis 3 Substituenten, unabhängig ausgewählt aus der Gruppe, bestehend aus:
Br, F, Cl, -CN, -NO₂, -CF₃, -OH, -OCF₃, -SO₂-CH₃, C₁-C₄-Alkyl, -CH₂CH₂-Br, -CH₂CH₂-S-(t-Butyl), O-C₁-C₆-Alkyl, - CH₂-C(O)-O-CH₂CH₃ und C(O)-C₁-C₄-Alkyl;
(b) 1 Substituent von J-R⁸, wobei J nicht vorhanden, -O-, C₁-C₄-Alkylen, O-C₁-C₄-Alkylen, C₁-C₄-Alkylen-C(O)- oder C₁-C₄-Alkylen-S- ist, wobei R⁸ eine gegebenenfalls substituierte Gruppe ist, ausgewählt aus der Gruppe, bestehend aus:
Piperidinyl, Morpholinyl, Tetrahydropyranyl, Tetrahydrothiopyranyl, 1,1-Dioxohexahydro-1λ⁶-thiopyranyl, die gegebenenfalls substituiert sind mit 1 bis 3 Gruppen, unabhängig ausgewählt aus der Gruppe, bestehend aus:
Br, F, Cl, -CN, -NO₂, -CF₃, -OH, -OCF₃, -SO₂-CH₃, C₁-C₄-Alkyl, -O-C₁-C₆-Alkyl. und -C (O)-NH₂;
(c) 1 Substituent von Z-R ⁹
wobei Z nicht vorhanden, -O-, -C₁-C₆-Alkylen, -O-C₁-C₆-Alkylen, -C₁-C₆-Alkylen-O- oder C₁-C₄-Alkylen-C(O)-ist,
wobei R⁹ ein C₄-C₇-Cycloalkyl ist, gegebenenfalls substituiert mit 1 bis 3 Gruppen, unabhängig ausgewählt aus der Gruppe, bestehend aus:
=O, Br, F, Cl, -CF₃, -OH, -OCF₃, -SO₂-CH₃, C₁-C₄-Alkyl und -C(O)-NH₂; und
(d) 1 oder 2 Substituenten, unabhängig ausgewählt aus (a) und 1 Substituent aus (b) oder (c).

7. Verbindung nach Anspruch 6, wobei die Verbindung ist:
3-(2-Cyclohexylmethoxybenzyloxy)-5,6-dimethoxybenzo[*b*]thiophen-2-carbonsäure(1*H*-tetrazol-5-yl)amid; oder
3-(4-Cyclohexylphenoxy)-5-hydroxy-6-methylbenzo[*b*]thiophen-2-carbonsäure(2*H*-tetrazol-5-yl)amid.

8. Verbindung nach Anspruch 1, wobei R² Methoxy ist und R³ H ist.

9. Verwendung einer Verbindung nach Anspruch 1 zur Herstellung eines Medikaments zum Behandeln eines Subjekts, umfassend:
Verabreichen an ein Subjekt, das an einer Krankheit leidet, ausgewählt aus der Gruppe, bestehend aus: rheumatoider Arthritis, Osteoarthritis, Arthritis psoriatica, Psoriasis, Entzündungserkrankungen, Autoimmunerkrankungen, Atemwegserkrankungen, Bronchitis, Asthma und chronischobstruktiver Lungenerkrankung, eine pharmazeutische Zusammensetzung, umfassend eine therapeutisch wirksame Menge einer Verbindung nach Anspruch 1 und einen pharmazeutisch annehmbaren Träger.

10. Verwendung einer Verbindung nach Anspruch 1 zur Herstellung eines Medikaments zum Behandeln eines Subjekts, umfassend:
Verabreichen an ein Subjekt, das an einer Krankheit leidet, ausgewählt aus der Gruppe, bestehend aus: Krebs, Darmkrebs, Glioblastom, Endometriumkarzinom, Leberzellkrebs, Lungenkrebs, Melanom, Nierenzellkarzinom, Thyreoideakarzinom, Zelllymphom, lymphoproliferativen Störungen, kleinzelligem Lungenkrebs, Plattenepithelkarzinom der Lunge, Gliom, Brustkrebs, Prostatakrebs, Ovarialkarzinom, Zervixkarzinom und Leukämie, eine pharmazeutische Zusammensetzung, umfassend eine therapeutisch wirksame Menge einer Verbindung nach Anspruch 1 und einen pharmazeutisch annehmbaren Träger.

11. Verwendung einer Verbindung nach Anspruch 1 zur Herstellung eines Medikaments zum Behandeln eines Subjekts, umfassend:
Verabreichen an ein Subjekt, das an einer Krankheit leidet, ausgewählt aus der Gruppe, bestehend aus: kardiovaskulären Erkrankungen, Atherosklerose, Hypertonie, tiefer Venenthrombose, Schlaganfall, Myokardinfarkt, instabiler Angina, Thromboembolie, Lungenembolie, thrombolytischen Erkrankungen, akuter arterieller Ischämie, peripheren thrombotischen Okklusionen, Koronarerkrankung, Atemwegserkrankungen, Bronchitis, Asthma und chronischobstruktiver Lungenerkrankung, eine pharmazeutische Zusammensetzung, umfassend eine therapeutisch wirksame Menge einer Verbindung nach Anspruch 1 und einen pharmazeutisch annehmbaren Träger.

12. Verwendung nach Anspruch 9, 10 oder 11, wobei die Verbindung eine Verbindung nach einem der Ansprüche 1 bis 8 ist.

13. Pharmazeutische Zusammensetzung, umfassend:
eine therapeutisch wirksame Menge einer Verbindung nach Anspruch 1 und einen pharmazeutisch annehmbaren Träger.

14. Pharmazeutische Zusammensetzung, umfassend:
eine therapeutisch wirksame Menge einer Verbindung nach einem der Ansprüche 1-8 und einen pharmazeutisch annehmbaren Träger.

## Revendications

1. Composé de Formule I : 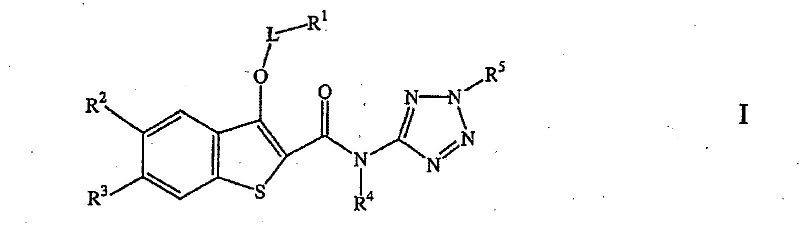 ou sel pharmaceutiquement acceptable correspondant, dans lequel:
R² et R³ sont sélectionnés dans le groupe consistant en :
(i) R² représente méthoxy et R³ est sélectionné dans le groupe consistant en H, méthyle et méthoxy ;
(ii) R² représente méthyle et R³ représente méthoxy ;
(iii) R² représente -O-CHF₂ et R³ représente méthyle;
(iv) R² représente -OH et R³ représente méthyle ;
(v) R² représente cyclopropyloxy et R³ est sélectionné dans le groupe consistant en H, méthyle et méthoxy ;
(vi) R² représente -O-CHF₂ et R³ représente. cyclopropyloxy ; et
(vii)R² représente éthoxy et R³ représente méthyle ;
R⁴ représente H ou CH₃ ;
R⁵ représente H ou CH₃ ;
L est soit absent, soit représente un groupe alkylène en C₁-C₄ ou (alkylène en C₁-C₄) -C (O) -;
R¹ est un groupe phényle substitué qui est substitué comme indiqué dans le groupe (a), (b), (c) ou (d); ou
R¹ est un groupe facultativement substitué sélectionné dans le groupe consistant en : naphtalényle, hétéroaryle à 5 éléments, hétéroaryle à 6 éléments, pyrimidinyle, pyridinylé, quinolinyle et indanyle, ledit groupe facultativement substitué pouvant être substitué comme indiqué dans (a), (b), (c) ou (d) ;
lesdits groupes (a), (b), (c) ou (d) étant :
(a) de 1 à 3 substituants indépendamment sélectionnés dans le groupe consistant en :
Br, F, Cl, -CN, -NO₂, -CF₃, -OH, -OCF₃, -SO₂-CH₃, alkyle en C₁-C₄, -CH₂CH₂-Br, -CH₂CH₂-S-(t-butyle), O-(alkyle en C₁-C₆), -C(NH)(NH₂)
-NH-C(O)-CH₃, NH₂, N(CH₃)₂, -CH₂-C (O) -O-CH₂CH₃, C(O)-(alkyle en C₁-C₄) et C(O)H;
(b) 1 substituant de formule J-R⁸,
J étant absent ou représentant -O-, alkylène en C₁-C₄, O- (alkylène en C₁-C₄); (alkylène en C₁-C₄)-C (O) - ou (alkylène en C₁-C₄) -S-,
R⁸ étant un groupe facultativement substitué sélectionné dans le groupe consistant en :
phényle, pipéridinyle, morpholinyle, tétrahydropyranyle, tétrahydrothiopyranyle, 1,1-dioxo-hexahydro-1λ⁶-thiopyranyle,
hétérocycloalkyle à '5 éléments, hétérocycloalkyle à 6 éléments, lesquels sont facultativement substitués par de 1 à 3 groupes indépendamment sélectionnés dans le groupe consistant en :
Br, F, Cl, -CN, -NO₂, -CF₃, -OH, -OCF₃, -SO₂-CH₃, alkyle en C₁-C₄, -O-(alkyle en C₁-C₆) , -C(NH)(NH₂), NH-C(O)-CH₃, NH₂, N(CH₃)₂, -C (O) -NH₂, -C (O) -CH₃, -C (O) - (alkyle en C₁-C₄), C(O)H et C (O)-C (CH₃)₂-NH-C(O)-O-t-butyle ;
(c) 1 substituant de formule Z-R⁹,
Z étant absent ou représentant -O-, -(alkylène en C₁-C₆), -O-(alkylène en C₁-C₆), -C(O)-, -CH(OH)-, - (alkylène en C₁-C₄)-S-, -(alkylène en C₁-C₆) -O- ou (alkylène en C₁-C₄)-C(O)-;
R⁹ étant un groupe cycloalkyle en C₄-C₇ facultativement substitué par 4 groupes méthyle ou par de 1 à 3 groupes indépendamment sélectionnés dans le groupe consistant en :
=O, Br, F, Cl, -CF₃, -OH, -OCF₃, -SO₂-CH₃, alkyle en C₁-C₄, -C (O) -NH₂, CH₂-O-CH₃, pipéridinyle et 1,3-dioxolan-2-yle ; et
(d) 1 ou 2 substituants indépendamment sélectionnés parmi (a) et 1 substituant parmi (b) ou (c).

2. Composé selon la revendication 1, dans lequel R² représente méthoxy, éthoxy ou -O-CHF₂ et R³ représente méthyle.

3. Composé selon la revendication 3, dans lequel R¹ est un groupe phényle substitué qui est substitué comme indiqué dans le groupe (a), (b), (c) ou (d) ; lesdits groupes (a), (b), (c) ou (d) étant :
(a) de 1 à 3 substituants indépendamment sélectionnés dans le groupe consistant en :
Br, F, Cl, -CN, -NO₂, -CF₃, -OH, -OCF₃, -SO₂-CH₃, alkyle en C₁-C₄, -CH₂CH₂-Br, -CH₂CH₂-S-(t-butyle), O-(alkyle en C₁-C₆), -CH₂-C(O)-O-CH₂CH₃ et C(O)-(alkyle en C₁-C₄);
(b) 1 substituant de formule J-R⁸,
J étant absent ou représentant -O-, alkylène en C₁-C₄, O-(alkylène en C₁-C₄), (alkylène en C₁-C₄)-C(O)- ou (alkylène en C₁-C₄)-S-,
R⁸ étant un groupe facultativement substitué sélectionné dans le groupe consistant en :
pipéridinyle, morpholinyle, tétrahydropyranyle, tétrahydrothiopyranyle, 1,1-dioxo-hexahydro-1λ⁶-thiopyranyle, lesquels sont facultativement substitués par de 1 à 3 groupes indépendamment
sélectionnés dans le groupe consistant en : Br, F, Cl, -CN, -NO₂, -CF₃, -OH, -OCF₃, -SO₂-CH₃, alkyle en C₁-C₄, -O-(alkyle en C₁-C₆) , et -C(O)-NH₂;
(c) 1 substituant de formule Z-R⁹,
Z étant absent ou représentant -O-, -(alkylène en C₁-C₆), -O-(alkylène en C₁-C₆), -(alkylène en C₁-C₆)-O- ou (alkylène en C₁-C₄)-C(O)-;
R⁹ étant un groupe cycloalkyle en C₄-C₇ facultativement substitué par de 1 à 3 groupes indépendamment sélectionnés dans le groupe consistant en : =O, Br, F, Cl, -CF₃, -OH, -OCF₃, -SO₂- CH₃, alkyle en C₁-C₄ et -C(O)-NH₂ ; et
(d) 1 ou 2 substituants indépendamment sélectionnés parmi (a) et 1 substituant parmi (b) ou (c).

4. Composé selon la revendication 3, dans lequel ledit composé est sélectionné dans le groupe consistant en:
• l'ester éthylique de l'acide {4-[5-méthoxy-6-méthyl-2-(2H-tétrazol-5-ylcarbamoyl)-benzo[*b*]thiophène-3-yloxyl-phényl}-acétique;
• le (2H-tétrazol-5-yl)-amide de l'acide 3-(4-isopropyl-phénoxy)-5-méthoxy-6-méthyl-benzo[*b*]thiophène-2-carboxylique;
• le (2H-tétrazol-5-yl)-amide de l'acide 3-(4-cyclopentyloxy-phénoxy)-5-méthoxy-6-méthyl-benzo[*b*]thiophène-2-carboxylique ;
• le (2H-tétrazol-5-yl)-amide de l'acide 3-(4-tert-butyl-phénoxy)-5-méthoxy-6-méthyl-benzo[*b*]thiophène-2-carboxylique ;
• le (2H-tétrazol-5-yl)-amide de l'acide 3-(4-bromo-phénoxy)-5-méthoxy-6-méthyl-benzo[*b*]thiophène-2-carboxylique ;
• le (2H-tétrazol-5-yl)-amide de l'acide 3-(4-fluoro-phénoxy)-5-méthoxy-6-méthyl-benzo[*b*]thiophène-2-carboxylique ;
• le (2H-tétrazol-5-yl)-amide de l'acide 3-(4-chloro-2-fluoro-phénoxy)-5-méthoxy-6-méthyl-benzo[*b*]thiophène-2-carboxylique ;
• le (2H-tétrazol-5-yl)-amide de l'acide 5-méthoxy-6-méthyl-3-(4-trifluorométhoxy-phénoxy)-benzo[*b*]thiophène-2-carboxylique;
• le (2H-tétrazol-5-yl)-amide de l'acide 3-[4-(1-carbamoyl-cyclopentyl)-phénoxy]-5-méthoxy-6-méthyl-benzo[*b*]thiophène-2-carboxylique;
• le (2H-tétrazol-5-yl)-amide de l'acide 5-méthoxy-6-méthyl-3-[4-(tétrahydro-pyran-4-yl)-phénoxy]-benzo[*b*]thiophène-2-carboxylique;
• le (2H-tétrazol-5-yl)-amide de l'acide 3-[4-(1,1-dioxo-hexahydro-1λ⁶-thiopyran-4-yl)-phénoxy]-5-méthoxy-6-méthyl-benzo[*b*]thiophène-2-carboxylique ;
• le (2H-tétrazol-5-yl)-amide de l'acide 5-méthoxy-6-méthyl-3-(2-nitro-4-cyclohexyl-phénoxy)-benzo[*b*]thiophène-2-carboxylique;
• le (2H-tétrazol-5-yl)-amide de l'acide 3-(2-chloro-4-cyclohexyl-phénoxy)-5-méthoxy-6-méthyl-benzo[*b*]thiophène-2-carboxylique ; et
• le (2H-tétrazol-5-yl)-amide de l'acide 3-(2-cyano-4-cyclohexyl-phénoxy)-5-méthoxy-6-méthyl-benzo[*b*]thiophène-2-carboxylique.

5. Composé selon la revendication 1, dans lequel R² représente méthoxy, méthyle ou -OH, et R³ représente méthoxy.

6. Composé selon la revendication 5, dans lequel R¹ est un groupe phényle substitué qui est substitué comme indiqué dans le groupe (a), (b), (c) ou (d); lesdits groupes (a), (b), (c) ou (d) étant:
(a) de 1 à 3 substituants indépendamment sélectionnés dans le groupe consistant en:
Br, F, Cl, -CN, -NO₂, -CF₃, -OH, -OCF₃, -SO₂-CH₃, alkyle en C₁-C₄, -CH₂CH₂-Br, -CH₂CH₂-S-(t-butyle), O-(alkyle en C₁-C₆), -CH₂-C (O) -O-CH₂CH₃ et C(O)-(alkyle en C₁-C₄);
(b) 1 substituant de formule J-R⁸,
J étant absent ou représentant -O-, alkylène en C₁-C₄, O-(alkylène en C₁-C₄), (alkylène en C₁-C₄)-C(O)- ou (alkylène en C₁-C₄)-S-,
R⁸ étant un groupe facultativement substitué sélectionné dans le groupe consistant en :
pipéridinyle, morpholinyle, tétrahydropyranyle, tétrahydrothiopyranyle, 1,1-dioxo-hexahydro-1λ⁶-thiopyranyle, lesquels sont facultativement substitués par de 1 à 3 groupes indépendamment
sélectionnés dans le groupe consistant en :
Br, F, Cl, -CN, -NO₂, -CF₃, -OH, -OCF₃, -SO₂-CH₃, alkyle en C₁-C₄, -O- (alkyle en C₁-C₆), et -C (O) -NH₂;
(c) 1 substituant de formule Z-R⁹,
Z étant absent ou représentant -O-, -(alkylène en C₁-C₆ ) , -O-(alkylène en C₁-C₆),- ( alkylène en C₁-C₆)-O- ou (alkylène en C₁-C₄)-C(O)-;
R⁹ étant un groupe cycloalkyle en C₄-C₇ facultativement substitué par de 1 à 3 groupes indépendamment, sélectionnés dans le groupe consistant en : =O, Br, F, Cl, -CF₃, -OH, -OCF₃, -SO₂-CH₃, alkyle en C₁-C₄ et -C(O)-NH₂ ; et
(d) 1 ou 2 substituants indépendamment sélectionnés parmi (a) et 1 substituant parmi (b) ou (c).

7. Composé selon la revendication 6, ledit composé étant :
• le (1H-tétrazol-5-yl)-amide de l'acide 3-(2-cyclohexyl-méthoxy-benzyloxy)-5,6-diméthoxy-benzo[*b*]thiophène-2-carboxylique ; ou
• le (2H-tétrazol-5-yl)-amide de l'acide 3-(4-cyclohexyl-phénoxy)-5-hydroxy-6-méthyl-benzo[*b*]thiophène-2-carboxylique.

8. Composé selon la revendication 1, dans lequel R² représente méthoxy, et R³ représente H.

9. Utilisation d'un composé selon la revendication 1 pour la préparation d'un médicament pour traiter un sujet, ladite utilisation comprenant :
l'administration, à un sujet souffrant d'une maladie sélectionnée dans le groupe consistant en : l'arthrite rhumatoïde, l'ostéoarthrite, l'arthrite psoriasique, le psoriasis, les maladies inflammatoires, les maladies auto-immunes, les maladies respiratoires, la bronchite, l'asthme et la maladie pulmonaire obstructive chronique, d'une composition pharmaceutique comprenant une quantité thérapeutiquement efficace d'un composé selon la revendication 1 et un support pharmaceutiquement acceptable.

10. Utilisation d'un composé selon la revendication 1 pour la préparation d'un médicament pour traiter un sujet, ladite utilisation comprenant :
l'administration, à un sujet souffrant d'une maladie sélectionnée dans le groupe consistant en : le cancer, le cancer du côlon, le glioblastome, le carcinome endométrial, le cancer hépatocellulaire, le cancer du poumon, le mélanome, le carcinome à cellules rénales, le carcinome de la thyroïde, le lymphome cellulaire, les troubles lymphoprolifératifs, le cancer du poumon à petites cellules, le carcinome du poumon à cellules squameuses, le gliome, le cancer du sein, le cancer de la prostate, le cancer de l'ovaire, le cancer du col de l'utérus et la leucémie, d'une composition pharmaceutique comprenant une quantité thérapeutiquement efficace d'un composé selon la revendication 1 et un support pharmaceutiquement acceptable.

11. Utilisation d'un composé selon la revendication 1 pour la préparation d'un médicament pour traiter un sujet, ladite utilisation comprenant:
l'administration, à un sujet souffrant d'une maladie sélectionnée dans le groupe consistant en : les maladies cardio-vasculaires, l'athérosclérose, l'hypertension, la thrombose veineuse profonde, l'accident vasculaire cérébral, l'infarctus du myocarde, l'angine instable, le thrombo-embolisme, l'embolisme pulmonaire, les maladies thrombolytiques, l'ischémie artérielle aiguë, les occlusions thrombotiques périphériques, la maladie des artères coronaires, les maladies respiratoires, la bronchite, l'asthme et la maladie pulmonaire obstructive chronique, d'une composition pharmaceutique comprenant une quantité thérapeutiquement, efficace d'un composé selon la revendication 1 et un support pharmaceutiquement acceptable.

12. Utilisation selon la revendication 9, 10 ou 11, dans laquelle ledit composé est un composé selon l'une quelconque des revendications 1 à 8.

13. Composition pharmaceutique comprenant :
une quantité thérapeutiquement efficace d'un composé selon la revendication 1 et un support pharmaceutiquement acceptable.

14. Composition pharmaceutique comprenant:
une quantité thérapeutiquement efficace d'un composé selon l'une quelconque des revendications 1 à 8 et un support pharmaceutiquement acceptable.
